# EUROPÄISCHE PATENTSCHRIFT

(11) **EP 2 210 954 B1**
(45) Veröffentlichungstag und Bekanntmachung des Hinweises auf die Patenterteilung: **22.06.2011**
(21) Anmeldenummer: 09151141.0
(22) Anmeldetag: 22.01.2009
(51) Int. Cl.: C12Q 1/68

(54) **Bestimmung des DNA-Methylierungsgrads**
Determining of the DNA methylation level
Détermination du niveau de méthylation de l'ADN

(43) Veröffentlichungstag der Anmeldung: 28.07.2010
(73) Patentinhaber: Heinrich-Heine-Universität Düsseldorf, 40225 Düsseldorf (DE)
(72) Erfinder: Santourlidis, Dr. rer. nat. Simeon, 41462 Neuss (DE)
(74) Vertreter: von Kreisler Selting Werner

(56) Entgegenhaltungen:
- WO-A-2006/060308
- WO-A-2006/111586
- WO-A-2008/134596
- US-A1- 2006 019 270
- US-A1- 2006 115 806
- ROMAN-GOMEZ ET AL: "Repetitive DNA hypomethylation in the advanced phase of chronic myeloid leukemia" LEUKEMIA RESEARCH, NEW YORK,NY, US, Bd. 32, Nr. 3, 26. Februar 2008 (2008-02-26), Seiten 487-490, XP022499004 ISSN: 0145-2126

## Beschreibung

Die Erfindung liegt auf dem Gebiet der Epigenetik, insbesondere der DNA-Methylierung. Sie stellt ein Amplifikationsverfahren für die Detektion epigenetischer Veränderungen bereit, die insbesondere für die klinische Diagnostik relevant sind. Weiterhin werden spezifische Primer für dieses Amplifikationsverfahren bereitgestellt.

Epigenetische Mechanismen verursachen Änderungen der Genexpression, die nicht mit einer Veränderung der kodierenden Sequenz der Gene einhergehen, jedoch z.B. mitotisch vererbt werden können. Die DNA-Methylierungsmuster werden replikationsgekoppelt von der Mutterzelle auf die Tochterzellen übertragen. Somit ist die Vererbung epigenetischer Erbinformation sichergestellt. In höheren Eukaryoten stellt neben RNA-assoziiertem Silencing und Histonmodifikation die DNA-Methylierung den am besten untersuchten epigenetischen Mechanismus dar (Serman et al., Coll Antropol. 2006; 30(3):665-71).

Das humane Genom besitzt in einer ausdifferenzierten gesunden Zelle ein spezifisches und weitgehend unveränderliches DNA-Methylierungsmuster, welches in entscheidender Art und Weise die Genexpression mitbestimmt. Hierbei sind genomische Regionen mit regulativer Funktion für die Transkription in vielen Fällen unmethyliert, wohingegen transkriptionell inaktive genomische Abschnitte methyliert sind.

Die DNA-Methylierung findet an den Cytosinresten der Nukleinsäure und vorzugsweise bei Dinukleotiden mit einer Cytosin-Guanin-Sequenz (CpG) statt. Die wichtigste Basenmodifizierung bei Eukaryoten ist die Methylierung an der 5'-Position des Cytosin.

In einer Tumorzelle, die sich u.a. durch eine erhöhte Proliferationsrate, eine veränderte Genexpression und chromosomale Anomalien auszeichnet, ist das genomische Methylierungsmuster aberrant (Schulz, DNA methylation in urological malignancies. Int J Oncol. 1998; 151-67). In vielen einschlägigen Reviews aus diesem Fachgebiet wird einhellig die Auffassung vertreten, dass diese epigenetischen Veränderungen ein immenses, diagnostisch und prognostisch relevantes Potential bergen, dessen Evaluierung zu modernen Verfahren der Krebsfrüherkennung, -prognose und -nachsorge führen kann.

Da jedoch bei Tumorgewebe chromosomale Anomalien auftreten, d.h. dieses Gewebe im Vergleich zu gesundem Gewebe eine unterschiedliche genomische Ausstattung aufweist, besteht das grundlegende Problem darin, eine solche aberrante DNA-Methylierung nicht nur qualitativ, sondern auch quantitativ und normiert zu bestimmen. Nur eine solche Bestimmung lässt einen direkten Vergleich von zwei Proben zu, von denen eine möglicherweise chromosomale Anomalien aufweist.

Diese Aufgabe wird erfindungsgemäß gelöst durch das Verfahren gemäß Anspruch 1, sowie durch die vorteilhaften Aus- und Weiterbildungen der abhängigen Ansprüche. Die vorliegende Erfahrung stellt zur Lösung dieser Probleme Methoden zur Bestimmung der normierten DNA-Methylierung und Methoden zur Bestimmung des relativen DNA-Methylierungsgrads zwischen zumindest zwei Proben bereit.

In einem ersten Aspekt wird ein Verfahren zur Bestimmung des normierten DNA-Methylierungsgrads offenbart, umfassend die Schritte: a) Quantitative Bestimmung der Gegenwart eines Transposons oder Fragments davon in einer DNA; b) Quantitative Bestimmung des Vorliegens von zumindest einem differentiell methylierten C eines CpG Dinukleotids aus dem Transposon oder Fragment davon; und c) Bestimmung des normierten DNA-Methylierungsgrads über die in den Schritten a) und b) bestimmten Werte.

Hierbei macht sich die vorliegende Erfindung die überraschende Feststellung zu nutze, dass der Methylierungsgrad von zufällig über das gesamte Genom verteilten Transposons als repräsentativ für den Methylierungsgrad des Gesamtgenoms angesehen werden kann. Das Prinzip der Ereindung liegt darin, in einem ersten Schritt quantitativ die Gegenwart eines Transposons (oder Fragments davon) in einer DNA aus z.B. einer Probe zu ermitteln und in einem weiteren Schritt quantitativ das Vorliegen von zumindest einem differentiell methylierten Cytosin eines CpG Dinukleotids desselben Transposons (oder Fragments davon) in derselben DNA zu ermitteln. Über das Verhältnis der bestimmten Werte kann dann ein normierter DNA-Methylierungsgrads bestimmt werden, der für das gesamte Genom repräsentativ ist.

Die Methylierung von DNA ist ein post-replikativer, epigenetischer Mechanismus, der in Eukaryoten von erheblicher Bedeutung für die Genregulation ist. Bei Eukaryoten spielt die Addition einer Methylgruppe an das Kohlenstoffatom Nr. 5 der Pyrimidinbase des Cytosins zur Bildung von 5-Methylcytosin (^{5m}C) die dominierende Rolle. Diese Methyladdition wird *in vivo* durch einen Transfer der Methylgruppe von S-Adenosylmethionin (Methyldonor) auf Cytosin (Methylakzeptor) mithilfe von DNA-Methylasen (DNMTs) katalysiert und tritt bevorzugt bei Cytosinen auf, die 5' zu einem Guanin lokalisiert liegen (CpG).

Im Vertebraten-Genom tritt ^{5m}C ausschließlich in CpG-Dinukleotiden auf (Bestor. The DNA methyltransferases of mammals. Hum Mol Genet 2000; 2395-2402) und im menschlichen Genom sind meistens beide Cytosine des palindromischen CpG-Dinukleotids methyliert.

CpG-Dinukleotide sind aufgrund evolutionärer Mechanismen und der Neigung von Methylcytosin spontan zu deaminieren zumindest im Säuger-Genom mit einer Häufigkeit von 0,8% stark unterrepräsentiert (der durchschnittliche GC-Gehalt beim Menschen beträgt ca. 40%, was zu einer rechnerischen Häufigkeit des CpG-Dinukleotids von 4% führen sollte) und treten in der Regel nur in "CpG-Inseln" gehäuft auf, die oft in den 5' oder 3'-NTRs von Genen liegen (Gardiner-Garden & Frommer. CpG islands in vertebrate genomes. J Mol Biol 1987; 261-282). Ursache für diese Begrenzung auf nicht-codierende Bereiche ist vermutlich das erhöhte Risiko von Punktmutationen durch die Desaminierung von ^{5m}C zu Thymin (Laird & Jaenisch. The role of DNA methylation in cancer genetic and epigenetics. Annu Rev Genet 1996; 441-464).

CpG-Inseln haben eine Größe von ca. 500 bp - 4 kb und einen erhöhten GC-Gehalt von > 55%. Sie weisen eine zehn- bis zwanzigfach erhöhte Frequenz des Dinukleotids 5'-CpG-3' auf. Mehr als drei Viertel aller (ca. 25.000) menschlichen Gene weisen CpG-Inseln in ihren Startbereichen auf.

Generell liegen Gene mit hoher transkriptioneller Aktivität in unmethylierten genomischen Bereichen. Demgegenüber liegen in methylierten Bereichen Gene, die transkriptionell nur wenig oder gar nicht aktiv sind. Es besteht eine Vernetzung von DNA-Methylierung und Chromatin-Kondensation, da generell Gene in dicht gepacktem Heterochromatin inaktiv sind. Eine solche dichtere Packung des Chromatins wird durch die Deacetylierung und Methylierung der Histone H3 und H4 eingeleitet, was in einer festeren Bindung der Nukleosomen an die DNA führt und somit eine schlechtere Zugänglichkeit der DNA für die Transkriptionsmaschinerie resultiert (Jenuwein. Re-SET-ting heterochromatin by histone methyltransferases. Trends Cell Biol 2001; 266-273). Das an CpG-methylierte DNA bindende Protein MeCP2 kann Histon-Deacetylasen rekrutieren und die Kondensierung des Chromatins einleiten (Razin & Razin. CpG methylation, chromatin structure and gene silencing-a three-way connection. EMBO J. 1998; 4905-4908). Auch Histon-Methylasen können jedoch DNA-Methyltransferasen in heterochromatische Regionen leiten und somit dort die DNA-Methylierung auslösen (Tamaru & Selker. A histone H3 methyltransferase controls DNA methylation in Neurospora crassa. Nature 2001; 277-283). Weiterhin führt die Histon-Acetylierung vermutlich zur aktiven Demethylierung des betroffenen Genabschnitts (Cervoni & Szyf. Demethylase activity is directed by histone acetylation. J. Biol. Chem. 2001; 40778-40787).

Wie eingangs erwähnt werden fehlerhafte DNA-Methylierungen meist stabil an Tochterzellen vererbt und können daher auf Organismenebene häufig die Ursache für Krankheiten sein. Insbesondere zeigen z.B. Tumorzellen oft Methylierungsmuster, die von denjenigen gesunder Gewebe signifikant abweichen. Daher wird erwogen, die Analyse des Methylierungsgrads für z.B. diagnostische Anwendungen einzusetzen. Weiterhin wird auch eine gerichtete Änderung/Korrektur des Methylierungszustands zum Zwecke der Genregulation erwogen.

Für die Analyse des Methylierungzustands von Nukleinsäuren, insbesondere des Methylierungsstatus von spezifischen CpG-Stellen, hat sich die Bisulfitierung mit z.B. anschließender Amplifikation/Sequenziemng bewährt, die erstmals durch Frommer et al. (Proc Natl Acad Sci USA. 1992; 89(5):1827-31) beschrieben wurde. Bei der Bisulfitierung werden unmethylierte Cytosinbasen der Nukleinsäure in Uracilbasen umgewandelt, während methylierte Cytosinbasen unverändert bleiben. Eine Übersicht über verschiedene Techniken zur Analyse des Methylierungszustands von Nukleinsäuren, insbesondere die Bisulfitierung, findet sich in Fraga et al., Biotechniques. 2002; 33(3):632, 634, 636-49 und Laird, Nat Rev Cancer. 2003;3(4):253-66. Die Bisulfitierungreaktion führt daher abhängig vom Methylierungszustand der Ausgangsnukleinsäure zu Nukleinsäuresequenzen mit unterschiedlicher Sequenz, nach deren Analyse, unter anderem durch PCR oder Sequenzierung, auf den Methylierungszustand der Ausgangsnukleinsäure zurückgeschlossen werden kann.

Diese Analyse des Methylierungsgrads einer Probe, wie z.B. Gewebe oder einem Bioptat, stößt mit herkömmlichen Verfahren schnell an ihre Grenzen, sobald ein Vergleich diese Probe mit einer weiteren Probe erfolgen soll und zwischen den beiden Proben eine unterschiedliche genomische Ausstattung vorliegt. Wie oben erwähnt weisen nämlich Tumorzellen in vielen Fällen nicht balancierte chromosomale Anomalien auf. Darunter fallen Gewinne und Verluste ganzer Chromosomen, einzelner Chromosomenarme und kürzerer DNA Sequenzabschnitte. Mehrere Studien liefern Evidenzen dafür, dass diese genomische Instabilitäten der Tumorzellen durch einen geringen Grad der DNA Methylierung hervorgerufen werden. Hierbei zeigt sich der Grad der DNA Hypomethylierung proportional zur genomischen Instabilität und zur Tumoragressivität.

Die vorliegende Erfindung macht sich die überraschende Feststellung zu nutze, dass der Methylierungsgrad von zufällig über das gesamte Genom verteilten Transposons als repräsentativ für den Methylierungsgrad des Gesamtgenoms angesehen werden kann. Somit ist eine normierte Bestimmung des Methylierungsgrads des Genoms möglich, wenn neben der quantitativen Gegenwart zumindest einer differentiellen Methylierung innerhalb eines Transposons auch die quantitative Gegenwart des Transposons "an sich" im Genom bestimmt wird.

WO 2008/134596 bescheibt ein Verfahren zur Bestimmung des "unmethylation index", in dem die copy number von nicht-methyliertem Transposon mit des copy number der Gesamtmenge an Transposon ions Verhältnis gesetzt wird.

Der Begriff "Transposon", wie hierin verwendet, bezeichnet einen DNA-Abschnitt bestimmter Länge im Genom. Ein Transposon umfasst ein oder mehrere Gene und hat die Möglichkeit seinen Ort im Genom zu verändern (Transposition). Transposons können Elemente sein, deren mobile Zwischenstufe von RNA gebildet wird (Retroelemente; Klasse-I-Transposon), oder solche, deren mobile Phase DNA ist (DNA-Transposon; Klasse-II-Transposon).

Der Begriff "Transposon", wie hierin verwendet, schließt immer auch Fragmente eines solchen Transposons ein. Solche Fragmente eines Transposons entstehen im Laufe der Evolution im Genom, da ein "gesprungenes" Transposon keinem Selektionsdruck ausgesetzt ist und somit die ursprüngliche Sequenz durch Mutationen im Genom verändert werden kann. So finden sich im Genom häufig nicht die kompletten Transposons, sondern nur noch Teilbereiche derselben wieder, die z.B. durch eine weitere Insertion eines Transposons oder Deletionen, die in den meisten Fällen im 5'-Bereich liegen, entstanden sind. Unter einem Fragment eines Transposons wird bevorzugt ein zusammenhängender Nukleinsäurebereich mit einer Länge von ≥ 40 bp, ≥ 80 bp, ≥ 100 bp, bevorzugt ≥ 150 bp, noch bevorzugter ≥ 200 bp verstanden, der zumindest eine Homologie von 75%, 80%, 85%, 90%, 95%, 97%, 98%, bevorzugt 98,5%, 99%, 99,5% und noch bevorzugter 99,7%, 99,9% oder mehr zu dem korrespondierenden Nukleinsäurebereich des Transposons aufweist. Eine solche Homologie ist z.B. über den FASTA-Algorithmus bestimmbar. Gleichbedeutend mit Transposon ist auch "transposables Element" zu verstehen.

Autonome DNA-Transposons bestehen aus DNA-Sequenzen, die für das Enzym Transposase codieren. Die Transposase ist in der Lage, ein Transposon aus dem Genom "herauszuschneiden", es an eine neue Stelle im Genom zu transportieren und dort in das Genom zu inserieren. Dieser Prozess wird konservative Transposition genannt. Beispiele für DNA-Transposons sind: Ac (Activator) transposable elements (autonomes Transposon) oder Ds (Dissociator) transposable elements (nicht-autonomes Transposon ohne eigene Transposase).

Retrotransposons stellen die Mehrheit der eukaryotischen transposablen Elemente dar und sind komplexer aufgebaut. Sie werden von der Wirtszelle als "normale" DNA-Sequenz innerhalb des Genoms erkannt und somit durch die Transkriptions-Maschinerie der Wirtszelle abgelesen und in RNA umgeschrieben. Retrotransposons codieren jedoch für eine reverse Transkriptase, die eine Umwandlung dieser RNA in DNA ermöglicht. Diese Transposase führt auch die Insertion der gebildeten DNA in das Genom der Wirtszelle durch. Dieser Prozess wird replikative Transposition genannt. Solange das Retrotransposon funktionell bleibt, werden daher mehrere Kopien im Genom erzeugt.

Die Anzahl der aktiven Transposons im Genom eines Organismus variiert in großem Maße mit der Spezies. Im menschlichen Genom ist z.B. lediglich ein sehr kleiner Teil der Transposons aktiv. Es wird hierbei davon ausgegangen, dass nur ca. 50 LINE Transposons (s.u.) und so gut wie keine DNA-Transposons aktiv sind, womit die Anzahl der Transposons im Laufe des Lebens eines Menschens als nahezu konstant angesehen werden kann.

Es gibt zwei Hauptarten von Retrotransposons: virale und nicht-virale Retrotransposons.

Virale Retrotransposons haben im weitesten sehr ähnliche Eigenschaften wie die Retroviruse. Beispiele für virale Retrotransposons sind: Ty transposable elements und drosophilacopia transposable elements.

Nicht-virale Retrotransposons stellen die Mehrheit aller Transposons in Säugetieren dar. Als Beispiele sind insbesondere zu nennen: LINEs (long interspersed (transposable) elements), SINEs (short interspersed (transposable) elements), und Alu Elemente.

Im menschlichen Genom kommen ungefähr 850.000 LINEs und 1.500.00 SINEs vor. Unter den SINEs befinden sich auch die Alu Elemente, die die im menschlichen Genom am häufigsten vorkommende Gruppe der transposablen Elemente darstellen und etwa 5% des Genoms ausmachen.

Zu den viralen Retrotransposons sind auch die HERVs (human endogenous retroviruses) zu zählen. Sie werden anhand einer charakteristischen Aminosäureposition in Unterfamilien eingeordnet (z.B. HERV-K, HERV-W). Sie machen schätzungsweise 8% des humanen Genoms aus. Ihren Ursprung haben sie in retroviralen Infektionen der Keimbahn, welche im Zuge der Evolution des Menschen wiederholt stattgefunden haben. Durch Mutationen und Deletionen sind die meisten dieser genetischen Elemente jedoch transkriptionell inaktiv geworden. Nur wenige weisen eine full-length Organisation, mit den viralen Genen gag, pol und env, auf. Diese sind dann von LTR (long terminal repeat) Sequenzen flankiert, welche regulatorische Sequenzmodule beinhalten. Durch DNA Methylierung sind potentiell aktive HERVs stillgelegt damit sie nicht mit der Integrität der Genexpression einer gesunden Zelle interferieren können. Hingegen findet man in verschiedenen Tumorentitäten eine erhöhte HERV-Transkription und auch -Proteinbiosynthese.

Mit der Bezeichnung "Methylierungsgrad" soll vorliegend die Demethylierung oder Methylierung einer DNA gemeint sein. Eine betrachtete DNA kann an zumindest einer Stelle entweder methyliert oder un- bzw. demethyliert vorliegen. Da dieser Zustand ein binärer ist und damit Demethylierung und Methylierung an einer bestimmten Position direkt miteinander in Verbindung stehen, kann der Methylierungsgrad entweder über die Demethylierung und/oder die Methylierung an dieser zumindest einen Stelle bestimmt werden. Somit kann der normierte DNA-Methylierungsgrad, sowie auch der relative Methylierungsgrad über die Methylierung und/oder Demethylierung der DNA bestimmt werden.

Vorliegend werden die Begriffe "Primer" und "Oligonukleotid" synonym verwendet. Ein Primer wird als spezifisch für eine gegebene Sequenz angesehen, wenn ≥ 75 %, ≥ 80 %, ≥ 85 %, ≥ 90 %, bevorzugt ≥ 95 %, ≥ 97 %, noch bevorzugter ≥ 99 %, oder ≥ 99,5 % Sequenzidentität zu der betrachteten Sequenz oder deren Komplement aufweist. In einer besonders bevorzugten Form weist der Primer 100% Sequenzidentität zu der betrachteten Sequenz oder deren Komplement auf. In anderen bevorzugten Ausführungsformen wird der Primer dann als spezifisch für eine gegebene Sequenz angesehen, wenn er unter Hochsalzbedingungen mit dieser (oder deren Komplement) hybridisiert.

Unter dem Term "Hochsalzbedingungen" wird im Folgenden ein Milieu verstanden, das einen Hochsalzpuffer verwendet, bevorzugt einen Hochsalzpuffer enthaltend chaotrope Salze. Durch Hochsalz, bevorzugt aufweisend chaotrope Salze, wird die Löslichkeit von Nukleinsäuren in Wasser herabgesetzt. Grund ist das Aufbrechen von Wasserstoffbrücken und damit verbunden eine Verringerung der Stabilisierung von Sekundär- und Tertiär- Strukturen der Nukleinsäuren in Wasser. Wird nun eine polare Oberfläche als Wasserstoffbrückendonor angeboten, binden die Nukleinsäuren an dieser Oberfläche, da sie dort eine bessere Stabilisierung erfahren als in Wasser. Wird die Salzkonzentration verringert, wird Wasser wieder ein besserer Wasserstoffbrückendonor als die polare Oberfläche, und die Nukleinsäuren lassen sich wieder von der Oberfläche ablösen.

Unter dem Term "Hochsalzpuffer" wird insbesondere - aber nicht darauf beschränkt - ein Puffer aufweisend eine hohe Salzkonzentration (bevorzugt chaotrope Substanzen), bevorzugt ≥ 100 mM, bevorzugter ≥ 500 mM und noch bevorzugter ≥ 1 M, verstanden.

Unter dem Term "chaotrope Substanzen" bzw. "chaotrope Salze" werden insbesondere - aber nicht darauf beschränkt - Substanzen, die die Sekundär-, Tertiär- und/oder Quartärstruktur von Proteinen und/oder Nukleinsäuren ändern und zumindest die Primärstruktur intakt lassen, die Löslichkeit polarer Substanzen in Wasser verringern und/oder hydrophobe Wechselwirkungen verstärken, verstanden. Bevorzugte chaotrope Substanzen sind Guanidinhydrochlorid, Guanidinium(iso)thiocyanat, Natriumiodid, Natriumperchlorat, Kaliumiodid, Natrium(iso)thiocyanat und/oder Harnstoff.

Unter dem Begriff "Amplifikation" oder "Amplifikationsreaktion" wird ein Verfahren verstanden, das es ermöglicht, die Konzentration einer betrachteten Nukleinsäuresequenz mindestens zu verdoppeln.

Man unterscheidet hier zwischen isothermen und thermocyclischen Amplifikationsreaktionen. Bei ersteren bleibt die Temperatur während des gesamten Verfahren stets gleich, während bei letzterer thermische Zyklen durchlaufen werden, mit deren Hilfe die Reaktion und die Amplifikation gesteuert wird.

Bevorzugte isotherme Amplifikationsreaktionen sind z.B.
- Loop mediated isothermal amplification (LAMP),
- Nucleic Acid Sequence Based Amplification (NASBA),
- Rolling Circle Chain Reaction (RCCR), oder Rolling Circle Amplification (RCA), und/oder
- Transcription mediated amplification (TMA)

Bevorzugte thermocyclische Amplifikationsreaktionen sind z.B.
- Ligase-Kettenreaktion (LCR), und/oder
- Polymerase Ketttenreaktion (PCR)

Unter dem Term "Polymerase Kettenreaktion" (PCR) wird ein Verfahren zur in vitro-Vervielfältigung von Nukleinsäuren verstanden, wie es z.B. in Bartlett & Stirling (2003) beschrieben ist.

Unter dem Term "Ligase-Kettenreaktion" (LCR) wird ein Nachweisverfahren für geringste Mengen von Nukleinsäuren verstanden, das ähnlich wie die Polymerase-Kettenreaktion funktioniert, nur unter Verwendung eines anderen Enzyms (anstatt der Polymerase eine Ligase). Zwei Proben pro DNA-Strang werden zu einer Probe ligiert. Die entstehenden, oft nur 30-50 bp langen Amplifikate eines Zyklus dienen in den folgenden Zyklen selbst wieder als Ansatzpunkt für die supplementierten Primer.

Unter dem Term "Loop-mediated Isothermal Amplification" (LAMP) wird eine Methode zur isothermalen Nukleinsäureamplifikation verstanden, bei welcher 6 verschiedene Primer eingesetzt werden, die bestimmte Regionen auf der Target-Sequenz erkennen und daran binden. LAMP nutzt eine DNA-Polymerase mit Strand-displacement Aktivität und läuft bei einer konstanten Temperatur von etwa 65°C ab. Amplifikation und Detektion der Target-Sequenz findet in einem einzigen Schritt statt.

Unter dem Term "Nucleic Acid Sequence Based Amplification" (NASBA), wird ein Verfahren zur Amplifikation von RNA verstanden (Compton 1991). Dabei wird eine RNA-Matrix zu einem Reaktionsgemisch gegeben, und ein erster Primer bindet an die komplementäre Sequenz im Bereich des 3'-Endes der Matrix. Anschließend wird mit einer Reversen Transkriptase der zur Matrix komplementäre DNA-Strang polymerisiert. Mit Hilfe von RNase H wird dann die RNA-Matrix verdaut (RNase H verdaut nur RNA in RNA-DNA Hybriden, nicht single-stranded RNA). Anschließend wird ein zweiter Primer an das 5' Ende des DNA-Stranges gebunden. Dieser wird von der T7 RNA Polymerase als Startpunkt für die Synthese eines zum DNA-Strang komplementären RNA-Moleküls verwendet, welches dann wieder als Ausgangsmatrix verwendet werden kann. NASBA wird bei einer konstanten Temperatur von normalerweise 41°C. durchgeführt und liefert unter bestimmten Umständen schnellere und bessere Resultate als PCR.

Unter dem Term "Transcription Mediated Amplification" (TMA) wird ein von der US-Firma Gen-Probe entwickeltes isothermales Amplifikationsverfahren verstanden, das der NASBA° ähnlich ist und bei dem ebenfalls RNA Polymerase und Reverse Transkriptase verwendet werden (Hill, 2001)

Der Term "Rolling Circle Chain Reaction" (RCCR) oder "Rolling circle Amplification" (RCA) betrifft ein Amplifikationsverfahren, das die allgemeine Nukleinsäure-Replikation nach dem rolling-circle-Prinzip imitiert und unter anderem in der US5854033 beschrieben ist.

Unter dem Term "Real-Time-PCR" auch quantitative PCR oder qPCR (nicht zu verwechseln mit revers transkribierter PCR) wird ein Verfahren verstanden, das auf dem Prinzip der bekannten Polymerase-Kettenreaktion (PCR) beruht, und zusätzlich die Quantifizierung der amplifizierten DNA ermöglicht. Die Quantifizierung wird mit Hilfe von Fluoreszenz-Messungen durchgeführt, die während eines PCR-Zyklus erfasst werden (daher der Name "Real Time"). Die Fluoreszenz nimmt proportional mit der Menge der PCR-Produkte zu. Am Ende eines Laufs (der aus mehreren Zyklen besteht) wird anhand von erhaltenen Fluoreszenzsignalen die Quantifizierung in der exponentiellen Phase der PCR vorgenommen. Nur in der exponentiellen Phase der PCR (die wenige Zyklen in einem Lauf dauert) ist die korrekte Quantifizierung möglich, da während dieser Phase die optimalen Reaktionsbedingungen herrschen. Diese Methode unterscheidet sich somit von anderen quantitativen PCR-Methoden, die erst nach Ablauf der PCR eine quantitative Auswertung (z. B. kompetitive PCR), meist unter Einbeziehung einer gelelektrophoretischen Auftrennung der PCR-Fragmente, vornehmen.

Für die Detektion kommen Farbstoffe wie z.B. Ethidiumbromid, SYBR Green I sowie FRET-Sonden oder sogenannte Double-Dye-Oligos (auch als TaqMan-Sonden bezeichnet) in Frage.

Der Begriff "CT-Wert" (Threshold Cycle = "Schwellenwert-Zyklus") bezeichnet den PCR-Zyklus, an dem erstmals ein Amplifikat nachweisbar ist; hierbei wird in der Regel die Fluoreszenz gemessen und der Zyklus angegeben, bei welcher letztere erstmalig signifikant über die Hintergrund-Fluoreszenz ansteigt.

In der Anfangsphase einer PCR-Reaktion ist die Menge an Template (d.h. an zu amplifiziernder DNA) noch begrenzt, während in der Schlussphase der Amplifikation die Menge der Produkte derart ansteigt, dass es zur Hemmung durch diese kommt, Produktfragmente zunehmend miteinander hybridisieren und die Edukte langsam verbraucht werden. Nur in der dazwischenliegenden Phase besteht ein exponentieller Zusammenhang zwischen Anzahl der Amplifikationszyklen und Amplifikatmenge ("exponentielle Phase"). Für die Bestimmung des Zeitpunkts, an welchem die exponentielle Phase beginnt, macht man sich den erwähnten CT-Wert zunutze.

Ein niedriger CT-Wert gibt überdies an, dass bereits eine geringe PCR-Zyklenzahl für einen erstmaligen signifikanten Anstieg der Fluoreszenz über das Grundrauschen ausreichend ist (also relativ viel Template vorhanden war), während ein hoher CT-Wert dementsprechend angibt, dass hierfür viele PCR-Zyklen benötigt werden (also relativ wenig Template vorhanden war).

In einem ersten Aspekt betrifft die vorliegende Erfindung ein Verfahren zur Bestimmung des normierten DNA-Methylierungsgrads umfassend die Schritte: a) Quantitative Bestimmung der Gegenwart eines Transposons oder Fragments davon in einer DNA; b) Quantitative Bestimmung des Vorliegens von zumindest einem differentiell methylierten C eines CpG Dinukleotids aus dem Transposon oder Fragment davon; und c) Bestimmung des normierten DNA-Methylierungsgrads über die in den Schritten a) und b) bestimmten Werte.

Erfingdungsgemäß wird somit in einem ersten Schritt die Gegenwart eines Transposons in einer gegebenen (z.B. aus einem Bioptat isolierten) DNA mengenmäßig bestimmt. Dieser Schritt liefert einen Wert, der Auskunft über die Dichte/Frequenz (in Bezug auf die eingesetzte DNA) oder Anzahl des Transposons in der untersuchten DNA gibt, also z.B. wie viele Kopien des Transposons in der untersuchten DNA vorhanden sind. In einer bevorzugten Ausführungsform wird diese Bestimmung an einer zuvor bisulfitierten DNA durchgeführt.

In einem zweiten Schritt wird dann das Vorliegen von zumindest einem differentiell methylierten Cytosin eines CpG Dinukleotids innerhalb der im ersten Schritt detektierten Transposons mengenmäßig bestimmt. In einer bevorzugten Ausführungsform wird hierbei dieselbe DNA wie im ersten Schritt verwendet. Zum Beispiel kann die aus einer Probe isolierte DNA in zwei Teile geteilt werden, wobei jedes Teil vorzugsweise die gleiche Menge an DNA enthält. In einer weiteren bevorzugten Ausführungsform wird diese Bestimmung im zweiten Schritt an einer bisulfitierten DNA durchgeführt (auf diesen Schritt wird weiter unten detaillierter eingegangen). Der erhaltene Wert gibt demnach Auskunft über die Gegenwart von methylierten oder unmethylierten Cytosinen an vielen verschiedenen, zufällig verteilten Positionen im Genom. Dieser zweite Schritt gibt somit Auskunft über die Anzahl/Menge von differentiell methylierten Cytosinen innerhalb des im ersten Schritt bestimmten Transposons. Demnach wird der Grad der differentiellen Methylierung der Cytosine in den untersuchten Transposons bestimmt.

Da Transposons zufällig über das Genom verteilt sind, kann mittels der beiden erhaltenen Werte in einem nächsten Schritt eine Normierung durchgeführt werden, bei der die im ersten und zweiten Schritt bestimmten Werte zueinander in Beziehung gesetzt werden. Aufgrund der hohen Anzahl der Transposons im Genom ist ein guter Stichprobenumfang gegeben. Der erhaltene Wert der normierten DNA-Methylierung spiegelt somit einen Wert der differentiellen Methylierung wieder, der als auf das jeweilige betrachtete Genom normiert angesehen werden kann.

In weiteren Ausführungsformen können der erste und der zweite Schritt von ihrer zeitlichen Abfolge vertauscht werden oder gleichzeitig, z.B. mittels real time PCR, ausgeführt werden.

Unter "quantitativer Bestimmung" soll vorliegend eine Detektion des Vorhandenseins eines Transposons oder des Vorhandenseins einer differentiellen Methylierung verstanden werden. Hierbei soll nicht eine bloße qualitative Detektion erfolgen, d.h. die Frage beantwortet werden, ob ein Transposon oder eine differentielle Methylierung in der untersuchten DNA vorhanden ist, sondern dieses Vorhandensein auch quantifiziert werden (zum Beispiel durch Mengenangaben, Anzahlen von Kopien und dergleichen).

Dem Fachmann sind verschiedene Verfahren bekannt, um eine solche quantitative Bestimmung durchzuführen. In einer bevorzugten Ausführungsform erfolgt diese quantitative Bestimmung über eine Amplifikation mit anschließender Messung der Menge des erzeugten Amplifikats. In einer bevorzugteren Ausführungsform ist die Amplifikation eine PCR. In einer weiteren noch bevorzugteren Ausführungsform erfolgt die quantitative Bestimmung mittels real time PCR zur Bestimmung des CT-Werts. In einer weiteren Ausführungsform erfolgt die quantitative Bestimmung über Hybridisierung einer markierten Sonde (z.B. Nukleinsäuresonde) mit anschließender Bestimmung der Höhe des durch die Markierung (direkt oder indirekt) erzeugten Signals. In einer weiteren Ausführungsform wird eine in situ Hybridisierung (z.B. FISH) durchgeführt mit anschließender Bestimmung der Höhe des durch die Markierung erzeugten Signals. Weitere Verfahren für solche quantitative Bestimmungen sind die Detektion mit 5-methyl-Cytosin-spezifischen Antikörpern, oder der indirekte Nachweis von an methylierte DNA bindenden Faktoren mittels spezifischer Antikörper. Zu solchen Faktoren gehören beispielsweise der nukleäre Repressor MeCP2, der an symmetrisch methylierte CpG Positionen des Genoms bindet, sowie MBD1, MBD2, MBD4.

In weiteren Ausführungsformen stammt die DNA aus Organismen, Gewebe, Zellen, Bioptat, oder einer Probe. Vorzugsweise ist die DNA isolierte DNA. In einer Ausführungsform ist die isolierte DNA genomische und/oder eukaryotische DNA. Bevorzugt ist diese DNA die DNA eines Vertebraten, noch bevorzugter die eines Menschen. In einer weiteren bevorzugten Ausführungsform umfasst das Bereitstellen der DNA nicht die Probenentnahme selbst, sondern baut auf bereits gewonnenem Probenmaterial auf. In einer weiteren bevorzugten Ausführungsform stammen die Gewebe, Zellen, Bioptate, Proben aus denen die DNA stammt von einem gesunden Individuum oder einem erkrankten Individuum oder Patienten.

In weiteren Ausführungsformen ist die Probe ausgewählt aus der Gruppe bestehend aus einer Blutprobe, einer Gewebeprobe, einer Speichelprobe, einer Urinprobe, einem Abstrich und einer Stuhlprobe. In einer bevorzugten Ausführungsform ist die Probe eine Urinprobe. Dies ist insbesondere für den Nachweis eines Blasen- und/oder Prostatakarzinoms vorteilhaft.

In weiteren Ausführungsformen der Erfindung ist das Transposon ausgewählt aus der Gruppe bestehend aus einem LINE Element, einem Alu Element (Alu Konsensussequenz; Kariya et al. Gene. 1987;53(1):1-10), einem HERV Element oder einem Fragment derselben. In einer besonderen Ausführungsform ist das Transposon ein LINE-1 Element (GenBank Accession M80343) oder ein Fragment davon. Noch bevorzugter ist das Fragment des Transposons der Promotorbereich eines Transposons. Dies hat den Vorteil, dass eine hohe Frequenz von CpG vorhanden ist und somit die der Methylierungsgrad besser bestimmt werden kann. In der bevorzugtesten Ausführungsform ist das Fragment des Transposons der Promotorbereich eines LINE-1 Elements.

Unter "differentieller Methylierung" wird vorliegend der in den verschiedenen möglichen Ausprägungsformen vorliegende Methylierungszustand einer gegebenen DNA verstanden. Ist von einer "differentiellen Methylierung eines Cytosins" (C) die Rede, so wird darunter der Methylierungszustand des betreffenden Cytosins verstanden. Dieser kann binär entweder methyliert sein, d.h. dieses Cytosin liegt als ^{5m}C vor, oder er kann unmethyliert (oder demethyliert) sein, d.h. das betrachtete Cytosin weist keine 5-ethylgruppe auf.

Ein bevorzugtes Verfahren zur Bestimmung des Vorliegens einer differentiellen Methylierung (bzw. des Vorliegens einer differentiellen Methylierung eines Cytosins) basiert auf der Bisulfitierung von DNA mit anschließender Analyse der erzeugten bisulfitierten DNA.

Um von isolierter DNA zu Bisulfit-konvertierter DNA zu gelangen, wird diese durch eine dem Fachmann hinlänglich bekannte Bisulfitierungsreaktion umgewandelt. Hierbei werden die unmethylierten Cytosine der DNA durch das Bisulfit in Uracil umgewandelt. Als Folge der Umwandlung können verschiedene Varianten einer umgewandelten Nukleinsäure vorliegen, die von der Anzahl der unmethylierten Cytosine dieser Nukleinsäure abhängen. Eine Nukleinsäure, die beispielsweise 2 Cytosine enthält kann demnach, je nach Methylierungszustand dieser Cytosine, zu 4 unterschiedlichen Varianten nach der Bisulfitierung führen, da entweder keines, das erste, das zweite, oder beide der Cytosine unmethyliert vorliegen kann/können und in Uracil umgewandelt wird/werden. Diese unterschiedlichen Varianten können in einer bevorzugten Ausführungsform mittels spezifischer Primer oder Primersätze detektiert werden. An die Umwandlung kann sich in einer Ausführungsform ein weiterer Schritt der Aufreinigung der bisulfitierten DNA anschließen. In einer weiteren Ausführungsform wird die Bisulfitierung der DNA als weiterer Schritt von dem Verfahren der Erfindung umfasst. Vorzugsweise als erster Schritt, oder vor der quantitativen Bestimmung des Vorliegens eines differentiell methylierten Cytosins eines CpG Dinukleotids.

In einer weiteren, besonders bevorzugten Ausführungsform umfasst Schritt a) die Amplifikation der nicht-bisulfitierten DNA mit zumindest einem Primerpaar, das spezifisch für ein Transposon oder Fragment davon ist, oder alternativ die Amplifikation der bisulfitierten DNA mit zumindest einem Primerpaar das spezifisch für ein bisulfitiertes Transposon oder Fragment davon ist, wobei die Primer keine differentiell methylierte Position des Transposons umfassen, d.h. kein C oder umgewandeltes U/T eines CpG Dinukleotids umfassen; weiterhin umfasst Schritt b) die Amplifikation der bisulfitierten DNA mit zumindest einem Primerpaar, das spezifisch für das Transposon oder Fragment davon ist (welches in Schritt a) bestimmt wurde) und das zumindest einen Primer umfasst, der zumindest eine differentiell methylierte Position des Transposons umfasst, d.h. zwischen zumindest einem C eines methylierten CpG und zumindest einem U/T eines bisulfitierten unmethylierten CpG diskriminieren kann; weiterhin umfasst Schritt c) die Bestimmung des normierten DNA-Methylierungsgrads über das Verhältnis der in den Schritten a) und b) gebildeten Amplifikate.

In anderen Worten kann für die Bestimmung in Schritt a) die DNA entweder nicht-bisulfitiert (also z.B. direkt nach der Isolation aus einer Probe) oder bereits bisulfitiert vorliegen. In ersterem Fall können beliebige für das Transposon spezifische Primer zu dessen Amplifizierung verwendet werden; in letzterem Fall sollte dafür Sorge getragen werden, dass die verwendeten Primer keine differentiell methylierte Stelle umfassen (also kein Cytosin eines CpG Dinukleotids). In einer bevorzugten Ausführungsform wird in den Schritten a) und b) das gleiche Volumen von DNA eingesetzt, dabei ist die DNA bevorzugt in einem Ansatz aus einer Probe isoliert worden. Noch bevorzugter werden in Schritt a) und b) gleiche Mengen an DNA eingesetzt. Auch hierbei ist die DNA bevorzugt in einem Ansatz aus einer Probe isoliert worden.

In Schritt b), der immer mit bisulfitierter DNA ausgeführt wird, ist dies genau umgekehrt; hier sollte zumindest ein Primer zumindest eine differentiell methylierte Stelle des in Schritt a) amplifizierten Transposons umfassen (also zumindest ein Cytosin eines CpG Dinukleotids). Es spielt hierbei keine Rolle, ob der zumindest eine Primer für die zumindest eine differentiell methylierte Stelle auf dem sense oder dem antisense Strang der DNA spezifisch ist. Über diese(n) Primer kann somit eine an der untersuchten gegebenen Position bestehende oder nicht bestehende Methylierung der Ausgangs-DNA nachgewiesen werden. Wird ein Amplifikat mit Primern erhalten, die spezifisch für ein CpG sind, so lag an der betreffenden Stelle eine Methylierung der ursprünglichen DNA vor, da keine Umwandlung bei der Bisulfitierungsreaktion erfolgte. Wird ein Amplifikat mit Primern erhalten, die spezifisch für ein bisulfitiertes CpG sind, so lag an der betreffenden Stelle keine Methylierung (eine Demethylierung) der ursprünglichen DNA vor. Demgemäß kann - je nach der Art der verwendeten Primer - die Methylierung oder die Demethylierung nachgewiesen werden.

Es kann für die erfindungsgemäße Bestimmung des Methylierungsgrads entweder die Methylierung oder die Demethylierung der DNA des Transposons bestimmt werden, da diese beiden Zustände direkt miteinander korrespondieren. Wird demnach ein CpG-spezifisches Primerpaar verwendet, so wird der DNA-Methylierungsgrad über die Methylierung bestimmt; wird ein für ein bisulfitiertes CpG spezifisches Primerpaar verwendet, so wird der DNA-Methylierungsgrad über die Demethylierung bestimmt.

In einer bevorzugten Ausführungsform ist zumindest ein Primer des zumindest einen Primerpaars für zumindest eine differentiell methylierte Position des Transposons spezifisch; noch bevorzugter sind beide Primer des zumindest einen Primerpaars für zumindest eine differentiell methylierte Position des Transposons spezifisch. Dies hat den Vorteil, dass eine bessere Spezifität und verbesserte Amplifikation erreicht wird.

In weiteren bevorzugten Ausführungsformen sind die verwendeten Primer für mehr als eine differentiell methylierte Position spezifisch. Solche Primer sind für mehr als ein Cytosin eines CpG bzw. bisulfitierten CpG spezifisch. In besonders bevorzugten Ausführungsformen sind die Primer für 2, 3, 4 oder mehr als 4 differentiell methylierte Position spezifisch.

Da die Primer weiterhin spezifisch für ein Transposon sind, sind sie in einer bevorzugten Ausführungsform spezifisch für ein LINE Element, ein Alu Element, ein HERV Element, ein HERV-K Element oder einem Fragment derselben. In einer besonders bevorzugten Ausführungsform ist sind die Primer spezifisch für ein LINE-1 Element oder ein Fragment davon. Noch bevorzugter sind die Primer spezifisch für den Promotorbereich eines Transposons. Dies hat den Vorteil, dass eine hohe Frequenz von CpG vorhanden ist und somit die der Methylierungsgrad besser bestimmt werden kann. In der bevorzugtesten Ausführungsform sind die Primer spezifisch für den Promotorbereich eines LINE-1 Elements.

In einer anderen Ausführungsform sind die verwendeten Primer wenigstens 15 Nukleotide lang, bevorzugt 18, 19, 20, 21, 22, 23, 24, 25 oder mehr als 25 Nukleotide lang. Ein Primerpaar kann Primer mit verschiedener Länge umfassen. In einer bevorzugten Ausführungsform sind die Primer 18 bis 35 Nukleotide lang, in einer weiteren bevorzugten Ausführungsform sind die Primer 20-30 Nukleotide lang.

In einer bevorzugten Ausführungsform sind die Primer eines Primerpaars entweder ausschließlich für zumindest ein Cytosin eines CpG Dinukleotids oder ausschließlich für zumindest ein Cytosin eines bisulfitierten CpG Dinukleotids spezifisch.

Die Primer können das zumindest eine für eine differentiell methylierte Position spezifische Nukleotid an jeder Stelle umfassen, d.h. am 5'-Ende des Primeroligonukleotids, am 3'-Ende oder jeder Position dazwischen. In einer besonders bevorzugten Ausführungsform liegt das zumindest eine, für eine differentiell methylierte Position spezifische Nukleotid am 3'-Ende des Primeroligonukleotids. Dies hat den Vorteil einer erhöhten Spezifität.

In einer weiteren besonders bevorzugten Ausführungsform liegen die in Schritt a) und Schritt b) verwendeten Primerpaare in direkter Nachbarschaft auf dem amplifizierten Bereich des Transposons. Der Term "direkte Nachbarschaft" soll hierbei so verstanden werden, dass zwischen den von dem in Schritt a) und dem in Schritt b) amplifizierten Bereichen des Transposons eine Distanz von ≤ 6000 bp , ≤ 5000 bp, ≤ 4000 bp, ≤ 3000 bp ≤ 2000 bp, ≤ 1000 bp, ≤ 800 bp, ≤ 600 bp, ≤ 500 bp, bevorzugter < 400 bp oder < 300 bp und noch bevorzugter < 200 bp oder ≤ 100 bp liegen. In noch bevorzugteren Ausführungsformen ist diese Distanz < 80 bp, ≤ 50 bp, oder ≤ 10 bp. In weiteren noch bevorzugteren Ausführungsformen ist die Distanz 0 bp, oder die amplifizierten Bereiche überlappen.

Der Fachmann ist aufgrund seines Fachwissens in der Lage eine Vielzahl von erfindungsgemäßen Primern zu erzeugen, die für zumindest eine differentiell methylierte Position eines Transposons spezifisch sind. Dies soll im Folgenden anhand des Promotorbereichs des LINE-1 Elements beschrieben werden.

Die Nukleinsäuresequenz dieses Promotorbereichs des LINE-1 Elements (GenBank Accession M80343) ist: wobei CpG durch Großschrift hervorgehoben wurden.

Im Falle einer kompletten Methylierung dieser Promotorsequenz mit anschließender Bisulfitierung ergibt sich demnach folgende Nukleinsäuresequenz (SEQ ID NO 1): wobei die methylierten CpG und die durch die Bisulfitierung von C in U (bzw T) umgewandelten Nukleotide in Großschrift dargestellt sind.

Im Falle einer kompletten Demethylierung dieser Promotorsequenz mit anschließender Bisulfitierung ergibt sich demnach folgende Nukleinsäuresequenz (SEQ ID NO 2): wobei die demethylierten (und umgewandelten) CpG und die durch die Bisulfitierung von C in U (bzw T) umgewandelten Nukleotide in Großschrift dargestellt sind.

Aufgrund von SEQI ID NO 1 und SEQ ID NO 2 können demnach Primer zur Diskriminierung zwischen an zumindest einer Stelle differentiell methylierter DNA ausgewählt werden. Selbstverständlich ist dem Fachmann geläufig, dass es auch einen antisense Strang zu dem gezeigten sense Strang gibt. Hier sind zu den 5'-CpG-3' Dinukleotiden korrespondierende 5'-CpG-3' Dinukleotide vorhanden, die ebenfalls differentiell methyliert vorliegen. Demnach können auch Primer anhand der Sequenzinformation des antisense Stranges ausgewählt werden.

Da nach der Bisulfitierungsreaktion sense und antisense Strang nicht mehr komplementär zueinander sind, können so bei Vorliegen einer differentiell methylierten Position zunächst vier verschiedene spezifische Primer generiert werden: 1) sequenzidentisch und spezifisch für den konvertierten sense Strang, 2) komplementär und spezifisch für den konvertierten sense Strang, 3) sequenzidentisch und spezifisch für den konvertierten antisense Strang, 4) komplementär und spezifisch für den konvertierten antisense Strang. Da die differentiell methylierte Position in zwei Zuständen vorliegen kann, ergeben sich somit acht mögliche Primer.

Als Beispiel sei ausgegangen von der doppelsträngigen DNA Sequenz:
5'-AGCACGT-3' (sense)
3 '-TCGTGCA-5' (antisense)

Nach der Bisulfitierungsreaktion ergeben sich hieraus je nach Methylierungszustand jeweils die zwei nicht mehr komplementären Stränge:

| | |
|---|---|
| Methyliert: | 5'-AGUACGT-3' und 3'-TUGTGCA-5'; |
| Demethyliert: | 5'-AGUAUGT-3' und 3'-TUGTGUA-5'. |

Für jede dieser 4 Sequenzen kann nun für die Amplifikation ein sequenzidentischer Primer und ein Primer erzeugt werden, der komplementär zu der Sequenz ist, also:

| | |
|---|---|
| Methyliert: | |
| 5'-AGUACGT-3' und 5'-ACGTACT-3', | 5'-ACGTGUT-3' und 5'-AACACGT-3', |
| Demethyliert: | |
| 5'-AGUAUGT-3' und 5'-ACATACT-3', | 5'-AUGTGUT-3' und 5'-AACACAT-3'. |

Beispiele und noch bevorzugtere Ausführungsformen für solche Primer, die für ein oder mehrere (bisulfitierte) Cytosine von CpG Dinukleotiden spezifisch sind und damit für zumindest eine differentiell methylierte Position eines Transposons spezifisch sind, sind in SEQ ID NO 3 - 1048 oder den Tabellen 1 - 12 gegeben. Hierbei geben Tabellen 1 - 4 besonders bevorzugte Primer für das LINE-1 Element, Tabellen 5 - 8 besonders bevorzugte Primer für das Alu Element und Tabellen 9 - 12 besonders bevorzugte Primer für das HERV-K Element an. Die Primer sind in 5' - 3' Orientierung angegeben.

In bevorzugten Ausführungsformen bezieht sich die Erfindung auf folgende solcher Oligonukleotide und deren Verwendung in den erfindungsgemäßen Verfahren:
Sequenzidentische oder komplementäre Primersequenzen, die spezifisch für den Bisulfit-konvertierten methylierten oder demethylierten sense oder antisense Strang des Promotorbereichs des LINE-1 Elements sind, d.h. SEQ ID NO 3 - 436; bevorzugter SEQ ID NO 3 - 112, oder SEQ ID NO 113 - 220, oder SEQ ID NO 221 - 336, oder SEQ ID NO 337 - 436; noch bevorzugter SEQ ID NO 3 - 57, oder SEQ ID NO 58 - 112, oder SEQ ID NO 113 - 166, oder SEQ ID NO 167 - 220, oder SEQ ID NO 221 - 278, oder SEQ ID NO 279 - 336, oder SEQ ID NO 337 - 386, oder SEQ ID NO 387 - 436.

Sequenzidentische oder komplementäre Primersequenzen, die spezifisch für den Bisulfit-konvertierten methylierten oder demethylierten sense oder antisense Strang des Alu Elements sind, d.h. SEQ ID NO 437 - 612; bevorzugter SEQ ID NO 437 -476, oder SEQ ID NO 477 - 522, oder SEQ ID NO 523 - 570, oder SEQ ID NO 571 - 612; noch bevorzugter SEQ ID NO 437 - 456, oder SEQ ID NO 457 - 476, oder SEQ ID NO 477 - 499, oder SEQ ID NO 500 - 522, oder SEQ ID NO 523 - 546, oder SEQ ID NO 547 - 570, oder SEQ ID NO 571 - 591, oder SEQ ID NO 592 - 612.

Sequenzidentische oder komplementäre Primersequenzen, die spezifisch für den Bisulfit-konvertierten methylierten oder demethylierten sense oder antisense Strang des HERV-K Elements sind, d.h. SEQ ID NO 613 - 1048; bevorzugter SEQ ID NO 613 - 708, oder SEQ ID NO 709 - 796, oder SEQ ID NO 797 - 922, oder SEQ ID NO 923 - 1048; noch bevorzugter SEQ ID NO 613 - 660, oder SEQ ID NO 661 - 708, oder SEQ ID NO 709 - 752, oder SEQ ID NO 753 - 796, oder SEQ ID NO 797 - 859, oder SEQ ID NO 860 - 922, oder SEQ ID NO 923 - 985, oder SEQ ID NO 986 - 1048.

**Tabelle 1: Bevorzugte sequenzidentische Primersequenzen, spezifisch für den Bisulfit-konvertierten methylierten bzw. demethylierten sense Strang des Promotorbereichs des LINE-1 Elements.**

| Methyliert | SEQ ID NO | Demethyliert | SEQ ID NO |
|---|---|---|---|
| GGGGAGGAGTTAAGATGGTC | 3 | GGGGAGGAGTTAAGATGGTT | 58 |
| GGTCGAATAGGAATAGTTTC | 4 | GGTTGAATAGGAATAGTTTT | 59 |
| TTCGGTTTATAGTTTTTAGC | 5 | TTTGGTTTATAGTTTTTAGT | 60 |
| TTATAGTTTTTAGCGTGAGC | 6 | TTATAGTTTTTAGTGTGAGT | 61 |
| TAGTTTTTAGCGTGAGCGAC | 7 | TAGTTTTTAGTGTGAGTGAT | 62 |
| GCGTGAGCGACGTAGAAGAC | 8 | GTGTGAGTGATGTAGAAGAT | 63 |
| GTATTTTTATTTGAGGTATC | 9 | GTATTTTTATTTGAGGTATT | 64 |
| GGGAGTGTTAGATAGTGGGC | 10 | GGGAGTGTTAGATAGTGGGT | 65 |
| GCGTAGGTTAGTGTGTGTGC | 11 | GTGTAGGTTAGTGTGTGTGT | 66 |
| GGTTAGTGTGTGTGCGTATC | 12 | GGTTAGTGTGTGTGTGTATT | 67 |
| AGTGTGTGTGCGTATCGTGC | 13 | AGTGTGTGTGTGTATTGTGT | 68 |
| TGTGTGTGCGTATCGTGCGC | 14 | TGTGTGTGTGTATTGTGTGT | 69 |
| GTGCGTATCGTGCGCGAGTC | 15 | GTGTGTATTGTGTGTGAGTT | 70 |
| TGCGCGAGTCGAAGTAGGGC | 16 | TGTGTGAGTTGAAGTAGGGT | 71 |
| TATTGTTTTATTTGGGAAGC | 17 | TATTGTTTTATTTGGGAAGT | 72 |
| GAGTTAAAGAAAGGGGTGAC | 18 | GAGTTAAAGAAAGGGGTGAT | 73 |
| TAAAGAAAGGGGTGACGGTC | 19 | TAAAGAAAGGGGTGATGGTT | 74 |
| ACGGTCGTATTTGGAAAATC | 20 | ATGGTTGTATTTGGAAAATT | 75 |
| AAATCGGGTTATTTTTATTC | 21 | AAATTGGGTTATTTTTATTT | 76 |
| TATTTTTATTCGAATATTGC | 22 | TATTTTTATTTGAATATTGT | 77 |
| AATATTGCGTTTTTTAGATC | 23 | AATATTGTGTTTTTTAGATT | 78 |
| TTTAGATCGGTTTAAGAAAC | 24 | TTTAGATTGGTTTAAGAAAT | 79 |
| AGATCGGTTTAAGAAACGGC | 25 | AGATTGGTTTAAGAAATGGT | 80 |
| TTTAAGAAACGGCGTATTAC | 26 | TTTAAGAAATGGTGTATTAT | 81 |
| TTATATTTTATATTTGGTTC | 27 | TTATATTTTATATTTGGTTT | 82 |
| TTTGGTTCGGAGGGTTTTAC | 28 | TTTGGTTTGGAGGGTTTTAT | 83 |
| TCGGAGGGTTTTACGTTTAC | 29 | TTGGAGGGTTTTATGTTTAT | 84 |
| TTTTACGTTTACGGAATTTC | 30 | TTTTATGTTTATGGAATTTT | 85 |
| TTGAGATTAAATTGTAAGGC | 31 | TTGAGATTAAATTGTAAGGT | 86 |
| TTAAATTGTAAGGCGGTAAC | 32 | TTAAATTGTAAGGTGGTAAT | 87 |
| AACGAGGTTGGGGGAGGGGC | 33 | AATGAGGTTGGGGGAGGGGT | 88 |
| AGGTTGGGGGAGGGGCGTTC | 34 | AGGTTGGGGGAGGGGTGTTT | 89 |
| TTTAGGTAAATAAAGTAGTC | 35 | TTTAGGTAAATAAAGTAGTT | 90 |
| ATAAAGTAGTCGGGAAGTTC | 36 | ATAAAGTAGTTGGGAAGTTT | 91 |
| AGTAGTGGTTTTTTTAGTAC | 37 | AGTAGTGGTTTTTTTAGTAT | 92 |
| GTAGTTGGAGATTTGAGAAC | 38 | GTAGTTGGAGATTTGAGAAT | 93 |
| GTTTTTGATTTTTGATTTTC | 39 | GTTTTTGATTTTTGATTTTT | 94 |
| GGTATATTGATATTTTATAC | 40 | GGTATATTGATATTTTATAT | 95 |
| TTAGAAAGGATATTTATATC | 41 | TTAGAAAGGATATTTATATT | 96 |
| AAAATTGGAAATTTTAAAAC | 42 | AAAATTGGAAATTTTAAAAT | 97 |
| GAAATTTTAAAACGTAGAGC | 43 | GAAATTTTAAAATGTAGAGT | 98 |
| TTTTTTTTTTTTAAAGGAAC | 44 | TTTTTTTTTTTTAAAGGAAT | 99 |
| GGATGGAGAATGATTTTGAC | 45 | GGATGGAGAATGATTTTGAT | 100 |
| GAGAGAAGAAGGTTTTAGAC | 46 | GAGAGAAGAAGGTTTTAGAT | 101 |
| ATTAAATTATTTTGAGTTAC | 47 | ATTAAATTATTTTGAGTTAT | 102 |
| GGAGTTGAAAATTAAGGTTC | 48 | GGAGTTGAAAATTAAGGTTT | 103 |
| AATTAAGGTTCGAGAATTAC | 49 | AATTAAGGTTTGAGAATTAT | 104 |
| ATGTAGAAGTTTTAGGAGTC | 50 | ATGTAGAAGTTTTAGGAGTT | 105 |
| GAAGTTTTAGGAGTCGATGC | 51 | GAAGTTTTAGGAGTTGATGT | 106 |
| TGAAATGAATGAAATGAAGC | 52 | TGAAATGAATGAAATGAAGT | 107 |
| TGTGAAAAGATTAAATTTAC | 53 | TGTGAAAAGATTAAATTTAT | 108 |
| ATTTAGTAAGGTAGGTTAAC | 54 | ATTTAGTAAGGTAGGTTAAT | 109 |
| ATTTAGGAAATATAGAGAAC | 55 | ATTTAGGAAATATAGAGAAT | 110 |
| GTTATAAAGATATTTTTC | 56 | GTTATAAAGATATTTTTT | 111 |
| GGTAGTTAGAGAGAAAGGTC | 57 | GGTAGTTAGAGAGAAAGGTT | 112 |

**Tabelle 2: Bevorzugte komplementäre Primersequenzen, spezifisch für den Bisulfit-konvertierten methylierten bzw. demethylierten sense Strang des Promotorbereichs des LINE-1 Elements.**

| Methyliert | SEQ ID NO | Demethyliert | SEQ ID NO |
|---|---|---|---|
| TTTCCTTTAAAAATAACCCG | 113 | TTTCCTTTAAAAATAACCCA | 167 |
| TCTTAAAATTACTCTTCTCG | 114 | TCTTAAAATTACTCTTCTCA | 168 |
| CGAAAAATATCTTTATAACG | 115 | CAAAAAATATCTTTATAACA | 169 |
| ATATTTCCTAAATCTAAACG | 116 | ATATTTCCTAAATCTAAACA | 170 |
| TCAAATACACCAATCAAACG | 117 | TCAAATACACCAATCAAACA | 171 |
| TCTCTAAACTTCCCTTCTCG | 118 | TCTCTAAACTTCCCTTCTCA | 172 |
| CCCTTTCTTCCAATTAATCG | 119 | CCCTTTCTTCCAATTAATCA | 173 |
| TCTTCCAATTAATCGCATCG | 120 | TCTTCCAATTAATCACATCA | 174 |
| AAACTTCTACATTCTTCACG | 121 | AAACTTCTACATTCTTCACA | 175 |
| CATTCTTCACGTAATTCTCG | 122 | CATTCTTCACATAATTCTCA | 176 |
| TTAATTTAAATATCCTCCCG | 123 | TTAATTTAAATATCCTCCCA | 177 |
| AACTCAAAATAATTTAATCG | 124 | AACTCAAAATAATTTAATCA | 178 |
| AACCTTCTTCTCTCAACTCG | 125 | AACCTTCTTCTCTCAACTCA | 179 |
| ATTACTAATAAAAAACTACG | 126 | ATTACTAATAAAAAACTACA | 180 |
| CCTTTAAAAAAAAAAAAACG | 127 | CCTTTAAAAAAAAAAAAACA | 181 |
| AAAAAAAAAAACGCTCTACG | 128 | AAAAAAAAAAACACTCTACA | 182 |
| AATATACAAATAAATTTTCG | 129 | AATATACAAATAAATTTTCA | 183 |
| TCTATTAAAATACCCTACCG | 130 | TCTATTAAAATACCCTACCA | 184 |
| CCTCCCAATTAAACTACTCG | 131 | CCTCCCAATTAAACTACTCA | 185 |
| AAAAACAATCTATCTACCCG | 132 | AAAAACAATCTATCTACCCA | 186 |
| TTCTCAAATCTCCAACTACG | 133 | TTCTCAAATCTCCAACTACA | 187 |
| AATAAACTCCACCCAATTCG | 134 | AATAAACTCCACCCAATTCA | 188 |
| CACCCAATTCGAACTTCCCG | 135 | CACCCAATTCAAACTTCCCA | 189 |
| AACCTAAACAATAACGAACG | 136 | AACCTAAACAATAACAAACA | 190 |
| ACGCCCCTCCCCCAACCTCG | 137 | ACACCCCTCCCCCAACCTCA | 191 |
| CTCCCCCAACCTCGTTACCG | 138 | CTCCCCCAACCTCATTACCA | 192 |
| TACTATACTAACAATCAACG | 139 | TACTATACTAACAATCAACA | 193 |
| TAACAATCAACGAAATTCCG | 140 | TAACAATCAACAAAATTCCA | 194 |
| TCAACGAAATTCCGTAAACG | 141 | TCAACAAAATTCCATAAACA | 195 |
| CGTAAACGTAAAACCCTCCG | 142 | CATAAACATAAAACCCTCCA | 196 |
| AAATATAAAATATAATCTCG | 143 | AAATATAAAATATAATCTCA | 197 |
| AAATATAATCTCGTAATACG | 144 | AAATATAATCTCATAATACA | 198 |
| TATAATCTCGTAATACGCCG | 145 | TATAATCTCATAATACACCA | 199 |
| ATACGCCGTTTCTTAAACCG | 146 | ATACACCATTTCTTAAACCA | 200 |
| TTAAACCGATCTAAAAAACG | 147 | TTAAACCAATCTAAAAAACA | 201 |
| TCTAAAAAACGCAATATTCG | 148 | TCTAAAAAACACAATATTCA | 202 |
| ATTCGAATAAAAATAACCCG | 149 | ATTCAAATAAAAATAACCCA | 203 |
| AACCCGATTTTCCAAATACG | 150 | AACCCAATTTTCCAAATACA | 204 |
| CGATTTTCCAAATACGACCG | 151 | CAATTTTCCAAATACAACCA | 205 |
| AAACTCCCTAACCCCTTACG | 152 | AAACTCCCTAACCCCTTACA | 206 |
| CCAAATAAAACAATACCTCG | 153 | CCAAATAAAACAATACCTCA | 207 |
| CAATACCTCGCCCTACTTCG | 154 | CAATACCTCACCCTACTTCA | 208 |
| CCTCGCCCTACTTCGACTCG | 155 | CCTCACCCTACTTCAACTCA | 209 |
| TCGCCCTACTTCGACTCGCG | 156 | TCACCCTACTTCAACTCACA | 210 |
| CCTACTTCGACTCGCGCACG | 157 | CCTACTTCAACTCACACACA | 211 |
| TTCGACTCGCGCACGATACG | 158 | TTCAACTCACACACAATACA | 212 |
| CGCACACACACTAACCTACG | 159 | CACACACACACTAACCTACA | 213 |
| TCCCTAATAAAATAAACCCG | 160 | TCCCTAATAAAATAAACCCA | 214 |
| TAAAAATACAAAAATCACCG | 161 | TAAAAATACAAAAATCACCA | 215 |
| AAAAATCACCGTCTTCTACG | 162 | AAAAATCACCATCTTCTACA | 216 |
| AATCACCGTCTTCTACGTCG | 163 | AATCACCATCTTCTACATCA | 217 |
| CGTCTTCTACGTCGCTCACG | 164 | CATCTTCTACATCACTCACA | 218 |
| ACGCTAAAAACTATAAACCG | 165 | ACACTAAAAACTATAAACCA | 219 |
| ACCGAAACTATTCCTATTCG | 166 | ACCAAAACTATTCCTATTCA | 220 |

**Tabelle 3: Bevorzugte sequenzidentischen Primersequenzen, spezifisch für den Bisulfit-konvertierten methylierten bzw. demethylierten antisense Strang des Promotorbereichs des LINE-1 Elements.**

| Methyliert | SEQ ID NO | Demethyliert | SEQ ID NO |
|---|---|---|---|
| TGTAGTTTTTTTTTAGTTTC | 221 | TGTAGTTTTTTTTTAGTTTT | 279 |
| TTTTGGTATGATTTTGTAGC | 222 | TTTTGGTATGATTTTGTAGT | 280 |
| ATTTTGTAGCGGTTGGTATC | 223 | ATTTTGTAGTGGTTGGTATT | 281 |
| TGGTTTGTAGGGTTTTTGTC | 224 | TGGTTTGTAGGGTTTTTGTT | 282 |
| TTTTTTTTTGAGGGTAATTC | 225 | TTTTTTTTTGAGGGTAATTT | 283 |
| GTTTTGGAGTTGTTTTTTTC | 226 | GTTTTGGAGTTGTTTTTTTT | 284 |
| TGTATTTTTTGAATTTGAAC | 227 | TGTATTTTTTGAATTTGAAT | 285 |
| TTTAGGTATATTAATTAGAC | 228 | TTTAGGTATATTAATTAGAT | 286 |
| TTTTTTAAATTTTTTTTTTC | 229 | TTTTTTAAATTTTTTTTTTT | 287 |
| ATTTTTTTTTTTAGTTGATC | 230 | ATTTTTTTTTTTAGTTGATT | 288 |
| TTTTTTTAGTTGATCGTATC | 231 | TTTTTTTAGTTGATTGTATT | 289 |
| GAGGTTTTTGTATTTTTTAC | 232 | GAGGTTTTTGTATTTTTTAT | 290 |
| GTATTTTTTACGTAGTTTTC | 233 | GTATTTTTTATGTAGTTTTT | 291 |
| TTTGGTTTGAATGTTTTTTC | 234 | TTTGGTTTGAATGTTTTTTT | 292 |
| TAGTTTAGAGTAATTTGATC | 235 | TAGTTTAGAGTAATTTGATT | 293 |
| AAGTTTTTTTTTTTTAGTTC | 236 | AAGTTTTTTTTTTTTAGTTT | 294 |
| TGTTGTTGGTGAGGAATTGC | 237 | TGTTGTTGGTGAGGAATTGT | 295 |
| TTTTTTGGAGGAGGAGAGGC | 238 | TTTTTTGGAGGAGGAGAGGT | 296 |
| GAGGAGGAGAGGCGTTTTGC | 239 | GAGGAGGAGAGGTGTTTTGT | 297 |
| TGATGTATAGATGGGTTTTC | 240 | TGATGTATAGATGGGTTTTT | 298 |
| GTTTGTTGGAATATTTTGTC | 241 | GTTTGTTGGAATATTTTGTT | 299 |
| GTTTTTTAGTTAGGTTGTTC | 242 | GTTTTTTAGTTAGGTTGTTT | 300 |
| AGGAGGTAGTTTGTTTGTTC | 243 | AGGAGGTAGTTTGTTTGTTT | 301 |
| GTTTTTAGATTTTTAGTTGC | 244 | GTTTTTAGATTTTTAGTTGT | 302 |
| TGGTGGGTTTTATTTAGTTC | 245 | TGGTGGGTTTTATTTAGTTT | 303 |
| TTATTTAGTTCGAGTTTTTC | 246 | TTATTTAGTTTGAGTTTTTT | 304 |
| AAGTAAGTTTGGGTAATGGC | 247 | AAGTAAGTTTGGGTAATGGT | 305 |
| AAGTTTGGGTAATGGCGGGC | 248 | AAGTTTGGGTAATGGTGGGT | 306 |
| GGCGTTTTTTTTTTAGTTTC | 249 | GGTGTTTTTTTTTTAGTTTT | 307 |
| TTTTTTTTAGTTTCGTTGTC | 250 | TTTTTTTTAGTTTTGTTGTT | 308 |
| TTGTTGTGTTAGTAATTAGC | 251 | TTGTTGTGTTAGTAATTAGT | 309 |
| TTAGTAATTAGCGAGATTTC | 252 | TTAGTAATTAGTGAGATTTT | 310 |
| ATTAGCGAGATTTCGTGGGC | 253 | ATTAGTGAGATTTTGTGGGT | 311 |
| TCGTGGGCGTAGGATTTTTC | 254 | TTGTGGGTGTAGGATTTTTT | 312 |
| TAGGTGTGGGATATAGTTTC | 255 | TAGGTGTGGGATATAGTTTT | 313 |
| GGGATATAGTTTCGTGGTGC | 256 | GGGATATAGTTTTGTGGTGT | 314 |
| ATATAGTTTCGTGGTGCGTC | 257 | ATATAGTTTTGTGGTGTGTT | 315 |
| GGTGCGTCGTTTTTTAAGTC | 258 | GGTGTGTTGTTTTTTAAGTT | 316 |
| TTTAAGTCGGTTTGAAAAGC | 259 | TTTAAGTTGGTTTGAAAAGT | 317 |
| GTTTGAAAAGCGTAATATTC | 260 | GTTTGAAAAGTGTAATATTT | 318 |
| TATTCGGGTGGGAGTGATTC | 261 | TATTTGGGTGGGAGTGATTT | 319 |
| TGATTCGATTTTTTAGGTGC | 262 | TGATTTGATTTTTTAGGTGT | 320 |
| TCGATTTTTTAGGTGCGATC | 263 | TTGATTTTTTAGGTGTGATT | 321 |
| GGAATTTTTTGATTTTTTGC | 264 | GGAATTTTTTGATTTTTTGT | 322 |
| TTTAGGTGAGGTAATGTTTC | 265 | TTTAGGTGAGGTAATGTTTT | 323 |
| GTAATGTTTCGTTTTGTTTC | 266 | GTAATGTTTTGTTTTGTTTT | 324 |
| GTTTCGTTTTGTTTCGGTTC | 267 | GTTTTGTTTTGTTTTGGTTT | 325 |
| TTCGTTTTGTTTCGGTTCGC | 268 | TTTGTTTTGTTTTGGTTTGT | 326 |
| TTTTGTTTCGGTTCGCGTAC | 269 | TTTTGTTTTGGTTTGTGTAT | 327 |
| TTTCGGTTCGCGTACGGTGC | 270 | TTTTGGTTTGTGTATGGTGT | 328 |
| GCGTATATATATTGGTTTGC | 271 | GTGTATATATATTGGTTTGT | 329 |
| TTTTTTAGTGAGATGAATTC | 272 | TTTTTTAGTGAGATGAATTT | 330 |
| ATGGAAATGTAGAAATTATC | 273 | ATGGAAATGTAGAAATTATT | 331 |
| TAGAAATTATCGTTTTTTGC | 274 | TAGAAATTATTGTTTTTTGT | 332 |
| AAATTATCGTTTTTTGCGTC | 275 | AAATTATTGTTTTTTGTGTT | 333 |
| TCGTTTTTTGCGTCGTTTAC | 276 | TTGTTTTTTGTGTTGTTTAT | 334 |
| TACGTTGGGAGTTGTAGATC | 277 | TATGTTGGGAGTTGTAGATT | 335 |
| GATCGGAGTTGTTTTTATTC | 278 | GATTGGAGTTGTTTTTATTT | 336 |

**Tabelle 4: Bevorzugte komplementäre Primersequenzen, spezifisch für den Bisulfit-konvertierten methylierten bzw. demethylierten antisense Strang des Promotorbereichs des Line-1 Elements.**

| Methyliert | SEQ ID NO | Demethyliert | SEQ ID NO |
|---|---|---|---|
| AAAAAAAAACCAAAATAACCG | 337 | AAAAAAAAACCAAAATAACCA | 387 |
| AACCGAATAAAAACAACTCCG | 338 | AACCAAATAAAAACAACTCCA | 388 |
| TCCGATCTACAACTCCCAACG | 339 | TCCAATCTACAACTCCCAACA | 389 |
| CTACAACTCCCAACGTAAACG | 340 | CTACAACTCCCAACATAAACA | 390 |
| CAACTCCCAACGTAAACGACG | 341 | CAACTCCCAACATAAACAACA | 391 |
| ACGTAAACGACGCAAAAAACG | 342 | ACATAAACAACACAAAAAACA | 392 |
| ACATTTCCATCTAAAATACCG | 343 | ACATTTCCATCTAAAATACCA | 393 |
| AAAAATACCAAACAATAAACG | 344 | AAAAATACCAAACAATAAACA | 394 |
| ACGCAAACCAATATATATACG | 345 | ACACAAACCAATATATATACA | 395 |
| AACCAATATATATACGCACCG | 346 | AACCAATATATATACACACCA | 396 |
| AATATATATACGCACCGTACG | 347 | AATATATATACACACCATACA | 397 |
| TATATATACGCACCGTACGCG | 348 | TATATATACACACCATACACA | 398 |
| ATACGCACCGTACGCGAACCG | 349 | ATACACACCATACACAAACCA | 399 |
| TACGCGAACCGAAACAAAACG | 350 | TACACAAACCAAAACAAAACA | 400 |
| CATTACCTCACCTAAAAAACG | 351 | CATTACCTCACCTAAAAAACA | 401 |
| AAATCAAAAAAAAAAATAACG | 352 | AAATCAAAAAAAAAAATAACA | 402 |
| CAAAAAAAAAAATAACGATCG | 353 | CAAAAAAAAAAATAACAATCA | 403 |
| ACGATCGCACCTAAAAAATCG | 354 | ACAATCACACCTAAAAAATCA | 404 |
| AAATCGAATCACTCCCACCCG | 355 | AAATCAAATCACTCCCACCCA | 405 |
| CACTCCCACCCGAATATTACG | 356 | CACTCCCACCCAAATATTACA | 406 |
| AATATTACGCTTTTCAAACCG | 357 | AATATTACACTTTTCAAACCA | 407 |
| TTCAAACCGACTTAAAAAACG | 358 | TTCAAACCAACTTAAAAAACA | 408 |
| AAACCGACTTAAAAAACGACG | 359 | AAACCAACTTAAAAAACAACA | 409 |
| CTTAAAAAACGACGCACCACG | 360 | CTTAAAAAACAACACACCACA | 410 |
| CTATATCCCACACCTAACTCG | 361 | CTATATCCCACACCTAACTCA | 411 |
| CCTAACTCGAAAAATCCTACG | 362 | CCTAACTCAAAAAATCCTACA | 412 |
| TCGAAAAATCCTACGCCCACG | 363 | TCAAAAAATCCTACACCCACA | 413 |
| TCCTACGCCCACGAAATCTCG | 364 | TCCTACACCCACAAAATCTCA | 414 |
| CTAAAATCAAACTACAAAACG | 365 | CTAAAATCAAACTACAAAACA | 415 |
| TCAAACTACAAAACGACAACG | 366 | TCAAACTACAAAACAACAACA | 416 |
| AACGAAACTAAAAAAAAAACG | 367 | AACAAAACTAAAAAAAAAACA | 417 |
| AAACTAAAAAAAAAACGCCCG | 368 | AAACTAAAAAAAAAACACCCA | 418 |
| CTTAAATAAACAAAACAACCG | 369 | CTTAAATAAACAAAACAACCA | 419 |
| ACAAAACAACCGAAAAACTCG | 370 | ACAAAACAACCAAAAAACTCA | 420 |
| AACAATAATTCTCCCAACACG | 371 | AACAATAATTCTCCCAACACA | 421 |
| GCAACTAAAAATCTAAAAACG | 372 | GCAACTAAAAATCTAAAAACA | 422 |
| ATCCCTAACTCCTAACCCCCG | 373 | ATCCCTAACTCCTAACCCCCA | 423 |
| AACACACTAACACCTCACACG | 374 | AACACACTAACACCTCACACA | 424 |
| CCAAAAAAAACATCTACACCG | 375 | CCAAAAAAAACATCTACACCA | 425 |
| AAAACTAAAAACTCTAAAACG | 376 | AAAACTAAAAACTCTAAAACA | 426 |
| AAAACTCTAAAACGCAAAACG | 377 | AAAACTCTAAAACACAAAACA | 427 |
| CTCTCCTCCTCCAAAAAAACG | 378 | CTCTCCTCCTCCAAAAAAACA | 428 |
| AAATAAAAAATAATTTTAACG | 379 | AAATAAAAAATAATTTTAACA | 429 |
| AAAAAAAAAAAACTTCAAACG | 380 | AAAAAAAAAAAACTTCAAACA | 430 |
| ATCAAATTACTCTAAACTACG | 381 | ATCAAATTACTCTAAACTACA | 431 |
| AAAACTAAAAACCAAAACTCG | 382 | AAAACTAAAAACCAAAACTCA | 432 |
| AACCAAAACTCGAAAACTACG | 383 | AACCAAAACTCAAAAACTACA | 433 |
| ATACAAAAACCTCAAAAACCG | 384 | ATACAAAAACCTCAAAAACCA | 434 |
| AAAACCTCAAAAACCGATACG | 385 | AAAACCTCAAAAACCAATACA | 435 |
| TAAAATAAATAAAATAAAACG | 386 | TAAAATAAATAAAATAAAACA | 436 |

**Tabelle 5: Bevorzugte sequenzidentische Primersequenzen, spezifisch für den Bisulfit-konvertierten methylierten bzw. demethylierten sense Strang des Alu Elements.**

| Methyliert | SEQ ID NO | Demethyliert | SEQ ID NO |
|---|---|---|---|
| GGTCGGGCGCGGTGGTTTAC | 437 | GGTTGGGTGTGGTGGTTTAT | 457 |
| TTTTAGTATTTTGGGAGGTC | 438 | TTTTAGTATTTTGGGAGGTT | 458 |
| GTATTTTGGGAGGTCGAGGC | 439 | GTATTTTGGGAGGTTGAGGT | 459 |
| TTTGGGAGGTCGAGGCGGGC | 440 | TTTGGGAGGTTGAGGTGGGT | 460 |
| TTATTTGAGGTTAGGAGATC | 441 | TTATTTGAGGTTAGGAGATT | 461 |
| GGTTAATATGGTGAAATTTC | 442 | GGTTAATATGGTGAAATTTT | 462 |
| TAAAAATATAAAAATTAGTC | 443 | TAAAAATATAAAAATTAGTT | 463 |
| AATATAAAAATTAGTCGGGC | 444 | AATATAAAAATTAGTTGGGT | 464 |
| AATTAGTCGGGCGTGGTGGC | 445 | AATTAGTTGGGTGTGGTGGT | 465 |
| TTAGTCGGGCGTGGTGGCGC | 446 | TTAGTTGGGTGTGGTGGTGT | 466 |
| AGTCGGGCGTGGTGGCGCGC | 447 | AGTTGGGTGTGGTGGTGTGT | 467 |
| GTTTGTAATTTTAGTTATTC | 448 | GTTTGTAATTTTAGTTATTT | 468 |
| GAGGTTGAGGTAGGAGAATC | 449 | GAGGTTGAGGTAGGAGAATT | 469 |
| TAGGAGAATCGTTTGAATTC | 450 | TAGGAGAATTGTTTGAATTT | 470 |
| ATCGTTTGAATTCGGGAGGC | 451 | ATTGTTTGAATTTGGGAGGT | 471 |
| GGTTGTAGTGAGTCGAGATC | 452 | GGTTGTAGTGAGTTGAGATT | 472 |
| TTGTAGTGAGTCGAGATCGC | 453 | TTGTAGTGAGTTGAGATTGT | 473 |
| TATTGTATTTTAGTTTGGGC | 454 | TATTGTATTTTAGTTTGGGT | 474 |
| TTTAGTTTGGGCGATAGAGC | 455 | TTTAGTTTGGGTGATAGAGT | 475 |
| GGGCGATAGAGCGAGATTTC | 456 | GGGTGATAGAGTGAGATTTT | 476 |

**Tabelle 6: Bevorzugte komplementäre Primersequenzen, spezifisch für den Bisulfit-konvertierten methylierten bzw. demethylierten sense Strang des Alu Elements.**

| Methyliert | SEQ ID NO | Demethyliert | SEQ ID NO |
|---|---|---|---|
| TTTTTTAAAACGAAATCTCG | 477 | TTTTTTAAAACAAAATCTCA | 500 |
| AACGAAATCTCGCTCTATCG | 478 | AACAAAATCTCACTCTATCA | 501 |
| CAAACTAAAATACAATAACG | 479 | CAAACTAAAATACAATAACA | 502 |
| AACTAAAATACAATAACGCG | 480 | AACTAAAATACAATAACACA | 503 |
| AATACAATAACGCGATCTCG | 481 | AATACAATAACACAATCTCA | 504 |
| TCGACTCACTACAACCTCCG | 482 | TCAACTCACTACAACCTCCA | 505 |
| ACTACAACCTCCGCCTCCCG | 483 | ACTACAACCTCCACCTCCCA | 506 |
| CCGCCTCCCGAATTCAAACG | 484 | CCACCTCCCAAATTCAAACA | 507 |
| TCTCCTACCTCAACCTCCCG | 485 | TCTCCTACCTCAACCTCCCA | 508 |
| AATAACTAAAATTACAAACG | 486 | AATAACTAAAATTACAAACA | 509 |
| TAACTAAAATTACAAACGCG | 487 | TAACTAAAATTACAAACACA | 510 |
| ACTAAAATTACAAACGCGCG | 488 | ACTAAAATTACAAACACACA | 511 |
| TACAAACGCGCGCCACCACG | 489 | TACAAACACACACCACCACA | 512 |
| AACGCGCGCCACCACGCCCG | 490 | AACACACACCACCACACCCA | 513 |
| TTATATTTTTAATAAAAACG | 491 | TTATATTTTTAATAAAAACA | 514 |
| TATTAACCAAAATAATCTCG | 492 | TATTAACCAAAATAATCTCA | 515 |
| TCCTAACCTCAAATAATCCG | 493 | TCCTAACCTCAAATAATCCA | 516 |
| AACCTCAAATAATCCGCCCG | 494 | AACCTCAAATAATCCACCCA | 517 |
| CAAATAATCCGCCCGCCTCG | 495 | CAAATAATCCACCCACCTCA | 518 |
| AAATACTAAAATTACAAACG | 496 | AAATACTAAAATTACAAACA | 519 |
| ATTACAAACGTAAACCACCG | 497 | ATTACAAACATAAACCACCA | 520 |
| TACAAACGTAAACCACCGCG | 498 | TACAAACATAAACCACCACA | 521 |
| AACGTAAACCACCGCGCCCG | 499 | AACGTAAACCACCGCGCCCA | 522 |

**Tabelle 7: Bevorzugte sequenzidentische Primersequenzen, spezifisch für den Bisulfit-konvertierten methylierten bzw. demethylierten antisense Strang des Alu Elements.**

| Methyliert | SEQ ID NO | Demethyliert | SEQ ID NO |
|---|---|---|---|
| TTTTTTTGAGACGGAGTTTC | 523 | TTTTTTTGAGACTGAGTTTT | 547 |
| AGACGGAGTTTCGTTTTGTC | 524 | AGACTGAGTTTCTTTTTGTT | 548 |
| AGACGGAGTTTCGTTTTGTC | 525 | AGACTGAGTTTCTTTTTGTT | 549 |
| TTAGGTTGGAGTGTAGTGGC | 526 | TTAGGTTGGAGTGTAGTGGT | 550 |
| AGGTTGGAGTGTAGTGGCGC | 527 | AGGTTGGAGTGTAGTGGCTT | 551 |
| GAGTGTAGTGGCGCGATTTC | 528 | GAGTGTAGTGGCTCTATTTT | 552 |
| TTCGGTTTATTGTAATTTTC | 529 | TTCTGTTTATTGTAATTTTT | 553 |
| TATTGTAATTTTCGTTTTTC | 530 | TATTGTAATTTTCTTTTTTT | 554 |
| TTCGTTTTTCGGGTTTAAGC | 531 | TTCTTTTTTCTGGTTTAAGT | 555 |
| TTTTTTTGTTTTAGTTTTTC | 532 | TTTTTTTGTTTTAGTTTTTT | 556 |
| GAGTAGTTGGGATTATAGGC | 533 | GAGTAGTTGGGATTATAGGT | 557 |
| GTAGTTGGGATTATAGGCGC | 534 | GTAGTTGGGATTATAGGCTT | 558 |
| AGTTGGGATTATAGGCGCGC | 535 | AGTTGGGATTATAGGCTCTT | 559 |
| TTATAGGCGCGCGTTATTAC | 536 | TTATAGGCTCTCTTTATTAT | 560 |
| AGGCGCGCGTTATTACGTTC | 537 | AGGCTCTCTTTATTACTTTT | 561 |
| TTTGTATTTTTAGTAGAGAC | 538 | TTTGTATTTTTAGTAGAGAT | 562 |
| ATGTTGGTTAGGATGGTTTC | 539 | ATGTTGGTTAGGATGGTTTT | 563 |
| TTTTTGATTTTAGGTGATTC | 540 | TTTTTGATTTTAGGTGATTT | 564 |
| TGATTTTAGGTGATTCGTTC | 541 | TGATTTTAGGTGATTCTTTT | 565 |
| TTAGGTGATTCGTTCGTTTC | 542 | TTAGGTGATTCTTTCTTTTT | 566 |
| AAAGTGTTGGGATTATAGGC | 543 | AAAGTGTTGGGATTATAGGT | 567 |
| GATTATAGGCGTGAGTTATC | 544 | GATTATAGGCTTGAGTTATT | 568 |
| TTATAGGCGTGAGTTATCGC | 545 | TTATAGGCTTGAGTTATCTT | 569 |
| AGGCGTGAGTTATCGCGTTC | 546 | AGGCTTGAGTTATCTCTTTT | 570 |

**Tabelle 8: Bevorzugte komplementäre Primersequenzen, spezifisch für den Bisulfit-konvertierten methylierten bzw. demethylierten antisense Strang des Alu Elements.**

| Methyliert | SEQ ID NO | Demethyliert | SEQ ID NO |
|---|---|---|---|
| ACCGAACGCGATAACTCACG | 571 | ACCAAACACAATAACTCACA | 592 |
| CCCAACACTTTAAAAAACCG | 572 | CCCAACACTTTAAAAAACCA | 593 |
| CACTTTAAAAAACCGAAACG | 573 | CACTTTAAAAAACCAAAACA | 594 |
| TTAAAAAACCGAAACGAACG | 574 | TTAAAAAACCAAAACAAACA | 595 |
| CACCTAAAATCAAAAAATCG | 575 | CACCTAAAATCAAAAAATCA | 596 |
| ACCAACATAATAAAACCCCG | 576 | ACCAACATAATAAAACCCCA | 597 |
| AAAAATACAAAAATTAACCG | 577 | AAAAATACAAAAATTAACCA | 598 |
| ATACAAAAATTAACCGAACG | 578 | ATACAAAAATTAACCAAACA | 599 |
| ATTAACCGAACGTAATAACG | 579 | ATTAACCAAACATAATAACA | 600 |
| TAACCGAACGTAATAACGCG | 580 | TAACCAAACATAATAACACA | 601 |
| ACCGAACGTAATAACGCGCG | 581 | ACCAAACATAATAACACACA | 602 |
| CCTATAATCCCAACTACTCG | 582 | CCTATAATCCCAACTACTCA | 603 |
| GAACTAAAACAAAAAAATCG | 583 | GAACTAAAACAAAAAAATCA | 604 |
| AAAAAAATCGCTTAAACCCG | 584 | AAAAAAATCACTTAAACCCA | 605 |
| TCGCTTAAACCCGAAAAACG | 585 | TCACTTAAACCCAAAAAACA | 606 |
| ACGAAAATTACAATAAACCG | 586 | ACAAAAATTACAATAAACCA | 607 |
| ATTACAATAAACCGAAATCG | 587 | ATTACAATAAACCAAAATCA | 608 |
| TACAATAAACCGAAATCGCG | 588 | TACAATAAACCAAAATCACA | 609 |
| ACTACACTCCAACCTAAACG | 589 | ACTACACTCCAACCTAAACA | 610 |
| CCAACCTAAACGACAAAACG | 590 | CCAACCTAAACAACAAAACA | 611 |
| AACGACAAAACGAAACTCCG | 591 | AACAACAAAACAAAACTCCA | 612 |

**Tabelle 9: Bevorzugte sequenzidentische Primersequenzen, spezifisch für den Bisulfit-konvertierten methylierten bzw. demethylierten sense Strang des HERV-K Elements.**

| Methyliert | SEQ ID NO | Demethyliert | SEQ ID NO |
|---|---|---|---|
| ATGATTTTATTTTTAATTTC | 613 | ATGATTTTATTTTTAATTTT | 661 |
| GGGTTAAATGGATTAAGGGC | 614 | GGGTTAAATGGATTAAGGGT | 662 |
| TTTAGGGATATAAAAATTGC | 615 | TTTAGGGATATAAAAATTGT | 663 |
| AGAGTTTGAAATATGGTTTC | 616 | AGAGTTTGAAATATGGTTTT | 664 |
| GGGAAGGGAAAGATTTGATC | 617 | GGGAAGGGAAAGATTTGATT | 665 |
| ATTTGATCGTTTTTTAGTTC | 618 | ATTTGATCTTTTTTTAGTTT | 666 |
| TTTGGGTAATGGAATGTTTC | 619 | TTTGGGTAATGGAATGTTTT | 667 |
| AATGTTTCGGTATAAAATTC | 620 | AATGTTTCTGTATAAAATTT | 668 |
| GGTATAAAATTCGATTGTAC | 621 | GGTATAAAATTCTATTGTAT | 669 |
| ATGTAAAGATTTTTGTTTAC | 622 | ATGTAAAGATTTTTGTTTAT | 670 |
| TTTTTTAGAGAAATATTTAC | 623 | TTTTTTAGAGAAATATTTAT | 671 |
| GGATTTTTTATATGTTGAAC | 624 | GGATTTTTTATATGTTGAAT | 672 |
| ATGTTGAACGTTGGTTTTTC | 625 | ATGTTGAACTTTGGTTTTTT | 673 |
| AGTTTTTTATTGTATTTTAC | 626 | AGTTTTTTATTGTATTTTAT | 674 |
| TTTTTTATTTGGTGTTTAAC | 627 | TTTTTTATTTGGTGTTTAAT | 675 |
| TTTGGGGTGAAGGTATATTC | 628 | TTTGGGGTGAAGGTATATTT | 676 |
| GGGTGAAGGTATATTCGAGC | 629 | GGGTGAAGGTATATTCTAGT | 677 |
| GTGGTTATTGAGGATAAGTC | 630 | GTGGTTATTGAGGATAAGTT | 678 |
| ATAAGTCGATAAGAGATTTC | 631 | ATAAGTCTATAAGAGATTTT | 679 |
| ATATTTATAGTTAGTTTTAC | 632 | ATATTTATAGTTAGTTTTAT | 680 |
| TACGGTAAGTTTGTGTATTC | 633 | TACTGTAAGTTTGTGTATTT | 681 |
| TATTTTAAATAGAAGATAGC | 634 | TATTTTAAATAGAAGATAGT | 682 |
| AAAAAATTTTAGAAGGAAAC | 635 | AAAAAATTTTAGAAGGAAAT | 683 |
| AAACGGAAATTTTATATTGC | 636 | AAACTGAAATTTTATATTGT | 684 |
| TGCGAATATGTAGTAGAGTC | 637 | TGCTAATATGTAGTAGAGTT | 685 |
| TCGTTAATGGTTTAGTTAAC | 638 | TCTTTAATGGTTTAGTTAAT | 686 |
| GTTATTAGAGTTTAAATTAC | 639 | GTTATTAGAGTTTAAATTAT | 687 |
| TTTTAGTAGGTTAGGTGATC | 640 | TTTTAGTAGGTTAGGTGATT | 688 |
| GTAATATTATAATTTTAAGC | 641 | GTAATATTATAATTTTAAGT | 689 |
| GTTTATTAATATTGGTTATC | 642 | GTTTATTAATATTGGTTATT | 690 |
| ATTAATATTGGTTATCGGTC | 643 | ATTAATATTGGTTATCTGTT | 691 |
| ATCGGTCGAATTTTAGTATC | 644 | ATCTGTCTAATTTTAGTATT | 692 |
| AGGGAGTTATATTTTTAGTC | 645 | AGGGAGTTATATTTTTAGTT | 693 |
| AAGGAAGGAGATATTGAGGC | 646 | AAGGAAGGAGATATTGAGGT | 694 |
| GCGTGGTAATTTTTAGTAAC | 647 | GCTTGGTAATTTTTAGTAAT | 695 |
| TTTTTAGTAACGTTAGAATC | 648 | TTTTTAGTAACTTTAGAATT | 696 |
| ATGTGGATTTTTGTGTTTAC | 649 | ATGTGGATTTTTGTGTTTAT | 697 |
| GATTTTTGTGTTTACGGATC | 650 | GATTTTTGTGTTTACTGATT | 698 |
| TTTGTGTTTACGGATCGATC | 651 | TTTGTGTTTACTGATCTATT | 699 |
| GATCGATCGTGGGAGGTTTC | 652 | GATCTATCTTGGGAGGTTTT | 700 |
| TGATTGAAATATTAAAAGGC | 653 | TGATTGAAATATTAAAAGGT | 701 |
| TTATAAATTTTATATTAATC | 654 | TTATAAATTTTATATTAATT | 702 |
| TAGGTGTATTTAATAGTTTC | 655 | TAGGTGTATTTAATAGTTTT | 703 |
| TTCGAAGAGATAGTGATATC | 656 | TTCTAAGAGATAGTGATATT | 704 |
| GAGATAGTGATATCGAGAAC | 657 | GAGATAGTGATATCTAGAAT | 705 |
| CGAGAACGGGTTATGATGAC | 658 | CTAGAACTGGTTATGATGAT | 706 |
| CGGGTTATGATGACGATGGC | 659 | CTGGTTATGATGACTATGGT | 707 |
| ATGACGATGGCGGTTTTGTC | 660 | ATGACTATGGCTGTTTTGTT | 708 |

**Tabelle 10: Bevorzugte komplementäre Primersequenzen, spezifisch für den Bisulfit-konvertierten methylierten bzw. demethylierten sense Strang des HERV-K Elements.**

| Methyliert | SEQ ID NO | Demethyliert | SEQ ID NO |
|---|---|---|---|
| AAAAAAAATAAAAAAACCCG | 709 | AAAAAAAATAAAAAAACCCA | 753 |
| AAAAACCCGAAAAACCAACG | 710 | AAAAACCCAAAAAACCAACA | 754 |
| TCAACATATAAAAAATCCCG | 711 | TCAACATATAAAAAATCCCA | 755 |
| CATTCATAAATATTTCTCCG | 712 | CATTCATAAATATTTCTCCA | 756 |
| AAAATCAACAAACAAACACG | 713 | AAAATCAACAAACAAACACA | 757 |
| AAACATCTCAATACTTTACG | 714 | AAACATCTCAATACTTTACA | 758 |
| ATAAATAAAATATTCAATCG | 715 | ATAAATAAAATATTCAATCA | 759 |
| AAAATCCCTACGACCTTTCG | 716 | AAAATCCCTACAACCTTTCA | 760 |
| ATTTCCCCCTTTTCTTTTCG | 717 | ATTTCCCCCTTTTCTTTTCA | 761 |
| TTTTCTTTTCGACAAAACCG | 718 | TTTTCTTTTCAACAAAACCA | 762 |
| TTTCGACAAAACCGCCATCG | 719 | TTTCAACAAAACCACCATCA | 763 |
| GCCATCGTCATCATAACCCG | 720 | GCCATCATCATCATAACCCA | 764 |
| GTCATCATAACCCGTTCTCG | 721 | GTCATCATAACCCATTCTCA | 765 |
| TCGATATCACTATCTCTTCG | 722 | TCAATATCACTATCTCTTCA | 766 |
| AACAAAACAAACACACAACG | 723 | AACAAAACAAACACACAACA | 767 |
| TAACAAAATTAAAATTTACG | 724 | TAACAAAATTAAAATTTACA | 768 |
| TTTTAAATCTATTTAAAACG | 725 | TTTTAAATCTATTTAAAACA | 769 |
| CAAAATATAAATAAATAACG | 726 | CAAAATATAAATAAATAACA | 770 |
| AAATAACGAAACCTCCCACG | 727 | AAATAACAAAACCTCCCACA | 771 |
| AACGAAACCTCCCACGATCG | 728 | AACAAAACCTCCCACAATCA | 772 |
| AACCTCCCACGATCGATCCG | 729 | AACCTCCCACAATCAATCCA | 773 |
| GCAACTTTATAAAAAAACCG | 730 | GCAACTTTATAAAAAAACCA | 774 |
| TTAAAATAAAATTTAAATCG | 731 | TTAAAATAAAATTTAAATCA | 775 |
| ATAATATAAAATAACTTACG | 732 | ATAATATAAAATAACTTACA | 776 |
| CTAAACTTTCTATTAAATCG | 733 | CTAAACTTTCTATTAAATCA | 777 |
| TTTCTATTAAATCGCTATCG | 734 | TTTCTATTAAATCACTATCA | 778 |
| AACGATCATAATAATTTCCG | 735 | AACAATCATAATAATTTCCA | 779 |
| CATTATTATAACAAATCTCG | 736 | CATTATTATAACAAATCTCA | 780 |
| CTTCTAAAACTATACCTACG | 737 | CTTCTAAAACTATACCTACA | 781 |
| CTAAAACTATACCTACGCCG | 738 | CTAAAACTATACCTACACCA | 782 |
| ACATTATCTCCTAATAAACG | 739 | ACATTATCTCCTAATAAACA | 783 |
| TAACTTTCTAAAAATAACCG | 740 | TAACTTTCTAAAAATAACCA | 784 |
| ATAACCGATACTAAAATTCG | 741 | ATAACCAATACTAAAATTCA | 785 |
| CCGATACTAAAATTCGACCG | 742 | CCAATACTAAAATTCAACCA | 786 |
| CTTATTTTCTCTAACCTACG | 743 | CTTATTTTCTCTAACCTACA | 787 |
| TTCGCAATATAAAATTTCCG | 744 | TTCACAATATAAAATTTCCA | 788 |
| TATCACCCTAACTTCTTCCG | 745 | TATCACCCTAACTTCTTCCA | 789 |
| CCGAATACACAAACTTACCG | 746 | CCAAATACACAAACTTACCA | 790 |
| ACTAACTATAAATATACTCG | 747 | ACTAACTATAAATATACTCA | 791 |
| ACTTATCCTCAATAACCACG | 748 | ACTTATCCTCAATAACCACA | 792 |
| ATCCTCAATAACCACGCTCG | 749 | ATCCTCAATAACCACACTCA | 793 |
| ACACCTATAAATATTTCTCG | 750 | ACACCTATAAATATTTCTCA | 794 |
| AAAAACCCGAAAAACCAACG | 751 | AAAAACCCAAAAAACCAACA | 795 |
| AAAATAAACAAACAAACACG | 752 | AAAATAAACAAACAAACACA | 796 |

**Tabelle 11: Bevorzugte sequenzidentische Primersequenzen, spezifisch für den Bisulfit-konvertierten methylierten bzw. demethylierten antisense Strang des HERV-K Elements.**

| Methyliert | SEQ ID NO | Demethyliert | SEQ ID NO |
|---|---|---|---|
| AGAAAGAAATAAGGGGGTTC | 797 | AGAAAGAAATAAGGGGGTTT | 860 |
| AGGGGGTTCGGGGAATTAGC | 798 | AGGGGGTTCTGGGAATTAGT | 861 |
| TTTAGTATATGGAGGATTTC | 799 | TTTAGTATATGGAGGATTTT | 862 |
| TTAGTATTTATTGATTATTC | 800 | TTAGTATTTATTGATTATTT | 863 |
| TTATTCGTGGGTGTTTTTTC | 801 | TTATTCTTGGGTGTTTTTTT | 864 |
| GAGGGTTAGTAGATAAATAC | 802 | GAGGGTTAGTAGATAAATAT | 865 |
| TAAATATTTTAATGTTTTAC | 803 | TAAATATTTTAATGTTTTAT | 866 |
| AGTAGATGGAATGTTTAATC | 804 | AGTAGATGGAATGTTTAATT | 867 |
| TTTTAGTATAGATTTTTTAC | 805 | TTTTAGTATAGATTTTTTAT | 868 |
| ATAGATTTTTTACGGGTGTC | 806 | ATAGATTTTTTACTGGTGTT | 869 |
| TTAGGTTTTTTTTTTTTTAC | 807 | TTAGGTTTTTTTTTTTTTAT | 870 |
| TTTTAGGTAGAGGTTTTTGC | 808 | TTTTAGGTAGAGGTTTTTGT | 871 |
| AGAGGTTTTTGCGGTTTTTC | 809 | AGAGGTTTTTGCTGTTTTTT | 872 |
| GTATATGTTTTAGAGAGTAC | 810 | GTATATGTTTTAGAGAGTAT | 873 |
| TATTTTTTTTTTTTTTTTTC | 811 | TATTTTTTTTTTTTTTTTTT | 874 |
| TTTTTTTTTTCGATAAAATC | 812 | TTTTTTTTTTCTATAAAATT | 875 |
| TTTTCGATAAAATCGTTATC | 813 | TTTTCTATAAAATCTTTATT | 876 |
| CGTTATCGTTATTATGGTTC | 814 | CTTTATCTTTATTATGGTTT | 877 |
| CGTTATTATGGTTCGTTTTC | 815 | CTTTATTATGGTTCTTTTTT | 878 |
| TTCGATGTTATTGTTTTTTC | 816 | TTCTATGTTATTGTTTTTTT | 879 |
| AGATAAAATAGGTATATAAC | 817 | AGATAAAATAGGTATATAAT | 880 |
| GTGATAGGGTTAAGATTTGC | 818 | GTGATAGGGTTAAGATTTGT | 881 |
| TAATTTTTGTTATAGTAGTC | 819 | TAATTTTTGTTATAGTAGTT | 882 |
| TTTTTGGATTTATTTAAAAC | 820 | TTTTTGGATTTATTTAAAAT | 883 |
| TTAAAATATGGATGGATGGC | 821 | TTAAAATATGGATGGATGGT | 884 |
| TGGATGGCGAGGTTTTTTAC | 822 | TGGATGGCTAGGTTTTTTAT | 885 |
| TGGCGAGGTTTTTTACGGTC | 823 | TGGCTAGGTTTTTTACTGTT | 886 |
| AGGTTTTTTACGGTCGGTTC | 824 | AGGTTTTTTACTGTCTGTTT | 887 |
| TGTTTTTATTAGTAGAATAC | 825 | TGTTTTTATTAGTAGAATAT | 888 |
| CGTAATTTTGTAAAGGAATC | 826 | CTTAATTTTGTAAAGGAATT | 889 |
| GTTAGAATGGAATTTAGGTC | 827 | GTTAGAATGGAATTTAGGTT | 890 |
| GATAGTATAAAATGGTTTAC | 828 | GATAGTATAAAATGGTTTAT | 891 |
| TTATTTGTGTATTTGGATAC | 829 | TTATTTGTGTATTTGGATAT | 892 |
| ATTGTGGTAGAATTGATTTC | 830 | ATTGTGGTAGAATTGATTTT | 893 |
| GTTTAATTTATAATAGTTTC | 831 | GTTTAATTTATAATAGTTTT | 894 |
| GTTTTGTAAATAATTTATTC | 832 | GTTTTGTAAATAATTTATTT | 895 |
| CGTGGTTTGAGTGATATTTC | 833 | CTTGGTTTGAGTGATATTTT | 896 |
| TTTAGGTTTGGTAGGGTAGC | 834 | TTTAGGTTTGGTAGGGTAGT | 897 |
| TGATTGGTGTTATTATTTTC | 835 | TGATTGGTGTTATTATTTTT | 898 |
| GTTATTATTTTCGTGGAGGC | 836 | GTTATTATTTTCTTGGAGGT | 899 |
| GTATTATATATGTAGAATTC | 837 | GTATTATATATGTAGAATTT | 900 |
| AGTATTTTTTAAAGGTTTAC | 838 | AGTATTTTTTAAAGGTTTAT | 901 |
| AGGAATGTTTAGAGTTGGTC | 839 | AGGAATGTTTAGAGTTGGTT | 902 |
| ATGGGGTTATATAATGTAGC | 840 | ATGGGGTTATATAATGTAGT | 903 |
| TTATTGTTGTAATAAATTTC | 841 | TTATTGTTGTAATAAATTTT | 904 |
| ATTTTTGAGGTTGTGTTTAC | 842 | ATTTTTGAGGTTGTGTTTAT | 905 |
| TTTGAGGTTGTGTTTACGTC | 843 | TTTGAGGTTGTGTTTACTTT | 906 |
| TTATAAGTATAGTTTTATGC | 844 | TTATAAGTATAGTTTTATGT | 907 |
| TTTTTTTTTTAGGTGGTATC | 845 | TTTTTTTTTTAGGTGGTATT | 908 |
| TAGGTGGTATCGGTTTTAAC | 846 | TAGGTGGTATCTGTTTTAAT | 909 |
| TTGATTTTTTGGGGGTGGTC | 847 | TTGATTTTTTGGGGGTGGTT | 910 |
| GGTGGTCGATATTGAAGTTC | 848 | GGTGGTCTATATTGAAGTTT | 911 |
| GTCGATATTGAAGTTCGGTC | 849 | GTCTATATTGAAGTTCTGTT | 912 |
| TTTTATTTTTTTTAATTTGC | 850 | TTTTATTTTTTTTAATTTGT | 913 |
| GTTTGAGGTTGTAATGTTAC | 851 | GTTTGAGGTTGTAATGTTAT | 914 |
| GCGTTGATTGAGTTATTAAC | 852 | GCTTTGATTGAGTTATTAAT | 915 |
| TTGTTATTTTAGTTTTTTTC | 853 | TTGTTATTTTAGTTTTTTTT | 916 |
| TTCGAGTGTATAAGTTTATC | 854 | TTCTAGTGTATAAGTTTATT | 917 |
| GATTTGTTTTTAATGATTAC | 855 | GATTTGTTTTTAATGATTAT | 918 |
| TGTTTTTAATGATTACGTTC | 856 | TGTTTTTAATGATTACTTTT | 919 |
| TATATTTGTGGGTGTTTTTC | 857 | TATATTTGTGGGTGTTTTTT | 920 |
| AGAAAGAAATAAGGGGGTTC | 858 | AGAAAGAAATAAGGGGGTTT | 921 |
| AGGGGGTTCGGGGAATTAAC | 859 | AGGGGGTTCTGGGAATTAAT | 922 |

**Tabelle 12: Bevorzugte komplementäre Primersequenzen, spezifisch für den Bisulfit-konvertierten methylierten bzw. demethylierten antisense Strang des HERV-K Elements.**

| Methyliert | SEQ ID NO | Demethyliert | SEQ ID NO |
|---|---|---|---|
| TAACCTTACCCCCAACCCCG | 923 | TAACCTTACCCCCAACCCCA | 986 |
| AATTAAATAAATTAAAAACG | 924 | AATTAAATAAATTAAAAACA | 987 |
| CCAAAAACACAAAAACTACG | 925 | CCAAAAACACAAAAACTACA | 988 |
| AAATCTAAAATATAACCTCG | 926 | AAATCTAAAATATAACCTCA | 989 |
| AAAAAAAAAAAACCTAACCG | 927 | AAAAAAAAAAAACCTAACCA | 990 |
| CCTAACCGTCCCCCAACCCG | 928 | CCTAACCATCCCCCAACCCA | 991 |
| CTAAACAATAAAATATCTCG | 929 | CTAAACAATAAAATATCTCA | 992 |
| ATATCTCGATATAAAACCCG | 930 | ATATCTCAATATAAAACCCA | 993 |
| ATATAAAACCCGATTATACG | 931 | ATATAAAACCCAATTATACA | 994 |
| TACAAAAACCTTTATTCACG | 932 | TACAAAAACCTTTATTCACA | 995 |
| CTCTCAAAAAAACACCCACG | 933 | CTCTCAAAAAAACACCCACA | 996 |
| AATCCTCCATATACTAAACG | 934 | AATCCTCCATATACTAAACA | 997 |
| TACTAAACGTTAATTCCCCG | 935 | TACTAAACATTAATTCCCCA | 998 |
| ATCTCTCATTACACCTTACG | 936 | ATCTCTCATTACACCTTACA | 999 |
| CCTTCATCTAATACCCAACG | 937 | CCTTCATCTAATACCCAACA | 1000 |
| CTAAAATAAAAATACACTCG | 938 | CTAAAATAAAAATACACTCA | 1001 |
| AATAAAAATACACTCGAACG | 939 | AATAAAAATACACTCAAACA | 1002 |
| TAATCATTAAAAACAAATCG | 940 | TAATCATTAAAAACAAATCA | 1003 |
| ATTTCAAACAAAAAATAACG | 941 | ATTTCAAACAAAAAATAACA | 1004 |
| AAAAATCCCAAAAAAAAACG | 942 | AAAAATCCCAAAAAAAAACA | 1005 |
| AACGAAAACTTTACATTACG | 943 | AACAAAAACTTTACATTACA | 1006 |
| ACGAATATATAACAAAACCG | 944 | ACAAATATATAACAAAACCA | 1007 |
| CGTTAATAACTCAATCAACG | 945 | CATTAATAACTCAATCAACA | 1008 |
| CCATTAAAATCTAAACCACG | 946 | CCATTAAAATCTAAACCACA | 1009 |
| TTCAACAAATCAAATAACCG | 947 | TTCAACAAATCAAATAACCA | 1010 |
| TAACATTACAACCTCAAACG | 948 | TAACATTACAACCTCAAACA | 1011 |
| CTTATCAATACTAACCACCG | 949 | CTTATCAATACTAACCACCA | 1012 |
| TCAATACTAACCACCGACCG | 950 | TCAATACTAACCACCAACCA | 1013 |
| CCGACCGAACTTCAATATCG | 951 | CCAACCAAACTTCAATATCA | 1014 |
| ATTACCAATAAAAAAACCCG | 952 | ATTACCAATAAAAAAACCCA | 1015 |
| AATAAAATTAATAACATACG | 953 | AATAAAATTAATAACATACA | 1016 |
| ATTAATAACATACGAAAACG | 954 | ATTAATAACATACAAAAACA | 1017 |
| TCAAAATATATAAAAACCCG | 955 | TCAAAATATATAAAAACCCA | 1018 |
| ATAAAATTAAAAAAACTACG | 956 | ATAAAATTAAAAAAACTACA | 1019 |
| AAAATAAACAACCATTATCG | 957 | AAAATAAACAACCATTATCA | 1020 |
| CTAATCTTAAAAAAATCACG | 958 | CTAATCTTAAAAAAATCACA | 1021 |
| AATTTAAAAACACTAATCCG | 959 | AATTTAAAAACACTAATCCA | 1022 |
| AACTATTACAAAACTTATCG | 960 | AACTATTACAAAACTTATCA | 1023 |
| TATTACAAAACTTATCGACG | 961 | TATTACAAAACTTATCAACA | 1024 |
| TATTACAACAATAAAATACG | 962 | TATTACAACAATAAAATACA | 1025 |
| AAAAATATTAATTAAATTCG | 963 | AAAAATATTAATTAAATTCA | 1026 |
| ATTAATCCGACAAAATTACG | 964 | ATTAATCCAACAAAATTACA | 1027 |
| TCAAAACTCCATATCAATCG | 965 | TCAAAACTCCATATCAATCA | 1028 |
| CAAAAAAAAACGCCTCCACG | 966 | CAAAAAAAAACACCTCCACA | 1029 |
| GAAATATCACTCAAACCACG | 967 | GAAATATCACTCAAACCACA | 1030 |
| ATTATAAATTAAACACCTCG | 968 | ATTATAAATTAAACACCTCA | 1031 |
| ACTCAAAACAAACTCAATCG | 969 | ACTCAAAACAAACTCAATCA | 1032 |
| ACAAATAAATCCAACTATCG | 970 | ACAAATAAATCCAACTATCA | 1033 |
| AAATCCAACTATCGATAACG | 971 | AAATCCAACTATCAATAACA | 1034 |
| ACTTTAAAAACAAAATATCG | 972 | ACTTTAAAAACAAAATATCA | 1035 |
| TAAACCATTTTATACTATCG | 973 | TAAACCATTTTATACTATCA | 1036 |
| ACCTAAATTCCATTCTAACG | 974 | ACCTAAATTCCATTCTAACA | 1037 |
| TATAAATCCCTATATCCACG | 975 | TATAAATCCCTATATCCACA | 1038 |
| ATCCCTATATCCACGAACCG | 976 | ATCCCTATATCCACAAACCA | 1039 |
| CTATATCCACGAACCGACCG | 977 | CTATATCCACAAACCAACCA | 1040 |
| ACCGACCGTAAAAAACCTCG | 978 | ACCAACCATAAAAAACCTCA | 1041 |
| CACTAAAACATAATTAAACG | 979 | CACTAAAACATAATTAAACA | 1042 |
| AAAAATTACTAATAACCTCG | 980 | AAAAATTACTAATAACCTCA | 1043 |
| CCGAAAAAACAATAACATCG | 981 | CCAAAAAAACAATAACATCA | 1044 |
| AAACAATAACATCGAAAACG | 982 | AAACAATAACATCAAAAACA | 1045 |
| GAAAACGAACCATAATAACG | 983 | GAAAACAAACCATAATAACA | 1046 |
| GAACCATAATAACGATAACG | 984 | GAACCATAATAACAATAACA | 1047 |
| TAACGATAACGATTTTATCG | 985 | TAACAATAACAATTTTATCA | 1048 |

Der Fachmann wird erkennen, dass jede der in den Tabellen 1 - 12 aufgeführten Oligonukleotidsequenzen lediglich als Kernsequenz zu verstehen ist, die vom 5'-Ende her und/oder vom 3'-Ende her verkürzt oder verlängert werden kann. Dies gilt für alle in dieser Erfindung offenbarten Oligonukleotide/Primer. In einer bevorzugten Ausführungsform sind die in den Tabellen 1 - 12 dargestellten Oligonukleotide um zwischen 1 - 20 Nukleotide vom 5'-Ende und/oder vom 3'-Ende her verlängert; noch bevorzugter sind die Oligonukleotide um zwischen 5 - 15 Nukleotide vom 5 `-Ende und/oder vom 3'-Ende her verlängert. In einer anderen Ausführungsform sind die Oligonukleotide um bis zu insgesamt 5 Nukleotide vom 5'-Ende und/oder 3'-Ende her verkürzt, wobei das Oligonukleotid immer für zumindest ein CpG oder bisulfitiertes CpG spezifisch bleibt.

In einer weiteren bevorzugten Ausführungsform haben die Primer eines Primerpaars einen nahezu identischen Tₘ, bevorzugt einen Tₘ, der ≤ 3° C, ≤ 2° C, ≤ 1° C, ≤ 0.5° C, ≤ 0.2° C oder ≤ 0.1° C voneinander abweicht.

In einer weiteren bevorzugten Ausführungsform haben die von den Primern eingeschlossenen Sequenzbereiche eine Länge von ≥ 1 und ≤ 3000 bp, bevorzugter ≥ 10 und ≤ 2000 bp, noch bevorzugter ≥ 30 und ≤ 800 bp und am bevorzugtesten ≥ 50 und ≤ 300 bp.

Der Fachmann wird erkennen, dass nicht nur ein Primerpaar eingesetzt werden kann, sondern auch eine Vielzahl derselben. In einer weiteren Ausführungsform wird das Verfahren daher mit 2, 3, 4, 5 oder mehr als 5 Primerpaaren durchgeführt, die spezifisch für ein Transposon oder Fragment davon sind und die jeweils zumindest einen Primer umfassen, der für zumindest ein Cytosin eines CpG Dinukleotids oder ein bisulfitiertes Cytosin eines CpG Dinukleotid spezifisch ist. Bevorzugt haben diese mehreren Primerpaare einen nahezu identischen Tₘ.

Weiterhin wird in Schritt a) entweder die nicht-bisulfitierte DNA mit zumindest einem Primerpaar amplifiziert, dessen Primerpaar spezifisch für Bereiche desselben Transposons oder Fragments davon sind; oder es wird eine bisulfitierte DNA mit zumindest einem Primerpaar amplifiziert, das ebenfalls spezifisch für das Transposon ist, wobei jedoch die Primer keine differentiell methylierte Position des Transposons umfassen. In einer bevorzugten Ausführungsform werden hierbei Primer verwendet, die spezifisch für stets unmethylierte Bereiche des Transposons sind. Hierbei kann der Fachmann durch vorherige Sequenzanalysen bestimmen, welche Positionen in einem gegebenen Genom stets unmethyliert vorliegen. Da bei Vertebraten ^{5m}C ausschließlich in CpG-Dinukleotiden auftreten, können somit für diese Fälle Bereiche gewählt werden, die keine Cytosine solcher CpG Sequenzen enthalten.

Tabellen 13 - 18 geben bevorzugte solcher Oligonukleotide zur Normierung wieder, die für Schritt a) des Verfahrens und bei Einsatz von bisulfitierter DNA verwendet werden können. Tabellen 13 - 14 offenbaren bevorzugte Primer für das LINE-1 Element, Tabellen 15 - 16 offenbaren bevorzugte Primer für das Alu Element und Tabellen 17 - 18 offenbaren bevorzugte Primer für das HERV-K Element. Die durch diesen Amplifikationsschritt erzeugten Amplifikate können dann für die erfindungsgemäße Normierung herangezogen werden.

In bevorzugten Ausführungsformen bezieht sich die Erfindung auf folgende solcher Oligonukleotide für die Normierung und deren Verwendung in den erfindungsgemäßen Verfahren:
Sequenzidentische oder komplementäre Primersequenzen, die spezifisch für den Bisulfit-konvertierten sense oder antisense Strang des Promotorbereichs des LINE-1 Elements sind, d.h. SEQ ID NO 1049 - 1227; bevorzugter SEQ ID NO 1049 - 1145, oder SEQ ID NO 1146 - 1227; noch bevorzugter SEQ ID NO 1049 - 1096, oder SEQ ID NO 1097 - 1145, oder SEQ ID NO 1146 - 1192, oder SEQ ID NO 1193 - 1227.

Sequenzidentische oder komplementäre Primersequenzen, die spezifisch für den Bisulfit-konvertierten sense oder antisense Strang des Alu Elements sind, d.h. SEQ ID NO 1228 - 1257; bevorzugter SEQ ID NO 1228 - 1243, oder SEQ ID NO 1244 - 1257; noch bevorzugter SEQ ID NO 1228 - 1237, oder SEQ ID NO 1238 - 1243, oder SEQ ID NO 1244 - 1250, oder SEQ ID NO 1251 - 1257.

Sequenzidentische oder komplementäre Primersequenzen, die spezifisch für den Bisulfit-konvertierten sense oder antisense Strang des HERV-K Elements sind, d.h. SEQ ID NO 1258 - 1415; bevorzugter SEQ ID NO 1258 - 1323, oder SEQ ID NO 1324 - 1415; noch bevorzugter SEQ ID NO 1258 - 1289, oder SEQ ID NO 1290 - 1323, oder SEQ ID NO 1324 - 1371, oder SEQ ID NO 1372 - 1415.

**Tabelle 13: Bevorzugte sequenzidentische und komplementäre Primersequenzen, spezifisch für den Bisulfit-konvertierten sense Strang des Promotorbereichs des LINE-1 Elements.**

| Sequenzidentisch | SEQ ID NO | Komplementäre | SEQ ID NO |
|---|---|---|---|
| GTGATTTTTGTATTTTTATT | 1049 | CTCTATATTTCCTAAATCTA | 1097 |
| GGTTTATTTTATTAGGGAGT | 1050 | TTAACCTACCTTACTAAATT | 1098 |
| AGGGAGTGTTAGATAGTGGG | 1051 | TAAATAATATCCTACAAAAT | 1099 |
| AGGTATTGTTTTATTTGGGA | 1052 | CACATCACTTTCAAATACAC | 1100 |
| TAAGGGGTTAGGGAGTTTTT | 1053 | ATTTAATCTTTTCACATAAT | 1101 |
| TTTTTGAGTTAAAGAAAGGG | 1054 | CTTAAAAACTTTACTCATTT | 1102 |
| AGATTATATTTTATATTTGG | 1055 | TTATTCTTTTTTCTCTAAAC | 1103 |
| TTGATTGTTAGTATAGTAGT | 1056 | TTCATTTCATTCATTTCATC | 1104 |
| TTTGAGATTAAATTGTAAGG | 1057 | ATACCCTTTCTTCCAATTAA | 1105 |
| TTATTGTTTAGGTTTGTTTA | 1058 | CCTAAAACTTCTACATTCTT | 1106 |
| GTTTAGGTAAATAAAGTAGT | 1059 | ATTTTCAACTCCATCAACTC | 1107 |
| AATTGGGTGGAGTTTATTAT | 1060 | TTATTCTAATTATACATTCT | 1108 |
| TAGTTTAAGGAGGTTTGTTT | 1061 | AAAATTTTCAACTTCTTTAC | 1109 |
| GTTTTTGTAGGTTTTATTTT | 1062 | GTAACTCAAAATAATTTAAT | 1110 |
| TGGGGGTAGGGTATAGATAA | 1063 | AAAACCTTCTTCTCTCAACT | 1111 |
| ATAAAAAGATAGTAGTAATT | 1064 | GTCAAAATCATTCTCCATCC | 1112 |
| TTTGTAGATTTAAGTGTTTT | 1065 | ATTCTATTACTAATAAAAAA | 1113 |
| TGTTTGATAGTTTTGAAGAG | 1066 | GTTCCTTTAAAAAAAAAAAA | 1114 |
| GAGTAGTGGTTTTTTTAGTA | 1067 | TTTAAAATTTCCAATTTTTC | 1115 |
| GGTAGATAGATTGTTTTTTT | 1068 | CCCATCTTTATAATTTTATC | 1116 |
| AAGTGGGTTTTTGATTTTTG | 1069 | TAATCTTTAATAATAATAAT | 1117 |
| ATTTTCGAGTAGTTTAATTG | 1070 | TAAATATCCTTTCTAATTAT | 1118 |
| GGAGGTATTTTTTAGTAGGG | 1071 | CAAACAAAACCCTCAACTAC | 1119 |
| GGGTATATTGATATTTTATA | 1072 | GTATAAAATATCAATATACC | 1120 |
| GTAGGGTATTTTAATAGATT | 1073 | AAATACCTCCCAATTAAACT | 1121 |
| TGTAGTTGAGGGTTTTGTTT | 1074 | AAAATCAAAAACCCACTTAA | 1122 |
| TTAGAAGGAAAATTAATAAT | 1075 | AAAAAAACAATCTATCTACC | 1123 |
| ATTAGAAAGGATATTTATAT | 1076 | GTTCTCAAATCTCCAACTA | 1124 |
| AAAATTTATTTGTATATTAT | 1077 | ACTAAAAAAACCACTACTCT | 1125 |
| TATTATTAAAGATTAAAAGT | 1078 | AAACAAAAACACTTAAATCT | 1126 |
| AGATAAAATTATAAAGATGG | 1079 | ATTACTACTATCTTTTTATT | 1127 |
| GGAAAAAATAGAATAGAAAA | 1080 | CCCCAAAAATAAAACCTACA | 1128 |
| AAAAATTGGAAATTTTAAAA | 1081 | CAAACCTCCTTAAACTATAA | 1129 |
| TTTTTTTTTTTTTAAAGGAA | 1082 | TTTACCTAAACAAACCTAAA | 1130 |
| TAGTTTTTTATTAGTAATAG | 1083 | CAATTTAATCTCAAACTACT | 1131 |
| AATAAAGTTGGATGGAGAAT | 1084 | CCAAATATAAAATATAATCT | 1132 |
| AGTTGAGAGAAGAAGGTTTT | 1085 | CACCCCTTTCTTTAACTCAA | 1133 |
| AGACGATTAAATTATTTTGA | 1086 | AAAAACTCCCTAACCCCTTA | 1134 |
| GGAGGATATTTAAATTAAAG | 1087 | TCCCAAATAAAACAATACCT | 1135 |
| GTAAAGAAGTTGAAAATTTT | 1088 | TCTAACACTCCCTAATAAAA | 1136 |
| TGAAAAAAATTTAGAAGAAT | 1089 | ACCTCAAATAAAAATACAAA | 1137 |
| GTATAATTAGAATAATTAAT | 1090 | ACCATCTTAACTCCTCCCCC | 1138 |
| ATAGAGAAGTGTTTAAAGGA | 1091 | CACTAAAACATAATTAAACA | 1139 |
| GTTGATGGAGTTGAAAATTA | 1092 | AAAAATTACTAATAACCTCA | 1140 |
| TGAAGAATGTAGAAGTTTTA | 1093 | CCAAAAAAACAATAACATCA | 1141 |
| ATTAATTGGAAGAAAGGGTA | 1094 | AAACAATAACATCAAAAACA | 1142 |
| TTAGTAATGGAAGATGAAAT | 1095 | GAAAACAAACCATAATAACA | 1143 |
| AGAAGGGAAGTTTAGAGAAA | 1096 | GAACCATAATAACAATAACA TAACAATAACAATTTTATCA | 1144 1145 |

**Tabelle 14: Bevorzugte sequenzidentische und komplementäre Primersequenzen, spezifisch für den Bisulfit-konvertierten antisense Strang des Promotorbereichs des LINE-1 Elements.**

| Sequenzidentisch | SEQ ID NO | Komplementär | SEQ ID NO |
|---|---|---|---|
| AattttgttgatTTtttTaa | 1146 | tctAcatttccatctAaAAt | 1193 |
| gtgtTtTtatttTTttTagt | 1147 | taAAAaAtAccaAacaAtAA | 1194 |
| aatgtgtttgTtTttgTttt | 1148 | AaAttccctttctAaAtcaa | 1195 |
| TaattttggatTtttTTtgT | 1149 | actatatcccacacctAAct | 1196 |
| tTTTtTtaTaTaTtgTtttg | 1150 | acaAcaAtctAaAatcaaac | 1197 |
| gtatgtggtgtTtttgttTt | 1151 | AActtActtaAAtaaacaaa | 1198 |
| aTatTtttatttTtgTTttT | 1152 | caccacaActcaaAAaAAcc | 1199 |
| aggttgttTagtttTTatgt | 1153 | ctAAAAAcaAAAcacaAaca | 1200 |
| tgTaTtgtggtTtgagagat | 1154 | AacttaaAtAtccctAtctA | 1201 |
| aTtatgtggtTaattttgga | 1155 | aaAaAaAcaAtAAttctccc | 1202 |
| gatttggggtggagagttTt | 1156 | caaAtAAAtccctAactcct | 1203 |
| ttTTtgggtatTTttgttga | 1157 | ccaAcaAAAAcacactAaca | 1204 |
| tgttaaagtTtTTTattatt | 1158 | tAaAAAtcctAtctAttaAa | 1205 |
| gTtttatgaatTtgggtgTt | 1159 | AaaAaccaaaaAtaAataaa | 1206 |
| tgttgaattgatTTTtttaT | 1160 | aacaAaacaaaActAAatAA | 1207 |
| atTagagaTtaggattgTaa | 1161 | tAaAaAaaAaaAActtcaAa | 1208 |
| ttggtagatTttTTtTTatT | 1162 | aaccaaaAAcaaaAaaAttA | 1209 |
| gTaTaTtgatgggtTttgaT | 1163 | tataactaAaataaccaata | 1210 |
| tTttttaattgTagaattta | 1164 | AatAAaActAaaaaccaaAA | 1211 |
| tttgTtTattagttgatgTa | 1165 | aatAcaAaaAcctcaAAaAc | 1212 |
| ttaTattttggTatgatttt | 1166 | caAcaatAAaaAatAaaatA | 1213 |
| gtgTttTTttTaggagTtTt | 1167 | aAaAaaaaaaAaataaaaaA | 1214 |
| aaagtattttatttTtTTtt | 1168 | AaaatatAAAactatAtAaa | 1215 |
| aaattTtgggttgaaaattT | 1169 | tAaaaAtAatAtAAaAaatA | 1216 |
| ggTtgTTTttaaTatttttt | 1170 | tAcaAAatattatccaAAaA | 1217 |
| aTaattatgtgtTttggagt | 1171 | AattcaAAaaatacaAaAaa | 1218 |
| gttTTattTtTTaTatTaTt | 1172 | AattcaccaaaAttAaaatA | 1219 |
| aTatagtTTTatatttTttg | 1173 | aaaAcccatcaAactaacaA | 1220 |
| TtgataTTTtttTttTTagt | 1174 | AaaAaAaAtAAAAAccaata | 1221 |
| TTtgaggTttTtgTattTtt | 1175 | aAccaaactaaActtcataa | 1222 |
| atTagTtTTtttaagTaTtt | 1176 | aaatAttAaAaAattttAtc | 1223 |
| tttTaaTttTtttgTTtttg | 1177 | AcactaaacatAAaaaAAaa | 1224 |
| gaagTTttTttTtTtTagTt | 1178 | atcatAccaaaatAtaaaAa | 1225 |
| tgttTtgttgTtggtgagga | 1179 | atcaactaatAaAcaaaatc | 1226 |
| tgatggtgatgtaTagatgg | 1180 | caAAatcaaattcacacata | 1227 |
| TTtTagTtgTaggtTtgttg | 1181 | | |
| agtgtgTTTTtgTtgggggg | 1182 | | |
| TTTaTttgaggaggTagtTt | 1183 | | |
| gTtgtTagaTagggaTaTtt | 1184 | | |
| TtgtgTTTtgTTTTTagagg | 1185 | | |
| agTtgtggtgggTtTTaTTT | 1186 | | |
| ttaagTaagTTtgggTaatg | 1187 | | |
| ttgatTtTagaTtgTtgtgT | 1188 | | |
| TTaggtgtgggatatagtTt | 1189 | | |
| tttTtttgaTtTagaaaggg | 1190 | | |
| TtggTaTtTTTtagtgagat | 1191 | | |
| TagatggaaatgTagaaatT | 1192 | | |

**Tabelle 15: Bevorzugte sequenzidentische und komplementäre Primersequenzen, spezifisch für den Bisulfit-konvertierten sense Strang des Alu Elements.**

| Sequenzidentisch | SEQ ID NO | Komplementär | SEQ ID NO |
|---|---|---|---|
| GTTTGTAATTTTAGTATTTT | 1228 | CCCAAACTAAAATACAATAA | 1238 |
| ATTTTAGTATTTTGGGAGGT | 1229 | ATTCTCCTACCTCAACCTCC | 1239 |
| GGATTATTTGAGGTTAGGAG | 1230 | TTTTATATTTTTAATAAAAA | 1240 |
| GAGATTATTTTGGTTAATAT | 1231 | CATATTAACCAAAATAATCT | 1241 |
| TGGTTAATATGGTGAAATTT | 1232 | TCTCCTAACCTCAAATAATC | 1242 |
| GTTTTTATTAAAAATATAAA | 1233 | CAAAATACTAAAATTACAAA | 1243 |
| TTAAAAATATAAAAATTAGT | 1234 | | |
| GTTTGTAATTTTAGTTATT | 1235 | | |
| GGGAGGTTGAGGTAGGAGAA | 1236 | | |
| GTTATTGTATTTTAGTTTGG | 1237 | | |

**Tabelle 16: Bevorzugte sequenzidentische und komplementäre Primersequenzen, spezifisch für den Bisulfit-konvertierten antisense Strang des Alu Elements.**

| Sequenzidentisch | SEQ ID NO | Komplementär | SEQ ID NO |
|---|---|---|---|
| TTTAGGTTGGAGTGTAGTGG | 1244 | ATCCCAACACTTTAAAAAAC | 1251 |
| ATTTTTTTGTTTTAGTTTTT | 1245 | AATCACCTAAAATCAAAAAA | 1252 |
| GAGTAGTTGGGATTATAGG | 1246 | TCCTAACCAACATAATAAAA | 1253 |
| TTTTTGTATTTTTAGTAGAG | 1247 | TACTAAAAATACAAAAATTA | 1254 |
| TTTTATTATGTTGGTTAGGA | 1248 | GCCTATAATCCCAACTACT | 1255 |
| ATTTTTTGATTTTAGGTGAT | 1249 | GAAAAACTAAAACAAAAAAA | 1256 |
| TTTTTAAAGTGTTGGGATTA | 1250 | CCACTACACTCCAACCTAAA | 1257 |

**Tabelle 17: Bevorzugte sequenzidentische und komplementäre Primersequenzen, spezifisch für den Bisulfit-konvertierten sense Strang des HERV-K Elements.**

| Sequenzidentisch | SEQ ID NO | Komplementär | SEQ ID NO |
|---|---|---|---|
| gtagttgagataagaggaag | 1258 | cttaatatttattaatcatt | 1290 |
| agggagaaattattttaggg | 1259 | tacatacacataaacatctc | 1291 |
| taaagtattgagatgtttat | 1260 | ttccctatctcaataaataa | 1292 |
| atatattttttttttagaga | 1261 | aacattccattacccaaaaa | 1293 |
| gaaatatttataggtgtgga | 1262 | ctcacataaaaaaaaacctt | 1294 |
| ggggtaaattaaaattaaaa | 1263 | taaaaaataataataactct | 1295 |
| atagaataattttgtttatg | 1264 | tccatttaacccaaaattta | 1296 |
| agtaggtaggaagggtaata | 1265 | aacaaaaaaatttttcttaa | 1297 |
| gttttagaattattttaaat | 1266 | actaacaacaaacaaaacaa | 1298 |
| Ggaagttgtataatagattg | 1267 | tcctaacaccaaatttaaat | 1299 |
| aattagtggggttattagag | 1268 | ctaaaataaaattatcttct | 1300 |
| gatttaattgttagtagttt | 1269 | tattctaaaatcataaacct | 1301 |
| tatggtattatttagtaggt | 1270 | aacttaccaatttttaatca | 1302 |
| gaaagagggagtaaaatagt | 1271 | aatcaaaatataaataaata | 1303 |
| gaattgatggggtataagaa | 1272 | aactaatttaataactatat | 1304 |
| taagtattaatgtaaaatga | 1273 | tatacttatatttatctaaa | 1305 |
| agagtttgggaaaaaattta | 1274 | cttaaaacaaattttccctt | 1306 |
| atagtaagataaggtttaaa | 1275 | catcctaatactctccctaa | 1307 |
| gatgtaattttagagtatgt | 1276 | cattataaaacttcaaatat | 1308 |
| atattgggttagttaatgtt | 1277 | taaaattttccactaactta | 1309 |
| ataaaaaatttttataggag | 1278 | cattactaaaaccatcaata | 1310 |
| ttagaagtgtattaaagtat | 1279 | tttactaataaatataaaac | 1311 |
| tggtttatatagggttaaaa | 1280 | atataaaatctcaatacttt | 1312 |
| ttagttatatggatggataa | 1281 | aaccttaatatataacaaaa | 1313 |
| gatttaatttttaattggta | 1282 | aactcccctaaaaacaaaaa | 1314 |
| atattttgattgaaatatta | 1283 | ctctacctattattataata | 1315 |
| gagtattattgggatatggt | 1284 | tctaaaattacatctaatcc | 1316 |
| ttatgattataaattttata | 1285 | accctacctactaaataata | 1317 |
| agtagatataggagatttta | 1286 | acctcctataattaattata | 1318 |
| tttgtttaggaaagttaggt | 1287 | attttaataaaactaaaata | 1319 |
| tttattgagatagggaaaaa | 1288 | cataaacaaaataaaaaatt | 1320 |
| ataaatattaagggaattta | 1289 | ctaatcctcctcaacacaaa | 1321 |
| | | ccttcaaacatctatttaac | 1322 |
| | | ttaacaacatctcaaaacaa | 1323 |

**Tabelle 18: Bevorzugte sequenzidentische und komplementäre Primersequenzen, spezifisch für den Bisulfit-konvertierten antisense Strang des HERV-K Elements.**

| Sequenzidentisch | SEQ ID NO | Komplementär | SEQ ID NO |
|---|---|---|---|
| tTTTttagtatttattgatT | 1324 | atctatAaccttacccccaa | 1372 |
| ggggatgtgtTagggtTaTa | 1325 | aacaAatActtAaaAAcaAc | 1373 |
| tgTatTatagaTaaggtaaa | 1326 | aatctcaaAtacccaAAAac | 1374 |
| atatgTataTaTataaaTat | 1327 | tccccatAtAaAaAtctAaa | 1375 |
| tttttTTTtatTtTagtaga | 1328 | AaAAaAAattaAtataaAaA | 1376 |
| gatgtTttTTtTttTtTtTa | 1329 | caccttaAAActAAaAAtAA | 1377 |
| ggatggtTaggtTtttTTTt | 1330 | cacatctccctctcaAaAaa | 1378 |
| agattagggagtggtgatga | 1331 | tttttcttttccaaAtctct | 1379 |
| ttgaTaTagTaTatgtttTa | 1332 | tttctctAAAAtAaaAAtac | 1380 |
| gattaaTagTatTtTaaggT | 1333 | cttaActtcattaaaattct | 1381 |
| gtaaTaatTtTatTtTtTtt | 1334 | aAcaAAtaAAaaAAAtaata | 1382 |
| gatttataatTatagtaTtt | 1335 | aattacaAAaAAtAatatat | 1383 |
| aaTtTTtgTaattgTTtTag | 1336 | ccaactAccaAtaActtatc | 1384 |
| tggaaatgtTtaaagtgaga | 1337 | aAtaAAcaAAAtaAtAaatt | 1385 |
| gTtTagaTtTattataaatt | 1338 | ccaAataaaAAtctttttaA | 1386 |
| TtgTaattaaagtaaaaatg | 1339 | tttacaatttaaAacttAAt | 1387 |
| ggtttaataaTtatatttTT | 1340 | aAttaAaActatctAcctta | 1388 |
| tTttggggtagagattTTtt | 1341 | ccattaaAccattaaaaAAa | 1389 |
| TtgagTaattgtggtagaat | 1342 | AtcaaaatAAtcatttaaaa | 1390 |
| TTaaaTtaaaaTttTtgtat | 1343 | tAataaaaatAAAcaaccat | 1391 |
| gataagtgaatTtaTtgtta | 1344 | caacccccactAtcccaaAt | 1392 |
| aggTattaaaTatTTtggtg | 1345 | AtAccaAtccaAAaAacaAA | 1393 |
| taTttTtataggattatTTa | 1346 | aaatcaAtAAccaaaaaatt | 1394 |
| aTttTaaatgtTtagtgggt | 1347 | tAaccaaAatAAAatatata | 1395 |
| tatttTTTatgtTttatttt | 1348 | taattcaAaaAaaatccaAc | 1396 |
| gTtagattaagttgTatTtg | 1349 | AaAAttAccaatAcaAAact | 1397 |
| agttgTaTaTatgaaatgtg | 1350 | atcccttaAccccactccaa | 1398 |
| tTtTaaTatTTTttgtagTT | 1351 | AtAAaaatAacccaAacaaa | 1399 |
| gTTaTttttTTattgTtgga | 1352 | AAaaaaAtAActtacacaAA | 1400 |
| TtattttgtTtgggtTattt | 1353 | aaAaaacttcccattttata | 1401 |
| tatTtTTTagTaatttttga | 1354 | acttattcacatttcatAtA | 1402 |
| GatttTttTtgaattaTaaa | 1355 | cacatAaaAAaaaactaatt | 1403 |
| TTTatTttggtTataatttt | 1356 | taatAataAtAtatAAAtac | 1404 |
| ggTTTtaagTaatgtaaaat | 1357 | cttatcaaaAatcattaaaa | 1405 |
| aatTTttTaagTtgttttTT | 1358 | tAcacaaAtAaAtccaActA | 1406 |
| TTttaatatatggTaggagt | 1359 | ctataacctAtAaaaattAt | 1407 |
| aagattagtTTtaTagtTtT | 1360 | AcaaaaAaattctacaaAat | 1408 |
| tTaaatttagaatgaTattg | 1361 | tAaAtctAaAcatcactAAA | 1409 |
| tgTttgggTTataagTatag | 1362 | tAttAttaAtctAcaAAtAt | 1410 |
| gTtTttttgaaTTttgtTtt | 1363 | aAAAtAAtAcaaAatAtAct | 1411 |
| atagttgatTtgTatTtatg | 1364 | taAtataaAaAAaaaAcatA | 1412 |
| taTtgttttaTtTTTtTttt | 1365 | actAccttaAAActAAaAAt | 1413 |
| gaTttTtTaataatttTatg | 1366 | tattAtcttAtAaccctAac | 1414 |
| aaatggttTtaaagTtgTtt | 1367 | tccaccttatAaAaaacacc | 1415 |
| TTtTTaTaTTtgtgggtgtt | 1368 | | |
| Taatagtggggagagggtga | 1369 | | |
| ggaaaTagatgTTttTTtTt | 1370 | | |
| Tttgagattagggagtggtg | 1371 | | |

Der Fachmann wird auch für jedes andere Transposon oder Fragment davon Primer nach dem zuvor erklärten Schema bereitstellen können.

Die Bestimmung des normierten DNA-Methylierungsgrads wird erfindungsgemäß über das Verhältnis der in den beiden Amplifikationsschritten (Schritt a) und b)) gebildeten Amplifikate bestimmt. In besonderen Ausführungsformen wird das Verhältnis über die gebildeten Mengen an Amplifikat bestimmt, bevorzugter über die Zunahme des pro Amplifikations-Zyklus gebildeten Amplifikats, noch bevorzugter über den cycle threshold (ct) Wert während einer real time PCR.

In einer Ausführungsform wird die Gesamtmenge der beiden gebildeten Amplifikate nach einer gleichen Anzahl von Amplifikationszyklen bestimmt und miteinander in Beziehung gesetzt. Für die Bestimmung der gebildeten Amplifikate sind dem Fachmann eine Reihe von unterschiedlichen Verfahren bekannt, einschließlich spektroskopischer Verfahren, der Anfärbung mittels Ethidiumbromid oder Silber und densitometrischer Bestimmung, oder der radioaktiven Markierung mit anschließender Bestimmung über, z.B., Szintillationsmessung.

In einer bevorzugten Ausführungsform erfolgt die Bestimmung der in den beiden Amplifikationsschritten gebildeten Amplifikate mit Hilfe der real time PCR während der Bildung der Amplifikate selbst. In einer weiteren bevorzugten Ausführungsform erfolgt die Bestimmung der in den beiden Amplifikationsschritten gebildeten Amplifikate simultan während einer real time PCR.

Nach der Bestimmung der in beiden Amplifikationsschritten gebildeten Amplifikate wird der Wert für das Amplifikat im zweiten Amplifikationsschritt (Schritt b)) mittels des Werts für das Amplifikat im ersten Amplifikationsschritts (Schritt a)) normiert; z.B. durch Division oder Subtraktion der ermittelten Werte. Hierdurch wird ein auf das Gesamtvorkommen des untersuchten DNA-Bereichs normierter Methylierungsgrad bestimmt (d.h. die normierte (De)-Methylierung), der als repräsentativ für den Methylierungsgrad des Gesamtgenoms angenommen werden kann.

In einer bevorzugten Ausführungsform erfolgten die Amplifikationen der Schritte a) und b) sowie die Bestimmung der gebildeten Amplifikate mittels der real time PCR. Hierbei werden cycle threshold (ct) Werte bestimmt, sowohl für das Primerpaar, welches für zumindest eine differentiell methylierte Position des Transposons spezifisch ist (Schritt b), ctₘ), als auch für das Primerpaar, welches für einen nicht differentiell methylierten Bereich des Transposons spezifisch ist (Schritt a), ctₖ). Der ct-Wert beschreibt den Zyklus der PCR, in dem das Fluoreszenzsignal erstmals signifikant über die Hintergrundfluoreszenz ansteigt und markiert damit den Anfang der exponentiellen Phase der PCR. Danach wird der ct-Wert aus Schritt a) vom ct-Wert aus Schritt b) abgezogen, um zum normierten Methylierungsgrad (Δct) zu gelangen. Δct läßt sich also berechnen als: Δct = ctₘ - ctₖ.

Wie eingangs erwähnt erlaubt das erfindungsgemäße Verfahren ebenfalls den Vergleich des Methylierungsgrads zweier Genome mit unterschiedlicher genomischer Ausstattung (z.B. bei teilweise in Tumorzellen auftretenden fehlerhaften Vervielfältigungen von einzelnen Chromosomen; Trisomien o.ä.). Bei einem Vergleich des normierten Methylierungsgrads einer DNA aus einem "Normalgenom" (Kontrolle) mit der eines Patienten können unterschiedliche Methylierungsgrade zwischen den beiden Genomen Hinweise auf eine Erkrankung geben. Das erfindungsgemäße Verfahren zur Bestimmung eines relativen DNA-Methylierungsgrads ist damit für die (klinische) Diagnostik von immenser Bedeutung.

Ein zweiter Aspekt der vorliegenden Erfindung betrifft daher ein Verfahren zur Bestimmung des relativen DNA-Methylierungsgrads umfassend die Schritte: a) Bestimmung des normierten Methylierungsgrads gemäß der Schritte a) bis c) des ersten Aspekts der Erfindung für eine erste DNA und eine zweite DNA; und b) Bestimmung des relativen DNA-Methylierungsgrads über das Verhältnis der für die erste und die zweite DNA bestimmten normierten Methylierungsgrade.

In anderen Worten wird das oben beschriebene Verfahren des ersten Aspekts der Erfindung für zwei verschiedene DNAs, die beispielsweise aus unterschiedlichen (klinischen) Proben stammen können, durchgeführt und die erhaltenen normierten Methylierungsgrade der beiden DNAs zueinander ins Verhältnis gesetzt. Aus dem Verhältnis der beiden Methylierungsgrade zueinander können dann Rückschlüsse und/oder Diagnosen gestellt werden, zum Beispiel hinsichtlich einer Krebserkrankung.

In einer Ausführungsform wird der relative Methylierungsgrad von mehr als zwei DNAs bestimmt. In einer weiteren Ausführungsform wird der Methylierungsgrad von zumindest einer zu untersuchenden DNA mit einem Mittelwert der Methylierungsgrade aus mehr als einem "Normalgenom" ins Verhältnis gesetzt. Zum Beispiel wird dazu der normierte Methylierungsgrad der DNA von ≥ 10, ≥ 50, oder ≥ 100 gesunden Probanden ermittelt und der daraus errechnete Mittelwert mit dem normierten DNA-Methylierungsgrad eines Patienten ins Verhältnis gesetzt. In einer anderen Ausführungsform wird der normierte DNA-Methylierungsgrad eines Patienten mit dem normierten DNA-Methylierungsgrad einer einzelnen Probe oder dem durchschnittlichen normierten DNA-Methylierungsgrad mehrerer Proben/DNAs (vorzugsweise ≥ 10, ≥50, oder ≥ 100) ins Verhältnis gesetzt, wobei diese letzteren Proben DNA enthalten, die das Methylierungsmuster einer Erkrankung aufweist.

In einer Ausführungsform stammt zumindest eine der beiden DNAs aus einer Probe; vorzugsweise wurde die DNA aus dieser Probe isoliert. In einer bevorzugten Ausführungsform stammen beide DNAs aus jeweils einer Probe. In einer weiteren bevorzugten Ausführungsform ist die erste Probe eine Probe eines gesunden Individuums, während die zweite Probe die Probe eines Patienten ist. In einer weiteren bevorzugten Ausführungsform ist die erste Probe eine Probe umfassend zumindest eine Tumorzelle und die zweite Probe die Probe eines Patienten. Die erste Probe/DNA dient damit als Negativ- bzw. Positivkontrolle mit der die Probe des Patienten verglichen wird. In einer weiteren Ausführungsform ist die Positivkontrolle HT1376-DNA.

In einer weiteren Ausführungsform wurde die Bestimmung des normierten Methylierungsgrads einer der beiden DNAs bereits mehr als einen Tag, eine Woche, einen Monat oder ein Jahr vor der Bestimmung des normierten Methylierungsgrads der zweiten DNA durchgeführt.

In einer weiteren Ausführungsform ist zumindest eine der beiden Proben ausgewählt aus der Gruppe bestehend aus einer Blutprobe, einer Gewebeprobe, einer Speichelprobe, einer Urinprobe, einem Abstrich und einer Stuhlprobe. In einer bevorzugten Ausführungsform ist die Probe eine Urinprobe.

In einem weiteren Schritt des erfindungsgemäßen Verfahrens erfolgt die Bestimmung des relativen DNA-Methylierungsgrads über das Verhältnis der für die erste und für die zweite DNA bestimmten normierten Methylierungsgrade, z.B. durch Division oder Subtraktion der ermittelten Werte.

In einer bevorzugten Ausführungsform erfolgte die Bestimmung des normierten Methylierungsgrads wie oben beschrieben mittels der real time PCR. Wird die Differenz aus dem Δct Wert der zweiten DNA (Δct₂), die zum Beispiel aus einer zu untersuchenden Patientenprobe stammen kann und dem Δct Wert der ersten DNA (Δct₁), die zum Beispiel aus einer Referenzprobe stammen kann als ΔΔct = Δct₂ - Δct₁ berechnet, so kann der relative Methylierungsgrad der zweiten DNA angegeben werden als 2^{-ΔΔct}. Hierbei wird die relative Methylierung der zweiten DNA zur ersten DNA errechnet, falls zur Amplifikation Primer verwendet wurden, die für ein Cytosin eines CpG spezifisch waren und es wird die relative Demethylierung berechnet, falls zur Amplifikation Primer verwendet wurden, die für das bisulfitierte Cytosin eines CpG spezifisch waren.

Dem Fachmann ist bekannt, dass das Verhältnis der in den beiden Amplifikationsschritten gebildeten Amplifikate mittels wiederholter Messung und Mittelwertbildung bestimmt werden kann, um die Genauigkeit des Verfahrens zu erhöhen. In einer Ausführungsform der Erfindung wird daher ein Mittelwert von mehreren für eine DNA bestimmten Amplifikatmengen oder ct-Werten errechnet.

In einer weiteren Ausführungsform offenbart die Erfindung ein Verfahren zum Nachweis oder zur Diagnose einer Erkrankung, die mit einer veränderten DNA-Methylierung in Verbindung gebracht wird. In einer bevorzugten Ausführungsform ist eine solche Erkrankung ein Tumor. In einer weiteren bevorzugten Ausführungsform wird hierbei der relative Methylierungsgrad der DNA aus einer Referenzprobe und einer zu untersuchenden Probe (z.B. aus einer der oben aufgeführten Proben wie einer Urin- oder Speichelprobe aus einem Patienten) gebildet. In einer bevorzugten Ausführungsform wird der Tumor bei einem Individuum oder in einer Probe eines Individuums diagnostiziert/nachgewiesen.

In einer Ausführungsform der Erfindung stammt die Referenzprobe aus einem gesunden Individuum und/oder die daraus gewonnene DNA weist einen Methylierungsgrad auf, der bei Abwesenheit eines Tumors vorherrscht. In einer anderen Ausführungsform der Erfindung stammt die Referenzprobe aus einem an einem Tumor erkrankten Individuum und/oder sie weist einen Methylierungsgrad auf, der bei Anwesenheit eines Tumors vorherrscht. In einer bevorzugten Ausführungsform der Erfindung stammt die Referenzprobe aus einem erkrankten Individuum, bei dem die Tumorerkrankung typisiert wurde. Die Referenzprobe kann auch aus kultivierten und vorzugsweise typisierten Tumorzellen bestehen, wie zum Beispiel HT1376 Zellen. Wie oben erwähnt können für die Referenzprobe auch Mittelwerte aus mehreren Referenzproben verwendet werden.

In einer Ausführungsform der Erfindung ist die Gewinnung der Proben aus dem Individuum Teil der erfindungsgemäßen Verfahren, in einer weiteren besonderen Ausführungsform der Erfindung ist die Gewinnung der Proben aus dem Individuum nicht Teil der erfindungsgemäßen Verfahren.

Weicht der normierte DNA-Methylierungsgrad der Patientenprobe von dem normierten DNA-Methylierungsgrad der Referenzprobe ab, d.h. ergibt zum Beispiel eine Division der beiden Werte zur Bildung des relativen DNA-Methylierungsgrads einen Wert von ungleich 1, so ist dies ein Indiz für das Vorliegen einer Erkrankung, die mit einer veränderten DNA-Methylierung in Verbindung gebracht wird; vorzugsweise einem Tumor.

In einer Ausführungsform deutet eine erniedrigte DNA-Methylierung, eine erniedrigte DNA-Demethylierung, eine erhöhte DNA-Methylierung, oder eine erhöhte DNA-Demethylierung der DNA aus der Probe gegenüber der DNA aus der Referenz auf das Vorliegen einer solchen Erkrankung hin.

In einer bevorzugten Ausführungsform deutet eine erniedrigte DNA-Methylierung oder eine erhöhte DNA-Demethylierung der DNA aus der Probe gegenüber der DNA aus der Referenz auf das Vorliegen eines Tumors hin. In einer noch bevorzugteren Ausführungsform der Erfindung korreliert die Differenz in der Erniedrigung der DNA-Methylierung bzw. der Erhöhung der DNA-Demethylierung mit der Aggressivität des Tumors.

In weiteren bevorzugten Ausführungsformen ist dieser Tumor ausgewählt aus der Gruppe bestehend aus: Blasentumor, Prostatatumor, Mammakarzinom, Bronchialkarzinom, Leukämien, Darmkrebs, Hodentumor, Nasopharyngealkarzinom, Gebährmutterhalskrebs, Pankreaskarzinom und/oder Magenkrebs.

In einem weiteren Aspekt ist die Erfindung gerichtet auf ein Oligonukleotid, welches zum Beispiel als Primer bei den Amplifikationsschritten in den Verfahren der vorliegenden Erfindung verwendet werden kann.

In einer Ausführungsform ist das Oligonukleotid spezifisch für ein Transposon oder Fragment davon, wobei das Transposon ausgewählt ist aus der Gruppe bestehend aus einem LINE Element, einem Alu Element, einem HERV Element, ein HERV-K Element oder einem Fragment derselben. In einer besonderen Ausführungsform ist das Transposon ein LINE-1 Element oder ein Fragment davon. Noch bevorzugter ist das Fragment des Transposons der Promotorbereich des LINE-1 Elements. In einer bevorzugten Ausführungsform ist das Oligonukleotid sequenzidentisch oder komplementär zum sense oder dem antisense Strang des bisulfitierten Transposons und umfasst zumindest eine differentiell methylierte Position des Transposons. In einer weiteren bevorzugten Ausführungsform ist das Oligonukleotid sequenzidentisch oder komplementär zum sense oder dem antisense Strang des bisulfitierten Transposons und umfasst keine differentiell methylierte Position des Transposons.

In einer bevorzugten Ausführungsform umfasst das Oligonukleotid zumindest eine differentiell methylierte Position des Transposons. In einer weiteren Ausführungsform ist das Oligonukleotid für einen Bereich des Transposons spezifisch, der stets unmethyliert vorliegt; bevorzugt für einen Bereich, der keine Cytosine von CpG-Dinukleotiden enthält.

In einer weiteren bevorzugten Ausführungsform hat das Oligonukleotid eine Länge von ≥ 15 Nukleotide; bevorzugt ≥ 18, ≥ 19, ≥ 20, ≥21, ≥ 22, ≥ 23, ≥ 24, oder ≥ 25 Nukleotide. In einer weiteren bevorzugten Ausführungsform ist das Oligonukleotid ≥ 18 und ≤ 35 Nukleotide lang; noch bevorzugter ist es ≥ 20 und ≤ 30 Nukleotide lang.

In einer weiteren Ausführungsform weist das Oligonukleotid die Sequenz auf, die ausgewählt ist aus der Gruppe bestehend aus SEQ ID NO 3 - 1415.

In einer weiteren Ausführungsform umfasst das Oligonukleotid eine Sequenz, die ausgewählt ist aus der Gruppe bestehend aus SEQ ID NO 3 - 1048, wobei die Sequenz vom 5'-Ende her und/oder vom 3'-Ende her verkürzt oder (gemäß des jeweiligen Transposons) verlängert werden kann. In einer bevorzugten Ausführungsform ist das erfindungsgemäße Oligonukleotid um zwischen 1-20 Nukleotide vom 5'-Ende und/oder vom 3'-Ende her verlängert; noch bevorzugter ist das Oligonukleotid um zwischen 5 - 15 Nukleotide vom 5 `-Ende und/oder vom 3'-Ende her verlängert. In einer anderen Ausführungsform ist das Oligonukleotid um bis zu insgesamt 5 Nukleotide vom 5`-Ende und/oder 3`-Ende her verkürzt, wobei das Oligonukleotid immer für zumindest ein CpG oder bisulfitiertes CpG spezifisch bleibt.

In einer weiteren Ausführungsform umfasst das Oligonukleotid das zumindest eine für eine differentiell methylierte Position spezifische Nukleotid an einer beliebigen Position im Oligonukleotid, d.h. am 5`-Ende des Oligonukleotids, am 3'-Ende oder jeder Position dazwischen. In einer besonders bevorzugten Ausführungsform liegt das zumindest eine, für eine differentiell methylierte Position spezifische Nukleotid am 3'-Ende des Oligonukleotids. Dies hat den Vorteil einer erhöhten Spezifität.

In weiteren Ausführungsformen ist das Oligonukleotid ausgewählt aus der Gruppe bestehend aus SEQ ID NO 3 - 436; bevorzugter SEQ ID NO 3 - 112, oder SEQ ID NO 113 - 220, oder SEQ ID NO 221 - 336, oder SEQ ID NO 337 - 436; noch bevorzugter SEQ ID NO 3 - 57, oder SEQ ID NO 58 - 112, oder SEQ ID NO 113 - 166, oder SEQ ID NO 167 - 220, oder SEQ ID NO 221 - 278, oder SEQ ID NO 279 - 336, oder SEQ ID NO 337 - 386, oder SEQ ID NO 387 - 436.

In weiteren Ausführungsformen ist das Oligonukleotid ausgewählt aus der Gruppe bestehend aus SEQ ID NO 437 - 612; bevorzugter SEQ ID NO 437 -476, oder SEQ ID NO 477 - 522, oder SEQ ID NO 523 - 570, oder SEQ ID NO 571 - 612; noch bevorzugter SEQ ID NO 437 - 456, oder SEQ ID NO 457 - 476, oder SEQ ID NO 477 - 499, oder SEQ ID NO 500 - 522, oder SEQ ID NO 523 - 546, oder SEQ ID NO 547 - 570, oder SEQ ID NO 571 - 591, oder SEQ ID NO 592 - 612.

In weiteren Ausführungsformen ist das Oligonukleotid ausgewählt aus der Gruppe bestehend aus SEQ ID NO 613 - 1048; bevorzugter SEQ ID NO 613 - 708, oder SEQ ID NO 709 - 796, oder SEQ ID NO 797 - 922, oder SEQ ID NO 923 - 1048; noch bevorzugter SEQ ID NO 613 - 660, oder SEQ ID NO 661 - 708, oder SEQ ID NO 709 - 752, oder SEQ ID NO 753 - 796, oder SEQ ID NO 797 - 859, oder SEQ ID NO 860 - 922, oder SEQ ID NO 923 - 985, oder SEQ ID NO 986 - 1048.

In weiteren Ausführungsformen ist das Oligonukleotid ausgewählt aus der Gruppe bestehend aus SEQ ID NO 1049 - 1227; bevorzugter SEQ ID NO 1049 - 1145, oder SEQ ID NO 1146 - 1227; noch bevorzugter SEQ ID NO 1049 - 1096, oder SEQ ID NO 1097 - 1145, oder SEQ ID NO 1146 - 1192, oder SEQ ID NO 1193 - 1227.

In weiteren Ausführungsformen ist das Oligonukleotid ausgewählt aus der Gruppe bestehend aus SEQ ID NO 1228 - 1257; bevorzugter SEQ ID NO 1228 - 1243, oder SEQ ID NO 1244 - 1257; noch bevorzugter SEQ ID NO 1228 - 1237, oder SEQ ID NO 1238 - 1243, oder SEQ ID NO 1244 - 1250, oder SEQ ID NO 1251 - 1257.

In weiteren Ausführungsformen ist das Oligonukleotid ausgewählt aus der Gruppe bestehend aus SEQ ID NO 1258 - 1415; bevorzugter SEQ ID NO 1258 - 1323, oder SEQ ID NO 1324 - 1415; noch bevorzugter SEQ ID NO 1258 - 1289, oder SEQ ID NO 1290 - 1323, oder SEQ ID NO 1324 - 1371, oder SEQ ID NO 1372 - 1415.

Fig. 1 zeigt das Ergebnis der Bestimmung der relativen DNA-Methylierung von Tumor-DNA in verschiedenen Verdünnungsstufen (mit gesundem Urothel) gemäß einer bevorzugten Ausführungsform der Erfindung.

Fig. 2 zeigt das Ergebnis der Bestimmung der relativen DNA-Demethylierung von 4 Patientenproben im Vergleich zu gesunden Probanden gemäß einer bevorzugten Ausführungsform der Erfindung.

### BEISPIELE

### Beispiel 1

DNA aus der Urothelkarzinomzellinie HT1376-DNA, aus einem Urothelkarzinom sowie aus gesundem Blasenepithel wurden mit einem kommerziell erhältlichen Kit (Qiagen; QIAamp DNA blood kit) isoliert. Die DNA der Urothelkarzinomzellinie wurde in verschiedenen Verdünnungsstufen mit DNA aus dem gesunden Blasenepithel verdünnt. Danach erfolgte eine Bisulfitierung der DNA in den unterschiedlichen Ansätzen mittels des EpiTect Bisulfite Kit (Qiagen). Daran anschließend erfolgte die erfindungsgemäße Bestimmung des relativen Methylierungsgrads der einzelnen Proben gegenüber der DNA aus dem gesunden Blasenepithel.

Für jede Probe wurde jeder Wert dreifach bestimmt und es wurden Mittelwerte gebildet.

Die Amplifikation erfolgte mittels real time PCR. Es wurden Primer verwendet, die für folgende LINE-1 Promotorsequenzen spezifisch waren:

| | |
|---|---|
| 5`-GCGCGAGTCGAAGTAGGGC | für den forward-Primer |
| 5`-CTCCGAACCAAATATAAAATATAATCTCG | für den reverse-Primer |

Diese beiden Primer schließen einen 193 bp Bereich des LINE-1 Elements ein und sind spezifisch für methylierte DNA.

Für den stets unmethylierten Bereich wurden Primer mit den folgenden Sequenzen verwendet:

| | |
|---|---|
| 5'-AGGTTTTATTTTTGGGGGTAGGGTATAG | als forward-Primer |
| 5`-CCCCTACTAAAAAATACCTCCCAATTAAAC | als reverse-Primer |

Die PCR wurde mit folgenden Bedingungen (pro Reaktion) durchgeführt:

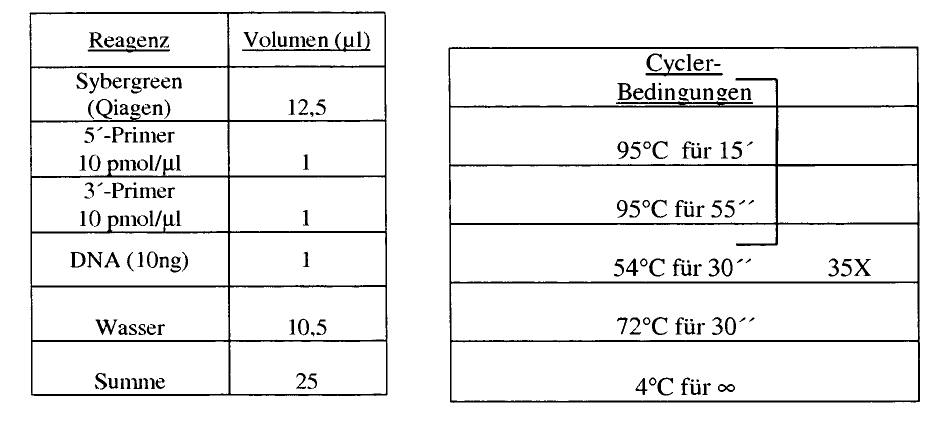

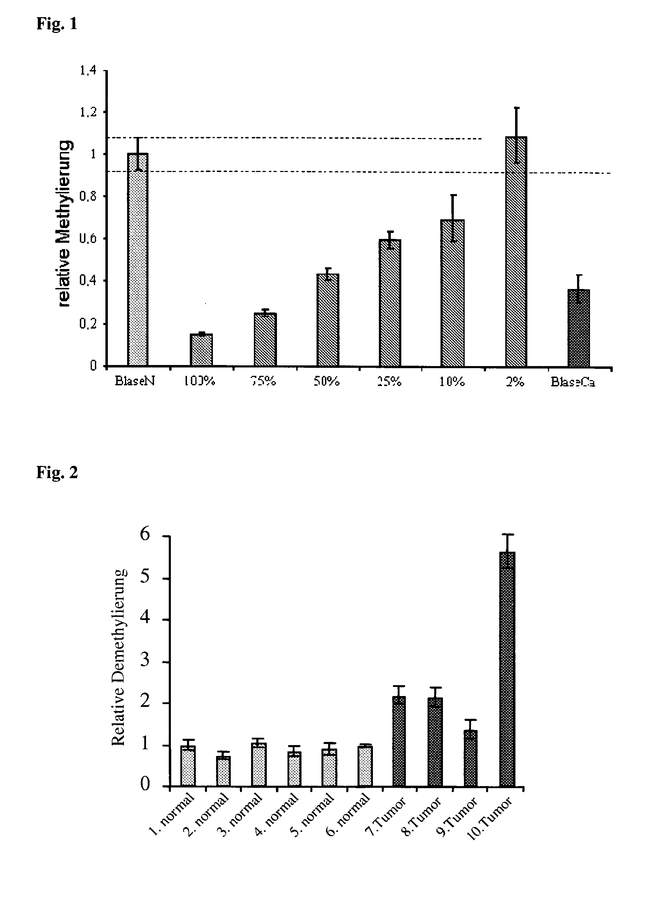

Das Ergebnis der Untersuchung unter Verwendung der Primer die für methylierte DNA spezifisch sind, ist in Fig. 1 zu sehen. Es ist zu erkennen, dass ein sicherer Nachweis von 2 ng der Tumor-DNA aus einem 1:10 Gemisch mit DNA aus gesundem Urothel erfolgen kann.

### Beispiel 2

Beispiel 1 wurde mit Primern, die spezifisch für die demethylierte LINE1 Sequenz sind, wiederholt.

Diese Primer hatten die folgenden Sequenzen:

| | |
|---|---|
| 5'-GTGTGTATTGTGTGTGAGTTGAAGTAGGGT | für den forward-Primer |
| 5' -ACCCTCCAAACCAAATATAAAATATAATCTCA | für den reverse-Primer |

Diese beiden Primer schließen einen 207 bp Bereich des LINE-1 Elements ein und sind spezifisch für demethylierte DNA.

Hierbei kamen als Proben jedoch Urinproben aus gesunden Probanden und Urothelkarzinompatienten zum Einsatz. Es wurden 1 ml Urin und 10 ng bisulfitierte DNA eingesetzt.

Fig. 2 zeigt das Ergebnis dieses Experiments. Es ist erkennbar, dass in 3 von 4 Proben der Urothelkarzinompatienten eine signifikante Hypomethylierung im Vergleich zu den verwendeten 6 Kontrollproben nachgewiesen werden konnte.

## Patentansprüche

1. Verfahren zur Bestimmung des normierten DNA-Methylierungsgrads umfassend die Schritte:
a) Quantitative Bestimmung der Gegenwart eines Transposons oder Fragments davon in einer genomischen DNA umfassend Amplifikation der nicht-bisulfitierten DNA mit zumindest einem Primerpaar, das spezifisch für ein Transposon oder Fragment davon ist, oder Amplifikation der bisulfitierten DNA mit zumindest einem Primerpaar das spezifisch für ein bisulfitierte Transposon oder Fragment davon ist, wobei die Primer keine differentiell methylierte Position des Transposons umfassen, wobei die quantitative Bestimmung mittels real-time PCR erfolgt, um einen cycle threshold-Wert zu bestimmen;
b) Quantitative Bestimmung des Vorliegens von zumindest einem differentiell methylierten C eines CpG Dinukleotids innerhalb desselben Transposons oder Fragments davon, umfassend Amplifikation der bisulfitierten DNA mit zumindest einem Primerpaar, das spezifisch für das Transposon oder Fragment davon ist und das zumindest einen Primer umfasst, der zumindest eine differentiell methylierte Position des Transposons umfasst, wobei dieselbe DNA wie im Schritt a) verwendet wird und , wobei die quantitative Bestimmung mittels real-time PCR erfolgt, um einen cycle threshold-Wert zu bestimmen; und
c) Bestimmung des normierten DNA-Methylierungsgrads über das Verhältnis der in den Schritten a) und b) bestimmten cycle threshold Werte.

2. Verfahren nach Anspruch 1, wobei das Transposon oder Fragment davon ausgewählt ist aus der Gruppe bestehend aus einem LINE Element, einem LINE-1 Element, einem Alu Element, einem HERV Element und bevorzugt dem Promotorbereich eines LINE-1 Elements.

3. Verfahren nach Anspruch 1, wobei beide Primer des Primerpaars in Schritt b) zumindest eine differentiell methylierte Position des Transposons umfassen.

4. Verfahren nach einem der vorhergenden Ansprüche, wobei der Primer in Schritt b) 2, 3, oder 4 differentiell methylierte Positionen des Transposons umfasst.

5. Verfahren nach einem der vorhergenden Ansprüche, wobei der Primer an seinem 3'-Ende eine differentiell methylierte Position des Transposons aufweist.

6. Verfahren nach einem der vorhergenden Ansprüche, wobei der zumindest eine Primer in Schritt b) ein Oligonukleotid umfasst, das ausgewählt ist aus der Gruppe bestehend aus SEQ ID NO 3 - 1048.

7. Verfahren zur Bestimmung des relativen DNA-Methylierungsgrads umfassend die Schritte:
a) Bestimmung des Methylierungsgrads gemäß der Schritte a) bis c) nach Anspruch 1 für eine erste DNA und eine zweite DNA; und
b) Bestimmung des relativen DNA-Methylierungsgrads über das Verhältnis der für die erste und die zweite DNA bestimmten Methylierungsgrade.

8. Verfahren nach Anspruch 7 zur Diagnose einer Erkrankung, die mit einer veränderten DNA-Methylierung in Verbindung gebracht wird, wobei die erste DNA eine Referenzprobe ist und die zweite DNA aus einer zu untersuchenden Probe stammt.

9. Verfahren nach Anspruch 8, wobei die Erkrankung ein Tumor ist.

10. Verwendung zumindest eines Oligonukleotids ausgewählt aus der Gruppe bestehend aus SEQ ID NO 3 - SEQ ID NO 1415, bevorzugt SEQ ID NO 3 - 436 und/oder SEQ ID NO 1049 - 1227 zur Bestimmung des normierten DNA-Methylierungsgrads gemäß Anspruch 1.

## Claims

1. A process for determining the normalized DNA methylation level, comprising the steps:
a) quantitative determination of the presence of a transposon or fragment thereof in a genomic DNA, comprising the amplification of the non-bisulfited DNA with at least one pair of primers that is specific for a transposon or fragment thereof, or the amplification of the bisulfited DNA with at least one pair of primers that is specific for a bisulfited transposon or fragment thereof, wherein said primers do not include a differentially methylated position of the transposon, wherein said quantitative determination is effected by real-time PCR to determine a cycle threshold value;
b) quantitative determination of the presence of at least one differentially methylated C of a CpG dinucleotide within the same transposon or fragment thereof, comprising the amplification of the bisulfited DNA with at least one pair of primers that is specific for a transposon or fragment thereof and that includes at least one primer that comprises at least one differentially methylated position of the transposon, wherein the same DNA as in step a) is used, and wherein said quantitative determination is effected by real-time PCR to determine a cycle threshold value; and
c) determination of the normalized DNA methylation level via the ratio of the cycle threshold values determined in steps a) and b).

2. The process according to claim 1, wherein said transposon or fragment thereof is selected from the group consisting of a LINE element, a LINE-1 element, an Alu element, a HERV element and preferably the promoter region of a LINE-1 element.

3. The process according to claim 1, wherein both primers of the pair of primers in step b) comprise at least one differentially methylated position of the transposon.

4. The process according to any of the preceding claims, wherein said primer in step b) comprises 2, 3 or 4 differentially methylated positions of the transposon.

5. The process according to any of the preceding claims, wherein said primer includes a differentially methylated position of the transposon at its 3' terminus.

6. The process according to any of the preceding claims, wherein said at least one primer in step b) comprises an oligonucleotide selected from the group consisting of SEQ ID Nos. 3-1048.

7. A process for determining the relative DNA methylation level, comprising the steps:
a) determination of the methylation level according to steps a) to c) of claim 1 for a first DNA and a second DNA; and
b) determination of the relative DNA methylation level via the ratio of the methylation levels determined for the first and second DNAs.

8. The process according to claim 7 for diagnosing a disease related to an altered DNA methylation, wherein said first DNA is a reference sample and said second DNA is derived from a sample to be examined.

9. The process according to claim 8, wherein said disease is a tumor.

10. Use of at least one oligonucleotide selected from the group consisting of SEQ ID No. 3 to SEQ ID No. 1415, preferably SEQ ID Nos. 3 to 436 and/or SEQ ID Nos. 1049 to 1227, for determining the normalized DNA methylation level according to claim 1.

## Revendications

1. Procédé de détermination du degré de méthylation normalisé de l'ADN comprenant les étapes suivantes:
a) détermination quantitative de la présence d'un transposon ou d'un fragment de celui-ci dans un ADN génomique comprenant l'amplification de l'ADN non traité par un bisulfite avec au moins une paire d'amorces spécifique pour un transposon ou un fragment de celui-ci, ou l'amplification de l'ADN traité par un bisulfite avec au moins une paire d'amorces spécifique pour un transposon bisulfité ou un fragment de celui-ci, dans lequel les amorces ne comprennent aucune position méthylée différentiellement du transposon, dans lequel la détermination quantitative est réalisée au moyen d'une PCR en temps réel, afin de déterminer une valeur de cycle seuil ;
b) détermination quantitative de la présence d'au moins un C méthylé différentiellement d'un dinucléotide CpG à l'intérieur du même transposon ou fragment de celui-ci, comprenant l'amplification de l'ADN traité par un bisulfite avec au moins une paire d'amorces spécifique pour le transposon ou le fragment de celui-ci et la paire présentant au moins une amorce, qui comprend au moins une positions méthylée différentiellement du transposon, dans lequel le même ADN que dans l'étape a) est mis en oeuvre et, dans lequel la détermination quantitative est réalisée au moyen d'une PCR en temps réel, afin de déterminer une valeur de cycle seuil ; et
c) détermination du degré de méthylation normalisé de l'ADN par l'intermédiaire du rapport entre les valeurs de cycle seuil déterminées dans les étapes a) et b).

2. Procédé selon la revendication 1, dans lequel le transposon ou le fragment de celui-ci est choisi dans le groupe formé par un élément LINE, un élément LINE-1, un élément Alu, un élément HERV et, de préférence, la région du promoteur d'un élément LINE-1.

3. Procédé selon la revendication 1, dans lequel les deux amorces de la paire d'amorces comprennent, dans l'étape b), au moins une position méthylée différentiellement du transposon.

4. Procédé selon l'une quelconque des revendications précédentes, dans lequel l'amorce dans l'étape b) 2, 3, ou 4 comprend des positions méthylées différentiellement du transposon.

5. Procédé selon l'une quelconque des revendications précédentes, dans lequel l'amorce, au niveau de son extrémité 3', présente une position méthylée différentiellement du transposon.

6. Procédé selon l'une quelconque des revendications précédentes, dans lequel la au moins une amorce dans l'étape b) comprend un oligonucléotide choisi dans le groupe formé par SEQ ID NO 3 à 1048.

7. Procédé de détermination du degré de méthylation relatif de l'ADN comprenant les étapes suivantes :
a) détermination du degré de méthylation selon les étapes a) à c) de la revendication 1 pour un premier ADN et un deuxième ADN ; et
b) détermination du degré de méthylation relatif de l'ADN par l'intermédiaire du rapport des degrés de méthylation déterminés pour le premier ADN et pour le deuxième ADN.

8. Procédé selon la revendication 7 pour le diagnostic d'une maladie qui est en rapport avec une méthylation modifiée de l'ADN, dans lequel le premier ADN est un échantillon de référence et le deuxième ADN provient d'un échantillon à examiner.

9. Procédé selon la revendication 8, dans lequel la maladie est une tumeur.

10. Utilisation d'au moins un oligonucléotide choisi dans le groupe formé par SEQ ID NO 3 à SEQ ID NO 1415, de préférence SEQ ID NO 3 à 436 et/ou SEQ ID NO 1049 à 1227 pour la détermination du degré de méthylation normalisé de l'ADN selon la revendication 1.


### SEQUENCE LISTING

<110> Heinrich-Heine-Universität Düsseldorf, Forschungs- und Technologietransfer
<120> Bestimmung des DNA-Methylierungsgrads
<130> UD40114
<160> 1415
<170> PatentIn version 3.3
<210> 1
   <211> 420
   <212> DNA
   <213> SEQ ID NO1
<400> 1
   ggggggagga gttaagatgg tcgaatagga atagtttcgg tttatagttt ttagcgtgag      60
   cgacgtagaa gacggtgatt tttgtatttt tatttgaggt atcgggttta ttttattagg     120
   gagtgttaga tagtgggcgt aggttagtgt gtgtgcgtat cgtgcgcgag tcgaagtagg     180
   gcgaggtatt gttttatttg ggaagcgtaa ggggttaggg agtttttttt ttgagttaaa     240
   gaaaggggtg acggtcgtat ttggaaaatc gggttatttt tattcgaata ttgcgttttt     300
   tagatcggtt taagaaacgg cgtattacga gattatattt tatatttggt tcggagggtt     360
   ttacgtttac ggaatttcgt tgattgttag tatagtagtt tgagattaaa ttgtaaggcg     420
<210> 2
   <211> 420
   <212> DNA
   <213> SEQ ID NO2
<400> 2
   ggggggagga gttaagatgg ttgaatagga atagttttgg tttatagttt ttagtgtgag      60
   tgatgtagaa gatggtgatt tttgtatttt tatttgaggt attgggttta ttttattagg     120
   gagtgttaga tagtgggtgt aggttagtgt gtgtgtgtat tgtgtgtgag ttgaagtagg     180
   gtgaggtatt gttttatttg ggaagtgtaa ggggttaggg agtttttttt ttgagttaaa     240
   gaaaggggtg atggttgtat ttggaaaatt gggttatttt tatttgaata ttgtgttttt     300
   tagattggtt taagaaatgg tgtattatga gattatattt tatatttggt ttggagggtt     360
   ttatgtttat ggaattttgt tgattgttag tatagtagtt tgagattaaa ttgtaaggtg     420
<210> 3
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 3
   ggggaggagt taagatggtc 20
<210> 4
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 4
   ggtcgaatag gaatagtttc 20
<210> 5
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 5
   ttcggtttat agtttttagc 20
<210> 6
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 6
   ttatagtttt tagcgtgagc 20
<210> 7
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 7
   tagtttttag cgtgagcgac 20
<210> 8
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 8
   gcgtgagcga cgtagaagac 20
<210> 9
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 9
   gtatttttat ttgaggtatc 20
<210> 10
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 10
   gggagtgtta gatagtgggc 20
<210> 11
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 11
   gcgtaggtta gtgtgtgtgc 20
<210> 12
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 12
   ggttagtgtg tgtgcgtatc 20
<210> 13
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 13
   agtgtgtgtg cgtatcgtgc 20
<210> 14
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 14
   tgtgtgtgcg tatcgtgcgc 20
<210> 15
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 15
   gtgcgtatcg tgcgcgagtc 20
<210> 16
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 16
   tgcgcgagtc gaagtagggc 20
<210> 17
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 17
   tattgtttta tttgggaagc 20
<210> 18
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 18
   gagttaaaga aaggggtgac 20
<210> 19
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 19
   taaagaaagg ggtgacggtc 20
<210> 20
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 20
   acggtcgtat ttggaaaatc 20
<210> 21
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 21
   aaatcgggtt atttttattc 20
<210> 22
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 22
   tatttttatt cgaatattgc 20
<210> 23
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 23
   aatattgcgt tttttagatc 20
<210> 24
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 24
   tttagatcgg tttaagaaac 20
<210> 25
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 25
   agatcggttt aagaaacggc 20
<210> 26
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 26
   tttaagaaac ggcgtattac 20
<210> 27
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 27
   ttatatttta tatttggttc 20
<210> 28
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 28
   tttggttcgg agggttttac 20
<210> 29
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 29
   tcggagggtt ttacgtttac 20
<210> 30
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 30
   ttttacgttt acggaatttc 20
<210> 31
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 31
   ttgagattaa attgtaaggc 20
<210> 32
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 32
   ttaaattgta aggcggtaac 20
<210> 33
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 33
   aacgaggttg ggggaggggc 20
<210> 34
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 34
   aggttggggg aggggcgttc 20
<210> 35
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 35
   tttaggtaaa taaagtagtc 20
<210> 36
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 36
   ataaagtagt cgggaagttc 20
<210> 37
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 37
   agtagtggtt tttttagtac 20
<210> 38
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 38
   gtagttggag atttgagaac 20
<210> 39
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 39
   gtttttgatt tttgattttc 20
<210> 40
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 40
   ggtatattga tattttatac 20
<210> 41
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 41
   ttagaaagga tatttatatc 20
<210> 42
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 42
   aaaattggaa attttaaaac 20
<210> 43
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 43
   gaaattttaa aacgtagagc 20
<210> 44
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 44
   tttttttttt ttaaaggaac 20
<210> 45
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 45
   ggatggagaa tgattttgac 20
<210> 46
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 46
   gagagaagaa ggttttagac 20
<210> 47
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 47
   attaaattat tttgagttac 20
<210> 48
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 48
   ggagttgaaa attaaggttc 20
<210> 49
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 49
   aattaaggtt cgagaattac 20
<210> 50
   <211> 19
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 50
   tgtagaagtt ttaggagtc 19
<210> 51
   <211> 19
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 51
   gaagttttag gagtcgatg 19
<210> 52
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 52
   tgaaatgaat gaaatgaagc 20
<210> 53
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 53
   tgtgaaaaga ttaaatttac 20
<210> 54
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 54
   atttagtaag gtaggttaac 20
<210> 55
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 55
   atttaggaaa tatagagaac 20
<210> 56
   <211> 18
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 56
   gttataaaga tatttttc 18
<210> 57
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 57
   ggtagttaga gagaaaggtc 20
<210> 58
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 58
   ggggaggagt taagatggtt 20
<210> 59
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 59
   ggttgaatag gaatagtttt 20
<210> 60
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 60
   tttggtttat agtttttagt 20
<210> 61
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 61
   ttatagtttt tagtgtgagt 20
<210> 62
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 62
   tagtttttag tgtgagtgat 20
<210> 63
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 63
   gtgtgagtga tgtagaagat 20
<210> 64
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 64
   gtatttttat ttgaggtatt 20
<210> 65
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 65
   gggagtgtta gatagtgggt 20
<210> 66
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements

<400> 66
   gtgtaggtta gtgtgtgtgt 20
<210> 67
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 67
   ggttagtgtg tgtgtgtatt 20
<210> 68
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 68
   agtgtgtgtg tgtattgtgt 20
<210> 69
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 69
   tgtgtgtgtg tattgtgtgt 20
<210> 70
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 70
   gtgtgtattg tgtgtgagtt 20
<210> 71
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 71
   tgtgtgagtt gaagtagggt 20
<210> 72
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 72
   tattgtttta tttgggaagt 20
<210> 73
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 73
   gagttaaaga aaggggtgat 20
<210> 74
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 74
   taaagaaagg ggtgatggtt 20
<210> 75
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 75
   atggttgtat ttggaaaatt 20
<210> 76
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 76
   aaattgggtt atttttattt 20
<210> 77
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 77
   tatttttatt tgaatattgt 20
<210> 78
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 78
   aatattgtgt tttttagatt 20
<210> 79
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 79
   tttagattgg tttaagaaat 20
<210> 80
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 80
   agattggttt aagaaatggt 20
<210> 81
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 81
   tttaagaaat ggtgtattat 20
<210> 82
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 82
   ttatatttta tatttggttt 20
<210> 83
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 83
   tttggtttgg agggttttat 20
<210> 84
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 84
   ttggagggtt ttatgtttat 20
<210> 85
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 85
   ttttatgttt atggaatttt 20
<210> 86
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 86
   ttgagattaa attgtaaggt 20
<210> 87
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 87
   ttaaattgta aggtggtaat 20
<210> 88
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 88
   aatgaggttg ggggaggggt 20
<210> 89
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 89
   aggttggggg aggggtgttt 20
<210> 90
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 90
   tttaggtaaa taaagtagtt 20
<210> 91
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 91
   ataaagtagt tgggaagttt 20
<210> 92
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 92
   agtagtggtt tttttagtat 20
<210> 93
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 93
   gtagttggag atttgagaat 20
<210> 94
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 94
   gtttttgatt tttgattttt 20
<210> 95
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 95
   ggtatattga tattttatat 20
<210> 96
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 96
   ttagaaagga tatttatatt 20
<210> 97
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 97
   aaaattggaa attttaaaat 20
<210> 98
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 98
   gaaattttaa aatgtagagt 20
<210> 99
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 99
   tttttttttt ttaaaggaat 20
<210> 100
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 100
   ggatggagaa tgattttgat 20
<210> 101
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 101
   gagagaagaa ggttttagat 20
<210> 102
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 102
   attaaattat tttgagttat 20
<210> 103
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 103
   ggagttgaaa attaaggttt 20
<210> 104
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 104
   aattaaggtt tgagaattat 20
<210> 105
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 105
   atgtagaagt tttaggagtt 20
<210> 106
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 106
   gaagttttag gagttgatgt 20
<210> 107
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 107
   tgaaatgaat gaaatgaagt 20
<210> 108
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 108
   tgtgaaaaga ttaaatttat 20
<210> 109
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 109
   atttagtaag gtaggttaat 20
<210> 110
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 110
   atttaggaaa tatagagaat 20
<210> 111
   <211> 18
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 111
   gttataaaga tatttttt 18
<210> 112
   <211> 20
   <212> DNA
   <213> SEQUENZIDENTISCHER Primer, spezifisch für den Bisulfit-konvertierten DEMETHYLIERTEN SENSE Strang des Promotorbereichs des LINE-1 Elements
<400> 112
   ggtagttaga gagaaaggtt 20
<210> 113
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 113
   tttcctttaa aaataacccg 20
<210> 114
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 114
   tcttaaaatt actcttctcg 20
<210> 115
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 115
   cgaaaaatat ctttataacg 20
<210> 116
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 116
   atatttccta aatctaaacg 20
<210> 117
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 117
   tcaaatacac caatcaaacg 20
<210> 118
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 118
   tctctaaact tcccttctcg 20
<210> 119
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 119
   ccctttcttc caattaatcg 20
<210> 120
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 120
   tcttccaatt aatcgcatcg 20
<210> 121
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 121
   aaacttctac attcttcacg 20
<210> 122
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 122
   cattcttcac gtaattctcg 20
<210> 123
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 123
   ttaatttaaa tatcctcccg 20
<210> 124
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 124
   aactcaaaat aatttaatcg 20
<210> 125
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 125
   aaccttcttc tctcaactcg 20
<210> 126
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 126
   attactaata aaaaactacg 20
<210> 127
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 127
   cctttaaaaa aaaaaaaacg 20
<210> 128
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 128
   aaaaaaaaaa acgctctacg 20
<210> 129
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 129
   aatatacaaa taaattttcg 20
<210> 130
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 130
   tctattaaaa taccctaccg 20
<210> 131
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 131
   cctcccaatt aaactactcg 20
<210> 132
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 132
   aaaaacaatc tatctacccg 20
<210> 133
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 133
   ttctcaaatc tccaactacg 20
<210> 134
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 134
   aataaactcc acccaattcg 20
<210> 135
   <211> 20
   <212> DNA
   <213> KOMPLEMENTÄRE Primersequenz, spezifische für den Bisulfit-konvertierten METHYLIERTEN SENSE Strang des Promotorbereichs des LINE-1 Elements

<400> 135
   cacccaattc gaacttcccg 20
<210> 136
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 136
   aacctaaaca ataacgaacg 20
<210> 137
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 137
   acgcccctcc cccaacctcg 20
<210> 138
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 138
   ctcccccaac ctcgttaccg 20
<210> 139
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 139
   tactatacta acaatcaacg 20
<210> 140
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 140
   taacaatcaa cgaaattccg 20
<210> 141
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 141
   tcaacgaaat tccgtaaacg 20
<210> 142
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 142
   cgtaaacgta aaaccctccg 20
<210> 143
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 143
   aaatataaaa tataatctcg 20
<210> 144
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 144
   aaatataatc tcgtaatacg 20
<210> 145
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 145
   tataatctcg taatacgccg 20
<210> 146
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 146
   atacgccgtt tcttaaaccg 20
<210> 147
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 147
   ttaaaccgat ctaaaaaacg 20
<210> 148
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 148
   tctaaaaaac gcaatattcg 20
<210> 149
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 149
   attcgaataa aaataacccg 20
<210> 150
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 150
   aacccgattt tccaaatacg 20
<210> 151
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 151
   cgattttcca aatacgaccg 20
<210> 152
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 152
   aaactcccta accccttacg 20
<210> 153
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 153
   ccaaataaaa caatacctcg 20
<210> 154
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 154
   caatacctcg ccctacttcg 20
<210> 155
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 155
   cctcgcccta cttcgactcg 20
<210> 156
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 156
   tcgccctact tcgactcgcg 20
<210> 157
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 157
   cctacttcga ctcgcgcacg 20
<210> 158
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 158
   ttcgactcgc gcacgatacg 20
<210> 159
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 159
   cgcacacaca ctaacctacg 20
<210> 160
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 160
   tccctaataa aataaacccg 20
<210> 161
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 161
   taaaaataca aaaatcaccg 20
<210> 162
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 162
   aaaaatcacc gtcttctacg 20
<210> 163
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 163
   aatcaccgtc ttctacgtcg 20
<210> 164
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 164
   cgtcttctac gtcgctcacg 20
<210> 165
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 165
   acgctaaaaa ctataaaccg 20
<210> 166
   <211> 20
   <212> DNA
   <213> KOMPLEMENTÄRE Primersequenz, spezifisch für den Bisulfit-konvertierten METHYLIERTEN SENSE Strang des Promotorbereichs des LINE-1 Elements
<400> 166
   accgaaacta ttcctattcg 20
<210> 167
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 167
   tttcctttaa aaataaccca 20
<210> 168
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 168
   tcttaaaatt actcttctca 20
<210> 169
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 169
   caaaaaatat ctttataaca 20
<210> 170
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 170
   atatttccta aatctaaaca 20
<210> 171
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 171
   tcaaatacac caatcaaaca 20
<210> 172
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 172
   tctctaaact tcccttctca 20
<210> 173
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 173
   ccctttcttc caattaatca 20
<210> 174
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 174
   tcttccaatt aatcacatca 20
<210> 175
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 175
   aaacttctac attcttcaca 20
<210> 176
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 176
   cattcttcac ataattctca 20
<210> 177
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 177
   ttaatttaaa tatcctccca 20
<210> 178
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 178
   aactcaaaat aatttaatca 20
<210> 179
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 179
   aaccttcttc tctcaactca 20
<210> 180
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 180
   aaccttcttc tctcaactca 20
<210> 181
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 181
   cctttaaaaa aaaaaaaaca 20
<210> 182
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 182
   aaaaaaaaaa acactctaca 20
<210> 183
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 183
   aatatacaaa taaattttca 20
<210> 184
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 184
   tctattaaaa taccctacca 20
<210> 185
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 185
   cctcccaatt aaactactca 20
<210> 186
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 186
   aaaaacaatc tatctaccca 20
<210> 187
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 187
   ttctcaaatc tccaactaca 20
<210> 188
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 188
   aataaactcc acccaattca 20
<210> 189
   <211> 20
   <212> DNA
   <213> KOMPLEMENTÄRE Primersequenz, spezifisch für den Bisulfit-konvertierten DEMETHYLIERTEN SENSE Strang des *Promotorbereichs des LINE-1 Elements
<400> 189
   cacccaattc aaacttccca 20
<210> 190
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 190
   aacctaaaca ataacaaaca 20
<210> 191
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 191
   acacccctcc cccaacctca 20
<210> 192
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 192
   ctcccccaac ctcattacca 20
<210> 193
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 193
   tactatacta acaatcaaca 20
<210> 194
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 194
   taacaatcaa caaaattcca 20
<210> 195
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 195
   tcaacaaaat tccataaaca 20
<210> 196
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 196
   cataaacata aaaccctcca 20
<210> 197
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 197
   aaatataaaa tataatctca 20
<210> 198
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 198
   aaatataatc tcataataca 20
<210> 199
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 199
   tataatctca taatacacca 20
<210> 200
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 200
   atacaccatt tcttaaacca 20
<210> 201
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 201
   ttaaaccaat ctaaaaaaca 20
<210> 202
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 202
   tctaaaaaac acaatattca 20
<210> 203
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 203
   attcaaataa aaataaccca 20
<210> 204
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements

<400> 204
   aacccaattt tccaaataca 20
<210> 205
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 205
   caattttcca aatacaacca 20
<210> 206
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 206
   aaactcccta accccttaca 20
<210> 207
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 207
   ccaaataaaa caatacctca 20
<210> 208
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 208
   caatacctca ccctacttca 20
<210> 209
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 209
   cctcacccta cttcaactca 20
<210> 210
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 210
   tcaccctact tcaactcaca 20
<210> 211
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 211
   cctacttcaa ctcacacaca 20
<210> 212
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 212
   ttcaactcac acacaataca 20
<210> 213
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 213
   cacacacaca ctaacctaca 20
<210> 214
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 214
   tccctaataa aataaaccca 20
<210> 215
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 215
   taaaaataca aaaatcacca 20
<210> 216
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 216
   aaaaatcacc atcttctaca 20
<210> 217
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 217
   aatcaccatc ttctacatca 20
<210> 218
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 218
   catcttctac atcactcaca 20
<210> 219
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 219
   acactaaaaa ctataaacca 20
<210> 220
   <211> 20
   <212> DNA
   <213> KOMPLEMENTÄRE Primersequenz, spezifisch für den Bisulfit-konvertierten DEMETHYLIERTEN SENSE Strang des Promotorbereichs des LINE-1 Elements
<400> 220
   accaaaacta ttcctattca 20
<210> 221
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 221
   tgtagttttt ttttagtttc 20
<210> 222
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 222
   ttttggtatg attttgtagc 20
<210> 223
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 223
   attttgtagc ggttggtatc 20
<210> 224
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 224
   tggtttgtag ggtttttgtc 20
<210> 225
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 225
   tttttttttg agggtaattc 20
<210> 226
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 226
   gttttggagt tgtttttttc 20
<210> 227
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 227
   tgtatttttt gaatttgaac 20
<210> 228
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 228
   tttaggtata ttaattagac 20
<210> 229
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 229
   ttttttaaat tttttttttc 20
<210> 230
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 230
   attttttttt ttagttgatc 20
<210> 231
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 231
   tttttttagt tgatcgtatc 20
<210> 232
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 232
   gaggtttttg tattttttac 20
<210> 233
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 233
   gtatttttta cgtagttttc 20
<210> 234
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 234
   tttggtttga atgttttttc 20
<210> 235
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 235
   tagtttagag taatttgatc 20
<210> 236
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 236
   aagttttttt tttttagttc 20
<210> 237
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 237
   tgttgttggt gaggaattgc 20
<210> 238
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 238
   ttttttggag gaggagaggc 20
<210> 239
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 239
   gaggaggaga ggcgttttgc 20
<210> 240
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 240
   tgatgtatag atgggttttc 20
<210> 241
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 241
   gtttgttgga atattttgtc 20
<210> 242
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 242
   gttttttagt taggttgttc 20
<210> 243
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 243
   aggaggtagt ttgtttgttc 20
<210> 244
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 244
   gtttttagat ttttagttgc 20
<210> 245
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 245
   tggtgggttt tatttagttc 20
<210> 246
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 246
   ttatttagtt cgagtttttc 20
<210> 247
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 247
   aagtaagttt gggtaatggc 20
<210> 248
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 248
   aagtttgggt aatggcgggc 20
<210> 249
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 249
   ggcgtttttt ttttagtttc 20
<210> 250
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 250
   ttttttttag tttcgttgtc 20
<210> 251
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 251
   ttgttgtgtt agtaattagc 20
<210> 252
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 252
   ttagtaatta gcgagatttc 20
<210> 253
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 253
   attagcgaga tttcgtgggc 20
<210> 254
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 254
   tcgtgggcgt aggatttttc 20
<210> 255
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 255
   taggtgtggg atatagtttc 20
<210> 256
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 256
   gggatatagt ttcgtggtgc 20
<210> 257
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 257
   atatagtttc gtggtgcgtc 20
<210> 258
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 258
   ggtgcgtcgt tttttaagtc 20
<210> 259
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 259
   tttaagtcgg tttgaaaagc 20
<210> 260
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 260
   gtttgaaaag cgtaatattc 20
<210> 261
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 261
   tattcgggtg ggagtgattc 20
<210> 262
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 262
   tgattcgatt ttttaggtgc 20
<210> 263
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 263
   tcgatttttt aggtgcgatc 20
<210> 264
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 264
   ggaatttttt gattttttgc 20
<210> 265
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 265
   tttaggtgag gtaatgtttc 20
<210> 266
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 266
   gtaatgtttc gttttgtttc 20
<210> 267
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 267
   gtttcgtttt gtttcggttc 20
<210> 268
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 268
   ttcgttttgt ttcggttcgc 20

<210> 269
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 269
   ttttgtttcg gttcgcgtac 20
<210> 270
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 270
   tttcggttcg cgtacggtgc 20
<210> 271
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 271
   gcgtatatat attggtttgc 20
<210> 272
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 272
   ttttttagtg agatgaattc 20
<210> 273
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 273
   atggaaatgt agaaattatc 20
<210> 274
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 274
   tagaaattat cgttttttgc 20
<210> 275
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 275
   aaattatcgt tttttgcgtc 20
<210> 276
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 276
   tcgttttttg cgtcgtttac 20
<210> 277
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 277
   tacgttggga gttgtagatc 20
<210> 278
   <211> 20
   <212> DNA
   <213> SEQUENZIDENTISCHE Primersequenz, spezifisch für den Bisulfit-konvertierten METHYLIERTEN ANTISENSE Strang des Promotorbereichs des LINE-1 Elements.
<400> 278
   gatcggagtt gtttttattc 20
<210> 279
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 279
   tgtagttttt ttttagtttt 20
<210> 280
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 280
   ttttggtatg attttgtagt 20
<210> 281
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 281
   attttgtagt ggttggtatt 20
<210> 282
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 282
   tggtttgtag ggtttttgtt 20
<210> 283
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 283
   tttttttttg agggtaattt 20
<210> 284
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 284
   gttttggagt tgtttttttt 20
<210> 285
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 285
   tgtatttttt gaatttgaat 20
<210> 286
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 286
   tttaggtata ttaattagat 20
<210> 287
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 287
   ttttttaaat tttttttttt 20
<210> 288
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 288
   attttttttt ttagttgatt 20
<210> 289
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 289
   tttttttagt tgattgtatt 20
<210> 290
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 290
   gaggtttttg tattttttat 20
<210> 291
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 291
   gtatttttta tgtagttttt 20
<210> 292
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 292
   tttggtttga atgttttttt 20
<210> 293
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 293
   tagtttagag taatttgatt 20
<210> 294
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 294
   aagttttttt tttttagttt 20
<210> 295
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 295
   tgttgttggt gaggaattgt 20
<210> 296
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 296
   ttttttggag gaggagaggt 20
<210> 297
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 297
   gaggaggaga ggtgttttgt 20
<210> 298
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 298
   tgatgtatag atgggttttt 20
<210> 299
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 299
   gtttgttgga atattttgtt 20
<210> 300
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 300
   gttttttagt taggttgttt 20
<210> 301
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 301
   aggaggtagt ttgtttgttt 20
<210> 302
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 302
   gtttttagat ttttagttgt 20
<210> 303
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 303
   tggtgggttt tatttagttt 20
<210> 304
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 304
   ttatttagtt tgagtttttt 20
<210> 305
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 305
   aagtaagttt gggtaatggt 20
<210> 306
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 306
   aagtttgggt aatggtgggt 20
<210> 307
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 307
   ggtgtttttt ttttagtttt 20
<210> 308
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 308
   ttttttttag ttttgttgtt 20
<210> 309
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 309
   ttgttgtgtt agtaattagt 20
<210> 310
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 310
   ttagtaatta gtgagatttt 20
<210> 311
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 311
   attagtgaga ttttgtgggt 20
<210> 312
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 312
   ttgtgggtgt aggatttttt 20
<210> 313
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 313
   taggtgtggg atatagtttt 20
<210> 314
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 314
   gggatatagt tttgtggtgt 20
<210> 315
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 315
   atatagtttt gtggtgtgtt 20
<210> 316
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 316
   ggtgtgttgt tttttaagtt 20
<210> 317
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 317
   tttaagttgg tttgaaaagt 20
<210> 318
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 318
   gtttgaaaag tgtaatattt 20
<210> 319
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 319
   tatttgggtg ggagtgattt 20
<210> 320
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 320
   tgatttgatt ttttaggtgt 20
<210> 321
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 321
   ttgatttttt aggtgtgatt 20
<210> 322
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 322
   ggaatttttt gattttttgt 20
<210> 323
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 323
   tttaggtgag gtaatgtttt 20
<210> 324
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 324
   gtaatgtttt gttttgtttt 20
<210> 325
   <211> 20
   <212> DNA <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 325
   gttttgtttt gttttggttt 20
<210> 326
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 326
   tttgttttgt tttggtttgt 20
<210> 327
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 327
   ttttgttttg gtttgtgtat 20
<210> 328
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 328
   ttttggtttg tgtatggtgt 20
<210> 329
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 329
   gtgtatatat attggtttgt 20
<210> 330
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 330
   ttttttagtg agatgaattt 20
<210> 331
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements

<400> 331
   atggaaatgt agaaattatt 20
<210> 332
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 332
   tagaaattat tgttttttgt 20
<210> 333
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 333
   aaattattgt tttttgtgtt 20
<210> 334
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 334
   ttgttttttg tgttgtttat 20
<210> 335
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 335
   tatgttggga gttgtagatt 20
<210> 336
   <211> 20
   <212> DNA
   <213> SEQUENZIDENTISCHE Primersequenz, spezifisch für den Bisulfit-konvertierten DEMETHYLIERTEN ANTISENSE Strang des Promotorbereichs des LINE-1 Elements
<400> 336
   gattggagtt gtttttattt 20
<210> 337
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 337
   aaaaaaaaac caaaataacc g 21
<210> 338
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 338
   aaccgaataa aaacaactcc g 21
<210> 339
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 339
   tccgatctac aactcccaac g 21
<210> 340
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 340
   ctacaactcc caacgtaaac g 21
<210> 341
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 341
   caactcccaa cgtaaacgac g 21
<210> 342
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 342
   acgtaaacga cgcaaaaaac g 21
<210> 343
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 343
   acatttccat ctaaaatacc g *21
<210> 344
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 344
   aaaaatacca aacaataaac g 21
<210> 345
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 345
   acgcaaacca atatatatac g 21
<210> 346
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 346
   aaccaatata tatacgcacc g 21
<210> 347
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 347
   aatatatata cgcaccgtac g 21
<210> 348
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 348
   tatatatacg caccgtacgc g 21
<210> 349
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 349
   atacgcaccg tacgcgaacc g 21
<210> 350
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 350
   tacgcgaacc gaaacaaaac g 21
<210> 351
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 351
   cattacctca cctaaaaaac g 21
<210> 352
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 352
   aaatcaaaaa aaaaaataac g 21
<210> 353
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 353
   caaaaaaaaa aataacgatc g 21
<210> 354
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 354
   acgatcgcac ctaaaaaatc g 21
<210> 355
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 355
   aaatcgaatc actcccaccc g 21
<210> 356
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 356
   cactcccacc cgaatattac g 21
<210> 357
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 357
   aatattacgc ttttcaaacc g 21
<210> 358
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 358
   ttcaaaccga cttaaaaaac g 21
<210> 359
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 359
   aaaccgactt aaaaaacgac g 21
<210> 360
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 360
   cttaaaaaac gacgcaccac g 21
<210> 361
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 361
   ctatatccca cacctaactc g 21
<210> 362
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 362
   cctaactcga aaaatcctac g 21
<210> 363
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 363
   tcgaaaaatc ctacgcccac g 21
<210> 364
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 364
   tcctacgccc acgaaatctc g 21
<210> 365
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 365 21
   ctaaaatcaa actacaaaac g 21
<210> 366
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 366
   tcaaactaca aaacgacaac g 21
<210> 367
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 367
   aacgaaacta aaaaaaaaac g 21
<210> 368
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 368
   aaactaaaaa aaaaacgccc g 21
<210> 369
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 369
   cttaaataaa caaaacaacc g 21
<210> 370
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 370
   acaaaacaac cgaaaaactc g 21
<210> 371
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 371
   aacaataatt ctcccaacac g 21
<210> 372
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 372
   gcaactaaaa atctaaaaac g 21
<210> 373
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 373
   atccctaact cctaaccccc g 21
<210> 374
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 374
   aacacactaa cacctcacac g 21
<210> 375
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 375
   ccaaaaaaaa catctacacc g 21
<210> 376
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 376
   aaaactaaaa actctaaaac g 21
<210> 377
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 377
   aaaactctaa aacgcaaaac g 21
<210> 378
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 378
   ctctcctcct ccaaaaaaac g 21
<210> 379
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 379
   aaataaaaaa taattttaac g 21
<210> 380
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 380
   aaaaaaaaaa aacttcaaac g 21
<210> 381
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 381
   atcaaattac tctaaactac g 21
<210> 382
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 382
   aaaactaaaa accaaaactc g 21
<210> 383
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 383
   aaccaaaact cgaaaactac g 21
<210> 384
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 384
   atacaaaaac ctcaaaaacc g 21
<210> 385
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 385 21
   aaaacctcaa aaaccgatac g 21
<210> 386
   <211> 21
   <212> DNA
   <213> KOMPLEMENTÄRER PRIMER, spezifisch für den Bisulfit-konvertierten METHYLIERTEN ANTISENSE Strang des Promotorbereichs des Line-1 Elements
<400> 386
   taaaataaat aaaataaaac g 21
<210> 387
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 387
   aaaaaaaaac caaaataacc a 21
<210> 388
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 388
   aaccaaataa aaacaactcc a 21
<210> 389
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 389
   tccaatctac aactcccaac a 21
<210> 390
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 390
   ctacaactcc caacataaac a 21
<210> 391
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 391
   caactcccaa cataaacaac a 21
<210> 392
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 392
   acataaacaa cacaaaaaac a 21
<210> 393
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 393
   acatttccat ctaaaatacc a 21
<210> 394
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 394
   aaaaatacca aacaataaac a 21
<210> 395
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 395
   acacaaacca atatatatac a 21
<210> 396
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 396
   aaccaatata tatacacacc a 21
<210> 397
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 397
   aatatatata cacaccatac a 21
<210> 398
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 398
   tatatataca caccatacac a 21
<210> 399
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 399
   atacacacca tacacaaacc a 21

<210> 400
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 400
   tacacaaacc aaaacaaaac a 21
<210> 401
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 401
   cattacctca cctaaaaaac a 21
<210> 402
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 402
   aaatcaaaaa aaaaaataac a 21
<210> 403
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 403
   caaaaaaaaa aataacaatc a 21
<210> 404
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 404
   acaatcacac ctaaaaaatc a 21
<210> 405
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 405
   aaatcaaatc actcccaccc a 21
<210> 406
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 406
   cactcccacc caaatattac a 21
<210> 407
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 407
   aatattacac ttttcaaacc a 21
<210> 408
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 408
   ttcaaaccaa cttaaaaaac a 21
<210> 409
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 409
   aaaccaactt aaaaaacaac a 21
<210> 410
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 410
   cttaaaaaac aacacaccac a 21
<210> 411
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 411
   ctatatccca cacctaactc a 21
<210> 412
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 412
   cctaactcaa aaaatcctac a 21
<210> 413
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 413
   tcaaaaaatc ctacacccac a 21
<210> 414
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 414
   tcctacaccc acaaaatctc a 21
<210> 415
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 415
   ctaaaatcaa actacaaaac a 21
<210> 416
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 416
   tcaaactaca aaacaacaac a 21
<210> 417
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 417
   aacaaaacta aaaaaaaaac a 21
<210> 418
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 418
   aaactaaaaa aaaaacaccc a 21
<210> 419
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 419
   cttaaataaa caaaacaacc a 21
<210> 420
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 420
   acaaaacaac caaaaaactc a 21
<210> 421
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 421
   aacaataatt ctcccaacac a 21
<210> 422
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 422
   gcaactaaaa atctaaaaac a 21
<210> 423
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 423
   atccctaact cctaaccccc a 21
<210> 424
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 424
   aacacactaa cacctcacac a 21
<210> 425
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 425
   ccaaaaaaaa catctacacc a 21
<210> 426
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 426
   aaaactaaaa actctaaaac a 21
<210> 427
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 427
   aaaactctaa aacacaaaac a 21
<210> 428
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 428
   ctctcctcct ccaaaaaaac a 21
<210> 429
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 429
   aaataaaaaa taattttaac a 21
<210> 430
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 430
   aaaaaaaaaa aacttcaaac a 21
<210> 431
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 431
   atcaaattac tctaaactac a 21
<210> 432
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 432
   aaaactaaaa accaaaactc a 21
<210> 433
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 433
   aaccaaaact caaaaactac a 21
<210> 434
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 434
   atacaaaaac ctcaaaaacc a 21
<210> 435
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 435
   aaaacctcaa aaaccaatac a 21
<210> 436
   <211> 21
   <212> DNA
   <213> KOMPLEMENTÄRER PRIMER, spezifisch für den Bisulfit-konvertierten DEMETHYLIERTEN ANTISENSE Strang des Promotorbereichs des Line-1 Elements
<400> 436
   taaaataaat aaaataaaac a 21
<210> 437
   <211> 20
   <212> DNA
   <213> SEQUENZIDENTISCHE Primersequenz, spezifisch für den Bisulfit-konvertierten METHYLIERTEN SENSE Strang des Alu Elements
<400> 437
   ggtcgggcgc ggtggtttac 20
<210> 438
   <211> 20
   <212> DNA
   <213> SEQUENZIDENTISCHE Primersequenz, spezifisch für den Bisulfit-konvertierten METHYLIERTEN SENSE Strang des Alu Elements
<400> 438
   ttttagtatt ttgggaggtc 20
<210> 439
   <211> 20
   <212> DNA
   <213> SEQUENZIDENTISCHE Primersequenz, spezifisch für den Bisulfit-konvertierten METHYLIERTEN SENSE Strang des Alu Elements
<400> 439
   gtattttggg aggtcgaggc 20
<210> 440
   <211> 20
   <212> DNA
   <213> SEQUENZIDENTISCHE Primersequenz, spezifisch für den Bisulfit-konvertierten METHYLIERTEN SENSE Strang des Alu Elements
<400> 440
   tttgggaggt cgaggcgggc 20
<210> 441
   <211> 20
   <212> DNA
   <213> SEQUENZIDENTISCHE Primersequenz, spezifisch für den Bisulfit-konvertierten METHYLIERTEN SENSE Strang des Alu Elements
<400> 441
   ttatttgagg ttaggagatc 20
<210> 442
   <211> 20
   <212> DNA
   <213> SEQUENZIDENTISCHE Primersequenz, spezifisch für den Bisulfit-konvertierten METHYLIERTEN SENSE Strang des Alu Elements
<400> 442
   ggttaatatg gtgaaatttc 20
<210> 443
   <211> 20
   <212> DNA
   <213> SEQUENZIDENTISCHE Primersequenz, spezifisch für den Bisulfit-konvertierten METHYLIERTEN SENSE Strang des Alu Elements
<400> 443
   taaaaatata aaaattagtc 20
<210> 444
   <211> 20
   <212> DNA
   <213> SEQUENZIDENTISCHE Primersequenz, spezifisch für den Bisulfit-konvertierten METHYLIERTEN SENSE Strang des Alu Elements
<400> 444
   aatataaaaa ttagtcgggc 20
<210> 445
   <211> 20
   <212> DNA
   <213> SEQUENZIDENTISCHE Primersequenz, spezifisch für den Bisulfit-konvertierten METHYLIERTEN SENSE Strang des Alu Elements
<400> 445
   aattagtcgg gcgtggtggc 20
<210> 446
   <211> 20
   <212> DNA
   <213> SEQUENZIDENTISCHE Primersequenz, spezifisch für den Bisulfit-konvertierten METHYLIERTEN SENSE Strang des Alu Elements
<400> 446
   ttagtcgggc gtggtggcgc 20
<210> 447
   <211> 20
   <212> DNA
   <213> SEQUENZIDENTISCHE Primersequenz, spezifisch für den Bisulfit-konvertierten METHYLIERTEN SENSE Strang des Alu Elements
<400> 447
   agtcgggcgt ggtggcgcgc 20
<210> 448
   <211> 20
   <212> DNA
   <213> SEQUENZIDENTISCHE Primersequenz, spezifisch für den Bisulfit-konvertierten METHYLIERTEN SENSE Strang des Alu Elements
<400> 448
   gtttgtaatt ttagttattc 20
<210> 449
   <211> 20
   <212> DNA
   <213> SEQUENZIDENTISCHE Primersequenz, spezifisch für den Bisulfit-konvertierten METHYLIERTEN SENSE Strang des Alu Elements
<400> 449
   gaggttgagg taggagaatc 20
<210> 450
   <211> 20
   <212> DNA
   <213> SEQUENZIDENTISCHE Primersequenz, spezifisch für den Bisulfit-konvertierten METHYLIERTEN SENSE Strang des Alu Elements
<400> 450
   taggagaatc gtttgaattc 20
<210> 451
   <211> 20
   <212> DNA
   <213> SEQUENZIDENTISCHE Primersequenz, spezifisch für den Bisulfit-konvertierten METHYLIERTEN SENSE Strang des Alu Elements
<400> 451
   atcgtttgaa ttcgggaggc 20
<210> 452
   <211> 20
   <212> DNA
   <213> SEQUENZIDENTISCHE Primersequenz, spezifisch für den Bisulfit-konvertierten METHYLIERTEN SENSE Strang des Alu Elements
<400> 452
   ggttgtagtg agtcgagatc 20
<210> 453
   <211> 20
   <212> DNA
   <213> SEQUENZIDENTISCHE Primersequenz, spezifisch für den Bisulfit-konvertierten METHYLIERTEN SENSE Strang des Alu Elements
<400> 453
   ttgtagtgag tcgagatcgc 20
<210> 454
   <211> 20
   <212> DNA
   <213> SEQUENZIDENTISCHE Primersequenz, spezifisch für den Bisulfit-konvertierten METHYLIERTEN SENSE Strang des Alu Elements
<400> 454
   tattgtattt tagtttgggc 20
<210> 455
   <211> 20
   <212> DNA
   <213> SEQUENZIDENTISCHE Primersequenz, spezifisch für den Bisulfit-konvertierten METHYLIERTEN SENSE Strang des Alu Elements
<400> 455
   tttagtttgg gcgatagagc 20
<210> 456
   <211> 20
   <212> DNA
   <213> SEQUENZIDENTISCHE Primersequenz, spezifisch für den Bisulfit-konvertierten METHYLIERTEN SENSE Strang des Alu Elements
<400> 456
   gggcgataga gcgagatttc 20
<210> 457
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 457
   ggttgggtgt ggtggtttat 20
<210> 458
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 458
   ttttagtatt ttgggaggtt 20
<210> 459
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 459
   gtattttggg aggttgaggt 20
<210> 460
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 460
   tttgggaggt tgaggtgggt 20
<210> 461
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 461
   ttatttgagg ttaggagatt 20
<210> 462
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 462
   ggttaatatg gtgaaatttt 20
<210> 463
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 463
   taaaaatata aaaattagtt 20
<210> 464
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 464
   aatataaaaa ttagttgggt 20
<210> 465
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 465
   aattagttgg gtgtggtggt 20
<210> 466
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 466
   ttagttgggt gtggtggtgt 20
<210> 467
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 467
   agttgggtgt ggtggtgtgt 20
<210> 468
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 468
   gtttgtaatt ttagttattt 20

<210> 469
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 469
   gaggttgagg taggagaatt 20
<210> 470
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 470
   taggagaatt gtttgaattt 20
<210> 471
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 471
   attgtttgaa tttgggaggt 20
<210> 472
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 472
   ggttgtagtg agttgagatt 20
<210> 473
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 473
   ttgtagtgag ttgagattgt 20
<210> 474
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 474
   tattgtattt tagtttgggt 20
<210> 475
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 475
   tttagtttgg gtgatagagt 20
<210> 476
   <211> 20
   <212> DNA
   <213> SEQUENZIDENTISCHE Primersequenz, spezifisch für den Bisulfit-konvertierten DEMETHYLIERTEN SENSE Strang des Alu Elements
<400> 476
   gggtgataga gtgagatttt 20
<210> 477
   <211> 20
   <212> DNA
   <213> KOMPLEMENTÄRER PRIMER, spezifisch für den Bisulfit-konvertierten METHYLIERTEN SENSE Strang des Alu Elements
<400> 477
   ttttttaaaa cgaaatctcg 20
<210> 478
   <211> 20
   <212> DNA
   <213> KOMPLEMENTÄRER PRIMER, spezifisch für den Bisulfit-konvertierten METHYLIERTEN SENSE Strang des Alu Elements
<400> 478
   aacgaaatct cgctctatcg 20
<210> 479
   <211> 20
   <212> DNA
   <213> KOMPLEMENTÄRER PRIMER, spezifisch für den Bisulfit-konvertierten METHYLIERTEN SENSE Strang des Alu Elements
<400> 479
   caaactaaaa tacaataacg 20
<210> 480
   <211> 20
   <212> DNA
   <213> KOMPLEMENTÄRER PRIMER, spezifisch für den Bisulfit-konvertierten METHYLIERTEN SENSE Strang des Alu Elements
<400> 480
   aactaaaata caataacgcg 20
<210> 481
   <211> 20
   <212> DNA
   <213> KOMPLEMENTÄRER PRIMER, spezifisch für den Bisulfit-konvertierten METHYLIERTEN SENSE Strang des Alu Elements
<400> 481
   aatacaataa cgcgatctcg 20
<210> 482
   <211> 20
   <212> DNA
   <213> KOMPLEMENTÄRER PRIMER, spezifisch für den Bisulfit-konvertierten METHYLIERTEN SENSE Strang des Alu Elements
<400> 482
   tcgactcact acaacctccg 20
<210> 483
   <211> 20
   <212> DNA
   <213> KOMPLEMENTÄRER PRIMER, spezifisch für den Bisulfit-konvertierten METHYLIERTEN SENSE Strang des Alu Elements
<400> 483
   actacaacct ccgcctcccg 20
<210> 484
   <211> 20
   <212> DNA
   <213> KOMPLEMENTÄRER PRIMER, spezifisch für den Bisulfit-konvertierten METHYLIERTEN SENSE Strang des Alu Elements
<400> 484
   ccgcctcccg aattcaaacg 20
<210> 485
   <211> 20
   <212> DNA
   <213> KOMPLEMENTÄRER PRIMER, spezifisch für den Bisulfit-konvertierten METHYLIERTEN SENSE Strang des Alu Elements
<400> 485
   tctcctacct caacctcccg 20
<210> 486
   <211> 20
   <212> DNA
   <213> KOMPLEMENTÄRER PRIMER, spezifisch für den Bisulfit-konvertierten METHYLIERTEN SENSE Strang des Alu Elements
<400> 486
   aataactaaa attacaaacg 20
<210> 487
   <211> 20
   <212> DNA
   <213> KOMPLEMENTÄRER PRIMER, spezifisch für den Bisulfit-konvertierten METHYLIERTEN SENSE Strang des Alu Elements
<400> 487
   taactaaaat tacaaacgcg 20
<210> 488
   <211> 20
   <212> DNA
   <213> KOMPLEMENTÄRER PRIMER, spezifisch für den Bisulfit-konvertierten METHYLIERTEN SENSE Strang des Alu Elements
<400> 488
   actaaaatta caaacgcgcg 20
<210> 489
   <211> 20
   <212> DNA
   <213> KOMPLEMENTÄRER PRIMER, spezifisch für den Bisulfit-konvertierten METHYLIERTEN SENSE Strang des Alu Elements
<400> 489
   tacaaacgcg cgccaccacg 20
<210> 490
   <211> 20
   <212> DNA
   <213> KOMPLEMENTÄRER PRIMER, spezifisch für den Bisulfit-konvertierten METHYLIERTEN SENSE Strang des Alu Elements
<400> 490
   aacgcgcgcc accacgcccg 20
<210> 491
   <211> 20
   <212> DNA
   <213> KOMPLEMENTÄRER PRIMER, spezifisch für den Bisulfit-konvertierten METHYLIERTEN SENSE Strang des Alu Elements
<400> 491
   ttatattttt aataaaaacg 20
<210> 492
   <211> 20
   <212> DNA
   <213> KOMPLEMENTÄRER PRIMER, spezifisch für den Bisulfit-konvertierten METHYLIERTEN SENSE Strang des Alu Elements
<400> 492
   tattaaccaa aataatctcg 20
<210> 493
   <211> 20
   <212> DNA
   <213> KOMPLEMENTÄRER PRIMER, spezifisch für den Bisulfit-konvertierten METHYLIERTEN SENSE Strang des Alu Elements
<400> 493
   tcctaacctc aaataatccg 20
<210> 494
   <211> 20
   <212> DNA
   <213> KOMPLEMENTÄRER PRIMER, spezifisch für den Bisulfit-konvertierten METHYLIERTEN SENSE Strang des Alu Elements
<400> 494
   aacctcaaat aatccgcccg 20
<210> 495
   <211> 20
   <212> DNA
   <213> KOMPLEMENTÄRER PRIMER, spezifisch für den Bisulfit-konvertierten METHYLIERTEN SENSE Strang des Alu Elements
<400> 495
   caaataatcc gcccgcctcg 20
<210> 496
   <211> 20
   <212> DNA
   <213> KOMPLEMENTÄRER PRIMER, spezifisch für den Bisulfit-konvertierten METHYLIERTEN SENSE Strang des Alu Elements
<400> 496
   aaatactaaa attacaaacg 20
<210> 497
   <211> 20
   <212> DNA
   <213> KOMPLEMENTÄRER PRIMER, spezifisch für den Bisulfit-konvertierten METHYLIERTEN SENSE Strang des Alu Elements
<400> 497
   attacaaacg taaaccaccg 20
<210> 498
   <211> 20
   <212> DNA
   <213> KOMPLEMENTÄRER PRIMER, spezifisch für den Bisulfit-konvertierten METHYLIERTEN SENSE Strang des Alu Elements
<400> 498
   tacaaacgta aaccaccgcg 20
<210> 499
   <211> 20
   <212> DNA
   <213> KOMPLEMENTÄRER PRIMER, spezifisch für den Bisulfit-konvertierten METHYLIERTEN SENSE Strang des Alu Elements
<400> 499
   aacgtaaacc accgcgcccg 20
<210> 500
   <211> 20
   <212> DNA
   <213> KOMPLEMENTÄRER PRIMER, spezifisch für den Bisulfit-konvertierten DEMETHYLIERTEN SENSE Strang des Alu Elements
<400> 500
   ttttttaaaa caaaatctca 20
<210> 501
   <211> 20
   <212> DNA
   <213> KOMPLEMENTÄRER PRIMER, spezifisch für den Bisulfit-konvertierten DEMETHYLIERTEN SENSE Strang des Alu Elements
<400> 501
   aacaaaatct cactctatca 20
<210> 502
   <211> 20
   <212> DNA
   <213> KOMPLEMENTÄRER PRIMER, spezifisch für den Bisulfit-konvertierten DEMETHYLIERTEN SENSE Strang des Alu Elements
<400> 502
   caaactaaaa tacaataaca 20
<210> 503
   <211> 20
   <212> DNA
   <213> KOMPLEMENTÄRER PRIMER, spezifisch für den Bisulfit-konvertierten DEMETHYLIERTEN SENSE Strang des Alu Elements
<400> 503
   aactaaaata caataacaca 20
<210> 504
   <211> 20
   <212> DNA
   <213> KOMPLEMENTÄRER PRIMER, spezifisch für den Bisulfit-konvertierten DEMETHYLIERTEN SENSE Strang des Alu Elements
<400> 504
   aatacaataa cacaatctca 20
<210> 505
   <211> 20
   <212> DNA
   <213> KOMPLEMENTÄRER PRIMER, spezifisch für den Bisulfit-konvertierten DEMETHYLIERTEN SENSE Strang des Alu Elements
<400> 505
   tcaactcact acaacctcca 20
<210> 506
   <211> 20
   <212> DNA
   <213> KOMPLEMENTÄRER PRIMER, spezifisch für den Bisulfit-konvertierten DEMETHYLIERTEN SENSE Strang des Alu Elements
<400> 506
   actacaacct ccacctccca 20
<210> 507
   <211> 20
   <212> DNA
   <213> KOMPLEMENTÄRER PRIMER, spezifisch für den Bisulfit-konvertierten DEMETHYLIERTEN SENSE Strang des Alu Elements
<400> 507
   ccacctccca aattcaaaca 20
<210> 508
   <211> 20
   <212> DNA
   <213> KOMPLEMENTÄRER PRIMER, spezifisch für den Bisulfit-konvertierten DEMETHYLIERTEN SENSE Strang des Alu Elements
<400> 508
   tctcctacct caacctccca 20
<210> 509
   <211> 20
   <212> DNA
   <213> KOMPLEMENTÄRER PRIMER, spezifisch für den Bisulfit-konvertierten DEMETHYLIERTEN SENSE Strang des Alu Elements
<400> 509
   aataactaaa attacaaaca 20
<210> 510
   <211> 20
   <212> DNA
   <213> KOMPLEMENTÄRER PRIMER, spezifisch für den Bisulfit-konvertierten DEMETHYLIERTEN SENSE Strang des Alu Elements
<400> 510
   taactaaaat tacaaacaca 20
<210> 511
   <211> 20
   <212> DNA
   <213> KOMPLEMENTÄRER PRIMER, spezifisch für den Bisulfit-konvertierten DEMETHYLIERTEN SENSE Strang des Alu Elements
<400> 511
   actaaaatta caaacacaca 20
<210> 512
   <211> 20
   <212> DNA
   <213> KOMPLEMENTÄRER PRIMER, spezifisch für den Bisulfit-konvertierten DEMETHYLIERTEN SENSE Strang des Alu Elements
<400> 512
   tacaaacaca caccaccaca 20
<210> 513
   <211> 20
   <212> DNA
   <213> KOMPLEMENTÄRER PRIMER, spezifisch für den Bisulfit-konvertierten DEMETHYLIERTEN SENSE Strang des Alu Elements
<400> 513
   aacacacacc accacaccca 20
<210> 514
   <211> 20
   <212> DNA
   <213> KOMPLEMENTÄRER PRIMER, spezifisch für den Bisulfit-konvertierten DEMETHYLIERTEN SENSE Strang des Alu Elements
<400> 514
   ttatattttt aataaaaaca 20
<210> 515
   <211> 20
   <212> DNA
   <213> KOMPLEMENTÄRER PRIMER, spezifisch für den Bisulfit-konvertierten DEMETHYLIERTEN SENSE Strang des Alu Elements
<400> 515
   tattaaccaa aataatctca 20
<210> 516
   <211> 20
   <212> DNA
   <213> KOMPLEMENTÄRER PRIMER, spezifisch für den Bisulfit-konvertierten DEMETHYLIERTEN SENSE Strang des Alu Elements
<400> 516
   tcctaacctc aaataatcca 20
<210> 517
   <211> 20
   <212> DNA
   <213> KOMPLEMENTÄRER PRIMER, spezifisch für den Bisulfit-konvertierten DEMETHYLIERTEN SENSE Strang des Alu Elements
<400> 517
   aacctcaaat aatccaccca 20
<210> 518
   <211> 20
   <212> DNA
   <213> KOMPLEMENTÄRER PRIMER, spezifisch für den Bisulfit-konvertierten DEMETHYLIERTEN SENSE Strang des Alu Elements
<400> 518
   caaataatcc acccacctca 20
<210> 519
   <211> 20
   <212> DNA
   <213> KOMPLEMENTÄRER PRIMER, spezifisch für den Bisulfit-konvertierten DEMETHYLIERTEN SENSE Strang des Alu Elements
<400> 519
   aaatactaaa attacaaaca 20
<210> 520
   <211> 20
   <212> DNA
   <213> KOMPLEMENTÄRER PRIMER, spezifisch für den Bisulfit-konvertierten DEMETHYLIERTEN SENSE Strang des Alu Elements
<400> 520
   attacaaaca taaaccacca 20
<210> 521
   <211> 20
   <212> DNA
   <213> KOMPLEMENTÄRER PRIMER, spezifisch für den Bisulfit-konvertierten DEMETHYLIERTEN SENSE Strang des Alu Elements
<400> 521
   tacaaacata aaccaccaca 20
<210> 522
   <211> 20
   <212> DNA
   <213> KOMPLEMENTÄRER PRIMER, spezifisch für den Bisulfit-konvertierten DEMETHYLIERTEN SENSE Strang des Alu Elements
<400> 522
   aacgtaaacc accgcgccca 20
<210> 523
   <211> 20
   <212> DNA
   <213> SEQUENZIDENTISCHER Primer, spezifisch für den Bisulfit-konvertierten METHYLIERTEN ANTISENSE Strang des Alu Elements
<400> 523
   tttttttgag acggagtttc 20
<210> 524
   <211> 20
   <212> DNA
   <213> SEQUENZIDENTISCHER Primer, spezifisch für den Bisulfit-konvertierten METHYLIERTEN ANTISENSE Strang des Alu Elements
<400> 524
   agacggagtt tcgttttgtc 20
<210> 525
   <211> 20
   <212> DNA
   <213> SEQUENZIDENTISCHER Primer, spezifisch für den Bisulfit-konvertierten METHYLIERTEN ANTISENSE Strang des Alu Elements
<400> 525
   agacggagtt tcgttttgtc 20
<210> 526
   <211> 20
   <212> DNA
   <213> SEQUENZIDENTISCHER Primer, spezifisch für den Bisulfit-konvertierten METHYLIERTEN ANTISENSE Strang des Alu Elements
<400> 526
   ttaggttgga gtgtagtggc 20
<210> 527
   <211> 20
   <212> DNA
   <213> SEQUENZIDENTISCHER Primer, spezifisch für den Bisulfit-konvertierten METHYLIERTEN ANTISENSE Strang des Alu Elements
<400> 527
   aggttggagt gtagtggcgc 20
<210> 528
   <211> 20
   <212> DNA
   <213> SEQUENZIDENTISCHER Primer, spezifisch für den Bisulfit-konvertierten METHYLIERTEN ANTISENSE Strang des Alu Elements
<400> 528
   gagtgtagtg gcgcgatttc 20
<210> 529
   <211> 20
   <212> DNA
   <213> SEQUENZIDENTISCHER Primer, spezifisch für den Bisulfit-konvertierten METHYLIERTEN ANTISENSE Strang des Alu Elements
<400> 529
   ttcggtttat tgtaattttc 20
<210> 530
   <211> 20
   <212> DNA
   <213> SEQUENZIDENTISCHER Primer, spezifisch für den Bisulfit-konvertierten METHYLIERTEN ANTISENSE Strang des Alu Elements
<400> 530
   tattgtaatt ttcgtttttc 20
<210> 531
   <211> 20
   <212> DNA
   <213> SEQUENZIDENTISCHER Primer, spezifisch für den Bisulfit-konvertierten METHYLIERTEN ANTISENSE Strang des Alu Elements
<400> 531
   ttcgtttttc gggtttaagc 20
<210> 532
   <211> 20
   <212> DNA
   <213> SEQUENZIDENTISCHER Primer, spezifisch für den Bisulfit-konvertierten METHYLIERTEN ANTISENSE Strang des Alu Elements
<400> 532
   tttttttgtt ttagtttttc 20
<210> 533
   <211> 20
   <212> DNA
   <213> SEQUENZIDENTISCHER Primer, spezifisch für den Bisulfit-konvertierten METHYLIERTEN ANTISENSE Strang des Alu Elements
<400> 533
   gagtagttgg gattataggc 20
<210> 534
   <211> 20
   <212> DNA
   <213> SEQUENZIDENTISCHER Primer, spezifisch für den Bisulfit-konvertierten METHYLIERTEN ANTISENSE Strang des Alu Elements
<400> 534
   gtagttggga ttataggcgc 20
<210> 535
   <211> 20
   <212> DNA
   <213> SEQUENZIDENTISCHER Primer, spezifisch für den Bisulfit-konvertierten METHYLIERTEN ANTISENSE Strang des Alu Elements
<400> 535
   agttgggatt ataggcgcgc 20
<210> 536
   <211> 20
   <212> DNA
   <213> SEQUENZIDENTISCHER Primer, spezifisch für den Bisulfit-konvertierten METHYLIERTEN ANTISENSE Strang des Alu Elements
<400> 536
   ttataggcgc gcgttattac 20
<210> 537
   <211> 20
   <212> DNA
   <213> SEQUENZIDENTISCHER Primer, spezifisch für den Bisulfit-konvertierten METHYLIERTEN ANTISENSE Strang des Alu Elements
<400> 537
   aggcgcgcgt tattacgttc 20

<210> 538
   <211> 20
   <212> DNA
   <213> SEQUENZIDENTISCHER Primer, spezifisch für den Bisulfit-konvertierten METHYLIERTEN ANTISENSE Strang des Alu Elements
<400> 538
   tttgtatttt tagtagagac 20
<210> 539
   <211> 20
   <212> DNA
   <213> SEQUENZIDENTISCHER Primer, spezifisch für den Bisulfit-konvertierten METHYLIERTEN ANTISENSE Strang des Alu Elements
<400> 539
   atgttggtta ggatggtttc 20
<210> 540
   <211> 20
   <212> DNA
   <213> SEQUENZIDENTISCHER Primer, spezifisch für den Bisulfit-konvertierten METHYLIERTEN ANTISENSE Strang des Alu Elements
<400> 540
   tttttgattt taggtgattc 20
<210> 541
   <211> 20
   <212> DNA
   <213> SEQUENZIDENTISCHER Primer, spezifisch für den Bisulfit-konvertierten METHYLIERTEN ANTISENSE Strang des Alu Elements
<400> 541
   tgattttagg tgattcgttc 20
<210> 542
   <211> 20
   <212> DNA
   <213> SEQUENZIDENTISCHER Primer, spezifisch für den Bisulfit-konvertierten METHYLIERTEN ANTISENSE Strang des Alu Elements
<400> 542
   ttaggtgatt cgttcgtttc 20
<210> 543
   <211> 20
   <212> DNA
   <213> SEQUENZIDENTISCHER Primer, spezifisch für den Bisulfit-konvertierten METHYLIERTEN ANTISENSE Strang des Alu Elements
<400> 543
   aaagtgttgg gattataggc 20
<210> 544
   <211> 20
   <212> DNA
   <213> SEQUENZIDENTISCHER Primer, spezifisch für den Bisulfit-konvertierten METHYLIERTEN ANTISENSE Strang des Alu Elements
<400> 544
   gattataggc gtgagttatc 20
<210> 545
   <211> 20
   <212> DNA
   <213> SEQUENZIDENTISCHER Primer, spezifisch für den Bisulfit-konvertierten METHYLIERTEN ANTISENSE Strang des Alu Elements
<400> 545
   ttataggcgt gagttatcgc 20
<210> 546
   <211> 20
   <212> DNA
   <213> SEQUENZIDENTISCHER Primer, spezifisch für den Bisulfit-konvertierten METHYLIERTEN ANTISENSE Strang des Alu Elements
<400> 546
   aggcgtgagt tatcgcgttc 20
<210> 547
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 547
   tttttttgag actgagtttt 20
<210> 548
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 548
   agactgagtt tctttttgtt 20
<210> 549
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 549
   agactgagtt tctttttgtt 20
<210> 550
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 550
   ttaggttgga gtgtagtggt 20
<210> 551
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 551
   aggttggagt gtagtggctt 20
<210> 552
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 552
   gagtgtagtg gctctatttt 20
<210> 553
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 553
   ttctgtttat tgtaattttt 20
<210> 554
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 554
   tattgtaatt ttcttttttt 20
<210> 555
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 555
   ttcttttttc tggtttaagt 20
<210> 556
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 556
   tttttttgtt ttagtttttt 20
<210> 557
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 557
   gagtagttgg gattataggt 20
<210> 558
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 558
   gtagttggga ttataggctt 20
<210> 559
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 559
   agttgggatt ataggctctt 20
<210> 560
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 560
   ttataggctc tctttattat 20
<210> 561
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 561
   aggctctctt tattactttt 20
<210> 562
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 562
   tttgtatttt tagtagagat 20
<210> 563
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 563
   atgttggtta ggatggtttt 20
<210> 564
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 564
   tttttgattt taggtgattt 20
<210> 565
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 565
   tgattttagg tgattctttt 20
<210> 566
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 566
   ttaggtgatt ctttcttttt 20
<210> 567
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 567
   aaagtgttgg gattataggt 20
<210> 568
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 568
   gattataggc ttgagttatt 20
<210> 569
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 569
   ttataggctt gagttatctt 20
<210> 570
   <211> 20
   <212> DNA
   <213> SEQUENZIDENTISCHER Primer, spezifisch für den Bisulfit-konvertierten DEMETHYLIERTEN ANTISENSE Strang des Alu Elements
<400> 570
   aggcttgagt tatctctttt 20
<210> 571
   <211> 20
   <212> DNA
   <213> KOMPLEMENTÄRER PRIMER, spezifisch für den Bisulfit-konvertierten METHYLIERTEN ANTISENSE Strang des Alu Elements
<400> 571
   accgaacgcg ataactcacg 20
<210> 572
   <211> 20
   <212> DNA
   <213> KOMPLEMENTÄRER PRIMER, spezifisch für den Bisulfit-konvertierten METHYLIERTEN ANTISENSE Strang des Alu Elements
<400> 572
   cccaacactt taaaaaaccg 20
<210> 573
   <211> 20
   <212> DNA
   <213> KOMPLEMENTÄRER PRIMER, spezifisch für den Bisulfit-konvertierten METHYLIERTEN ANTISENSE Strang des Alu Elements
<400> 573
   cactttaaaa aaccgaaacg 20
<210> 574
   <211> 20
   <212> DNA
   <213> KOMPLEMENTÄRER PRIMER, spezifisch für den Bisulfit-konvertierten METHYLIERTEN ANTISENSE Strang des Alu Elements
<400> 574
   ttaaaaaacc gaaacgaacg 20
<210> 575
   <211> 20
   <212> DNA
   <213> KOMPLEMENTÄRER PRIMER, spezifisch für den Bisulfit-konvertierten METHYLIERTEN ANTISENSE Strang des Alu Elements
<400> 575
   cacctaaaat caaaaaatcg 20
<210> 576
   <211> 20
   <212> DNA
   <213> KOMPLEMENTÄRER PRIMER, spezifisch für den Bisulfit-konvertierten METHYLIERTEN ANTISENSE Strang des Alu Elements
<400> 576
   accaacataa taaaaccccg 20
<210> 577
   <211> 20
   <212> DNA
   <213> KOMPLEMENTÄRER PRIMER, spezifisch für den Bisulfit-konvertierten METHYLIERTEN ANTISENSE Strang des Alu Elements
<400> 577
   aaaaatacaa aaattaaccg 20
<210> 578
   <211> 20
   <212> DNA
   <213> KOMPLEMENTÄRER PRIMER, spezifisch für den Bisulfit-konvertierten METHYLIERTEN ANTISENSE Strang des Alu Elements
<400> 578
   atacaaaaat taaccgaacg 20
<210> 579
   <211> 20
   <212> DNA
   <213> KOMPLEMENTÄRER PRIMER, spezifisch für den Bisulfit-konvertierten METHYLIERTEN ANTISENSE Strang des Alu Elements
<400> 579
   attaaccgaa cgtaataacg 20
<210> 580
   <211> 20
   <212> DNA
   <213> KOMPLEMENTÄRER PRIMER, spezifisch für den Bisulfit-konvertierten METHYLIERTEN ANTISENSE Strang des Alu Elements
<400> 580
   taaccgaacg taataacgcg 20
<210> 581
   <211> 20
   <212> DNA
   <213> KOMPLEMENTÄRER PRIMER, spezifisch für den Bisulfit-konvertierten METHYLIERTEN ANTISENSE Strang des Alu Elements
<400> 581
   accgaacgta ataacgcgcg 20
<210> 582
   <211> 20
   <212> DNA
   <213> KOMPLEMENTÄRER PRIMER, spezifisch für den Bisulfit-konvertierten METHYLIERTEN ANTISENSE Strang des Alu Elements
<400> 582
   cctataatcc caactactcg 20
<210> 583
   <211> 20
   <212> DNA
   <213> KOMPLEMENTÄRER PRIMER, spezifisch für den Bisulfit-konvertierten METHYLIERTEN ANTISENSE Strang des Alu Elements
<400> 583
   gaactaaaac aaaaaaatcg 20
<210> 584
   <211> 20
   <212> DNA
   <213> KOMPLEMENTÄRER PRIMER, spezifisch für den Bisulfit-konvertierten METHYLIERTEN ANTISENSE Strang des Alu Elements
<400> 584
   aaaaaaatcg cttaaacccg 20
<210> 585
   <211> 20
   <212> DNA
   <213> KOMPLEMENTÄRER PRIMER, spezifisch für den Bisulfit-konvertierten METHYLIERTEN ANTISENSE Strang des Alu Elements
<400> 585
   tcgcttaaac ccgaaaaacg 20
<210> 586
   <211> 20
   <212> DNA
   <213> KOMPLEMENTÄRER PRIMER, spezifisch für den Bisulfit-konvertierten METHYLIERTEN ANTISENSE Strang des Alu Elements
<400> 586
   acgaaaatta caataaaccg 20
<210> 587
   <211> 20
   <212> DNA
   <213> KOMPLEMENTÄRER PRIMER, spezifisch für den Bisulfit-konvertierten METHYLIERTEN ANTISENSE Strang des Alu Elements
<400> 587
   attacaataa accgaaatcg 20
<210> 588
   <211> 20
   <212> DNA
   <213> KOMPLEMENTÄRER PRIMER, spezifisch für den Bisulfit-konvertierten METHYLIERTEN ANTISENSE Strang des Alu Elements
<400> 588
   tacaataaac cgaaatcgcg 20
<210> 589
   <211> 20
   <212> DNA
   <213> KOMPLEMENTÄRER PRIMER, spezifisch für den Bisulfit-konvertierten METHYLIERTEN ANTISENSE Strang des Alu Elements
<400> 589
   actacactcc aacctaaacg 20
<210> 590
   <211> 20
   <212> DNA
   <213> KOMPLEMENTÄRER PRIMER, spezifisch für den Bisulfit-konvertierten METHYLIERTEN ANTISENSE Strang des Alu Elements
<400> 590
   ccaacctaaa cgacaaaacg 20
<210> 591
   <211> 20
   <212> DNA
   <213> KOMPLEMENTÄRER PRIMER, spezifisch für den Bisulfit-konvertierten METHYLIERTEN ANTISENSE Strang des Alu Elements
<400> 591
   aacgacaaaa cgaaactccg 20
<210> 592
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 592
   accaaacaca ataactcaca 20
<210> 593
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 593
   cccaacactt taaaaaacca 20
<210> 594
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 594
   cactttaaaa aaccaaaaca 20
<210> 595
   <211> 20
   <212> DNA
   <213> KOMPLEMENTÄRER PRIMER, spezifisch für den Bisulfit-konvertierten DEMETHYLIERTEN ANTISENSE Strang des Alu Elements
   <400> 595
   ttaaaaaacc aaaacaaaca 20
<210> 596
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 596
   cacctaaaat caaaaaatca 20
<210> 597
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 597
   accaacataa taaaacccca 20
<210> 598
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 598
   aaaaatacaa aaattaacca 20
<210> 599
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 599
   atacaaaaat taaccaaaca 20
<210> 600
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 600
   attaaccaaa cataataaca 20
<210> 601
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 601
   taaccaaaca taataacaca 20
<210> 602
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 602
   accaaacata ataacacaca 20
<210> 603
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 603
   cctataatcc caactactca 20
<210> 604
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 604
   gaactaaaac aaaaaaatca 20
<210> 605
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 605
   aaaaaaatca cttaaaccca 20
<210> 606
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 606
   tcacttaaac ccaaaaaaca 20

<210> 607
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 607
   acaaaaatta caataaacca 20
<210> 608
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 608
   attacaataa accaaaatca 20
<210> 609
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 609
   tacaataaac caaaatcaca 20
<210> 610
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 610
   actacactcc aacctaaaca 20
<210> 611
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 611
   ccaacctaaa caacaaaaca 20
<210> 612
   <211> 20
   <212> DNA
   <213> KOMPLEMENTÄRER PRIMER, spezifisch für den Bisulfit-konvertierten DEMETHYLIERTEN ANTISENSE Strang des Alu Elements
<400> 612
   aacaacaaaa caaaactcca 20
<210> 613
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 613
   atgattttat ttttaatttc 20
<210> 614
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 614
   gggttaaatg gattaagggc 20
<210> 615
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 615
   tttagggata taaaaattgc 20
<210> 616
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 616
   agagtttgaa atatggtttc 20
<210> 617
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 617
   gggaagggaa agatttgatc 20
<210> 618
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 618
   atttgatcgt tttttagttc 20
<210> 619
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 619
   tttgggtaat ggaatgtttc 20
<210> 620
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 620
   aatgtttcgg tataaaattc 20
<210> 621
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 621
   ggtataaaat tcgattgtac 20
<210> 622
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 622
   atgtaaagat ttttgtttac 20
<210> 623
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 623
   ttttttagag aaatatttac 20
<210> 624
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 624
   ggatttttta tatgttgaac 20
<210> 625
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 625
   atgttgaacg ttggtttttc 20
<210> 626
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 626
   agttttttat tgtattttac 20
<210> 627
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 627
   ttttttattt ggtgtttaac 20
<210> 628
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 628
   tttggggtga aggtatattc 20
<210> 629
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 629
   gggtgaaggt atattcgagc 20
<210> 630
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 630
   gtggttattg aggataagtc 20
<210> 631
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 631
   ataagtcgat aagagatttc 20
<210> 632
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 632
   atatttatag ttagttttac 20
<210> 633
   <211> 19
   <212> DNA
   <213> SEQUENZIDENTISCHE Primersequenz, spezifisch für den Bisulfit-konvertierten METHYLIERTEN SENSE Strang des HERV-K Elements
<400> 633
   tacggtaagt ttgtgtatt 19
<210> 634
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 634
   tattttaaat agaagatagc 20
<210> 635
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 635
   aaaaaatttt agaaggaaac 20
<210> 636
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 636
   aaacggaaat tttatattgc 20
<210> 637
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 637
   tgcgaatatg tagtagagtc 20
<210> 638
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 638
   tcgttaatgg tttagttaac 20
<210> 639
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 639
   gttattagag tttaaattac 20
<210> 640
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 640
   ttttagtagg ttaggtgatc 20
<210> 641
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 641
   gtaatattat aattttaagc 20
<210> 642
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 642
   gtttattaat attggttatc 20
<210> 643
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 643
   attaatattg gttatcggtc 20
<210> 644
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 644
   atcggtcgaa ttttagtatc 20
<210> 645
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 645
   agggagttat atttttagtc 20
<210> 646
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 646
   aaggaaggag atattgaggc 20
<210> 647
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 647
   gcgtggtaat ttttagtaac 20
<210> 648
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 648
   tttttagtaa cgttagaatc 20
<210> 649
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 649
   atgtggattt ttgtgtttac 20
<210> 650
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 650
   gatttttgtg tttacggatc 20
<210> 651
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 651
   tttgtgttta cggatcgatc 20
<210> 652
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 652
   gatcgatcgt gggaggtttc 20
<210> 653
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 653
   tgattgaaat attaaaaggc 20
<210> 654
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 654
   ttataaattt tatattaatc 20
<210> 655
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 655
   taggtgtatt taatagtttc 20
<210> 656
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 656
   ttcgaagaga tagtgatatc 20
<210> 657
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 657
   gagatagtga tatcgagaac 20
<210> 658
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 658
   cgagaacggg ttatgatgac 20
<210> 659
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 659
   cgggttatga tgacgatggc 20
<210> 660
   <211> 20
   <212> DNA
   <213> SEQUENZIDENTISCHE Primersequenz, spezifisch für den Bisulfit-konvertierten METHYLIERTEN SENSE Strang des HERV-K Elements
<400> 660
   atgacgatgg cggttttgtc 20
<210> 661
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 661
   atgattttat ttttaatttt 20
<210> 662
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 662
   gggttaaatg gattaagggt 20
<210> 663
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 663
   tttagggata taaaaattgt 20
<210> 664
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 664
   agagtttgaa atatggtttt 20
<210> 665
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 665
   gggaagggaa agatttgatt 20
<210> 666
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 666
   atttgatctt tttttagttt 20
<210> 667
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 667
   tttgggtaat ggaatgtttt 20
<210> 668
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 668
   aatgtttctg tataaaattt 20
<210> 669
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 669
   ggtataaaat tctattgtat 20
<210> 670
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 670
   atgtaaagat ttttgtttat 20
<210> 671
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 671
   ttttttagag aaatatttat 20
<210> 672
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 672
   ggatttttta tatgttgaat 20
<210> 673
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 673
   atgttgaact ttggtttttt 20
<210> 674
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 674
   agttttttat tgtattttat 20
<210> 675
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 675
   ttttttattt ggtgtttaat 20

<210> 676
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 676
   tttggggtga aggtatattt 20
<210> 677
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 677
   gggtgaaggt atattctagt 20
<210> 678
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 678
   gtggttattg aggataagtt 20
<210> 679
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 679
   ataagtctat aagagatttt 20
<210> 680
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 680
   atatttatag ttagttttat 20
<210> 681
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 681
   tactgtaagt ttgtgtattt 20
<210> 682
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 682
   tattttaaat agaagatagt 20
<210> 683
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 683
   aaaaaatttt agaaggaaat 20
<210> 684
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 684
   aaactgaaat tttatattgt 20
<210> 685
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 685
   tgctaatatg tagtagagtt 20
<210> 686
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 686
   tctttaatgg tttagttaat 20
<210> 687
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 687
   gttattagag tttaaattat 20
<210> 688
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 688
   ttttagtagg ttaggtgatt 20
<210> 689
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 689
   gtaatattat aattttaagt 20
<210> 690
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 690
   gtttattaat attggttatt 20
<210> 691
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 691
   attaatattg gttatctgtt 20
<210> 692
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 692
   atctgtctaa ttttagtatt 20
<210> 693
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 693
   agggagttat atttttagtt 20
<210> 694
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 694
   aaggaaggag atattgaggt 20
<210> 695
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 695
   gcttggtaat ttttagtaat 20
<210> 696
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 696
   tttttagtaa ctttagaatt 20
<210> 697
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 697
   atgtggattt ttgtgtttat 20
<210> 698
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 698
   gatttttgtg tttactgatt 20
<210> 699
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 699
   tttgtgttta ctgatctatt 20
<210> 700
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 700
   gatctatctt gggaggtttt 20
<210> 701
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 701
   tgattgaaat attaaaaggt 20
<210> 702
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 702
   ttataaattt tatattaatt 20
<210> 703
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 703
   taggtgtatt taatagtttt 20
<210> 704
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 704
   ttctaagaga tagtgatatt 20
<210> 705
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 705
   gagatagtga tatctagaat 20
<210> 706
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 706
   ctagaactgg ttatgatgat 20
<210> 707
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 707
   ctggttatga tgactatggt 20
<210> 708
   <211> 20
   <212> DNA
   <213> SEQUENZIDENTISCHE Primersequenz, spezifisch für den Bisulfit-konvertierten DEMETHYLIERTEN SENSE Strang des HERV-K Elements
<400> 708
   atgactatgg ctgttttgtt 20
<210> 709
   <211> 20
   <212> DNA
   <213> komplementäre Primersequenze, spezifisch für den Bisulfit-konvertierten methylierten sense Strang des HERV-K Elements
<400> 709
   aaaaaaaata aaaaaacccg 20
<210> 710
   <211> 20
   <212> DNA
   <213> komplementäre Primersequenze, spezifisch für den Bisulfit-konvertierten methylierten sense Strang des HERV-K Elements
<400> 710
   aaaaacccga aaaaccaacg 20
<210> 711
   <211> 20
   <212> DNA
   <213> komplementäre Primersequenze, spezifisch für den Bisulfit-konvertierten methylierten sense Strang des HERV-K Elements
<400> 711
   tcaacatata aaaaatcccg 20
<210> 712
   <211> 20
   <212> DNA
   <213> komplementäre Primersequenze, spezifisch für den Bisulfit-konvertierten methylierten sense Strang des HERV-K Elements
<400> 712
   cattcataaa tatttctccg 20
<210> 713
   <211> 20
   <212> DNA
   <213> komplementäre Primersequenze, spezifisch für den Bisulfit-konvertierten methylierten sense Strang des HERV-K Elements
<400> 713
   aaaatcaaca aacaaacacg 20
<210> 714
   <211> 20
   <212> DNA
   <213> komplementäre Primersequenz, spezifisch für den Bisulfit-konvertierten methylierten sense Strang des HERV-K Elements
<400> 714
   aaacatctca atactttacg 20
<210> 715
   <211> 20
   <212> DNA
   <213> komplementäre Primersequenze, spezifisch für den Bisulfit-konvertierten methylierten sense Strang des HERV-K Elements
<400> 715
   ataaataaaa tattcaatcg 20
<210> 716
   <211> 20
   <212> DNA
   <213> komplementäre Primersequenze, spezifisch für den Bisulfit-konvertierten methylierten sense Strang des HERV-K Elements
<400> 716
   aaaatcccta cgacctttcg 20
<210> 717
   <211> 20
   <212> DNA
   <213> komplementäre Primersequenze, spezifisch für den Bisulfit-konvertierten methylierten sense Strang des HERV-K Elements
<400> 717
   atttccccct tttcttttcg 20
<210> 718
   <211> 20
   <212> DNA
   <213> komplementäre Primersequenze, spezifisch für den Bisulfit-konvertierten methylierten sense Strang des HERV-K Elements
<400> 718
   ttttcttttc gacaaaaccg 20
<210> 719
   <211> 20
   <212> DNA
   <213> komplementäre Primersequenze, spezifisch für den Bisulfit-konvertierten methylierten sense Strang des HERV-K Elements
<400> 719
   ttttcttttc gacaaaaccg 20
<210> 720
   <211> 20
   <212> DNA
   <213> komplementäre Primersequenze, spezifisch für den Bisulfit-konvertierten methylierten sense Strang des HERV-K Elements
<400> 720
   gccatcgtca tcataacccg 20
<210> 721
   <211> 20
   <212> DNA
   <213> komplementäre Primersequenze, spezifisch für den Bisulfit-konvertierten methylierten sense Strang des HERV-K Elements
<400> 721
   gtcatcataa cccgttctcg 20
<210> 722
   <211> 20
   <212> DNA
   <213> komplementäre Primersequenze, spezifisch für den Bisulfit-konvertierten methylierten sense Strang des HERV-K Elements
<400> 722
   tcgatatcac tatctcttcg 20
<210> 723
   <211> 20
   <212> DNA
   <213> komplementäre Primersequenze, spezifisch für den Bisulfit-konvertierten methylierten sense Strang des HERV-K Elements
<400> 723
   aacaaaacaa acacacaacg 20
<210> 724
   <211> 20
   <212> DNA
   <213> komplementäre Primersequenze, spezifisch für den Bisulfit-konvertierten methylierten sense Strang des HERV-K Elements
<400> 724
   taacaaaatt aaaatttacg 20
<210> 725
   <211> 20
   <212> DNA
   <213> komplementäre Primersequenze, spezifisch für den Bisulfit-konvertierten methylierten sense Strang des HERV-K Elements
<400> 725
   ttttaaatct atttaaaacg 20
<210> 726
   <211> 20
   <212> DNA
   <213> komplementäre Primersequenze, spezifisch für den Bisulfit-konvertierten methylierten sense Strang des HERV-K Elements
<400> 726
   caaaatataa ataaataacg 20
<210> 727
   <211> 20
   <212> DNA
   <213> komplementäre Primersequenze, spezifisch für den Bisulfit-konvertierten methylierten sense Strang des HERV-K Elements
<400> 727
   aaataacgaa acctcccacg 20
<210> 728
   <211> 20
   <212> DNA
   <213> komplementäre Primersequenze, spezifisch für den Bisulfit-konvertierten methylierten sense Strang des HERV-K Elements
<400> 728
   aacgaaacct cccacgatcg 20
<210> 729
   <211> 20
   <212> DNA
   <213> komplementäre Primersequenze, spezifisch für den Bisulfit-konvertierten methylierten sense Strang des HERV-K Elements
<400> 729
   aacctcccac gatcgatccg 20
<210> 730
   <211> 20
   <212> DNA
   <213> komplementäre Primersequenze, spezifisch für den Bisulfit-konvertierten methylierten sense strang des HERV-K Elements
<400> 730
   gcaactttat aaaaaaaccg 20
<210> 731
   <211> 20
   <212> DNA
   <213> komplementäre Primersequenz, spezifisch für den Bisulfit-konvertierten methylierten sense Strang des HERV-K Elements
<400> 731
   ttaaaataaa atttaaatcg 20
<210> 732
   <211> 20
   <212> DNA
   <213> komplementäre Primersequenz, spezifisch für den Bisulfit-konvertierten methylierten sense Strang des HERV-K Elements
<400> 732
   ataatataaa ataacttacg 20
<210> 733
   <211> 20
   <212> DNA
   <213> komplementäre Primersequenz, spezifisch für den Bisulfit-konvertierten methylierten sense Strang des HERV-K Elements
<400> 733
   ctaaactttc tattaaatcg 20
<210> 734
   <211> 20
   <212> DNA
   <213> komplementäre Primersequenz, spezifisch für den Bisulfit-konvertierten methylierten sense Strang des HERV-K Elements
<400> 734
   tttctattaa atcgctatcg 20
<210> 735
   <211> 20
   <212> DNA
   <213> komplementäre Primersequenz, spezifisch für den Bisulfit-konvertierten methylierten sense Strang des HERV-K Elements
<400> 735
   aacgatcata ataatttccg 20
<210> 736
   <211> 20
   <212> DNA
   <213> komplementäre Primersequenz, spezifisch für den Bisulfit-konvertierten methylierten sense Strang des HERV-K Elements
<400> 736
   cattattata acaaatctcg 20
<210> 737
   <211> 20
   <212> DNA
   <213> komplementäre Primersequenz, spezifisch für den Bisulfit-konvertierten methylierten sense Strang des HERV-K Elements
<400> 737
   cttctaaaac tatacctacg 20
<210> 738
   <211> 20
   <212> DNA
   <213> komplementäre Primersequenz, spezifisch für den Bisulfit-konvertierten methylierten sense Strang des HERV-K Elements
<400> 738
   ctaaaactat acctacgccg 20
<210> 739
   <211> 20
   <212> DNA
   <213> komplementäre Primersequenz, spezifisch für den Bisulfit-konvertierten methylierten sense Strang des HERV-K Elements
<400> 739
   acattatctc ctaataaacg 20
<210> 740
   <211> 20
   <212> DNA
   <213> komplementäre Primersequenz, spezifisch für den Bisulfit-konvertierten methylierten sense Strang des HERV-K Elements
<400> 740
   taactttcta aaaataaccg 20
<210> 741
   <211> 20
   <212> DNA
   <213> komplementäre Primersequenz, spezifisch für den Bisulfit-konvertierten methylierten sense Strang des HERV-K Elements
<400> 741
   ccgatactaa aattcgaccg 20
<210> 742
   <211> 20
   <212> DNA
   <213> komplementäre Primersequenz, spezifisch für den Bisulfit-konvertierten methylierten sense Strang des HERV-K Elements
<400> 742
   ccgatactaa aattcgaccg 20
<210> 743
   <211> 20
   <212> DNA
   <213> komplementäre Primersequenz, spezifisch für den Bisulfit-konvertierten methylierten sense Strang des HERV-K Elements
<400> 743
   cttattttct ctaacctacg 20
<210> 744
   <211> 20
   <212> DNA
   <213> komplementäre Primersequenz, spezifisch für den Bisulfit-konvertierten methylierten sense Strang des HERV-K Elements
<400> 744
   ttcgcaatat aaaatttccg 20

<210> 745
   <211> 20
   <212> DNA
   <213> komplementäre Primersequenz, spezifisch für den Bisulfit-konvertierten methylierten sense Strang des HERV-K Elements
<400> 745
   tatcacccta acttcttccg 20
<210> 746
   <211> 20
   <212> DNA
   <213> komplementäre Primersequenz, spezifisch für den Bisulfit-konvertierten methylierten sense Strang des HERV-K Elements
<400> 746
   ccgaatacac aaacttaccg 20
<210> 747
   <211> 20
   <212> DNA
   <213> komplementäre Primersequenz, spezifisch für den Bisulfit-konvertierten methylierten sense Strang des HERV-K Elements
<400> 747
   actaactata aatatactcg 20
<210> 748
   <211> 20
   <212> DNA
   <213> komplementäre Primersequenz, spezifisch für den Bisulfit-konvertierten methylierten sense Strang des HERV-K Elements
<400> 748
   acttatcctc aataaccacg 20
<210> 749
   <211> 20
   <212> DNA
   <213> komplementäre Primersequenz, spezifisch für den Bisulfit-konvertierten methylierten sense Strang des HERV-K Elements
<400> 749
   atcctcaata accacgctcg 20
<210> 750
   <211> 20
   <212> DNA
   <213> komplementäre Primersequenz, spezifisch für den Bisulfit-konvertierten methylierten sense Strang des HERV-K Elements
<400> 750
   acacctataa atatttctcg 20
<210> 751
   <211> 20
   <212> DNA
   <213> komplementäre Primersequenz, spezifisch für den Bisulfit-konvertierten methylierten sense Strang des HERV-K Elements
<400> 751
   aaaaacccga aaaaccaacg 20
<210> 752
   <211> 20
   <212> DNA
   <213> komplementäre Primersequenz, spezifisch für den Bisulfit-konvertierten methylierten sense Strang des HERV-K Elements
<400> 752
   aaaataaaca aacaaacacg 20
<210> 753
   <211> 20
   <212> DNA
   <213> komplementäre Primersequenz, spezifisch für den Bisulfit-konvertierten demethylierten sense Strang des HERV-K Elements
<400> 753
   aaaaaaaata aaaaaaccca 20
<210> 754
   <211> 20
   <212> DNA
   <213> komplementäre Primersequenz, spezifisch für den Bisulfit-konvertierten demethylierten sense Strang des HERV-K Elements
<400> 754
   aaaaacccaa aaaaccaaca 20
<210> 755
   <211> 20
   <212> DNA
   <213> komplementäre Primersequenz, spezifisch für den Bisulfit-konvertierten demethylierten sense Strang des HERV-K Elements
<400> 755
   tcaacatata aaaaatccca 20
<210> 756
   <211> 20
   <212> DNA
   <213> komplementäre Primersequenz, spezifisch für den Bisulfit-konvertierten demethylierten sense Strang des HERV-K Elements
<400> 756
   cattcataaa tatttctcca 20
<210> 757
   <211> 20
   <212> DNA
   <213> komplementäre Primersequenz, spezifisch für den Bisulfit-konvertierten demethylierten sense Strang des HERV-K Elements
<400> 757
   aaaatcaaca aacaaacaca 20
<210> 758
   <211> 20
   <212> DNA
   <213> komplementäre Primersequenz, spezifisch für den Bisulfit-konvertierten demethylierten sense Strang des HERV-K Elements
<400> 758
   aaacatctca atactttaca 20
<210> 759
   <211> 20
   <212> DNA
   <213> komplementäre Primersequenz, spezifisch für den Bisulfit-konvertierten demethylierten sense Strang des HERV-K Elements
<400> 759
   ataaataaaa tattcaatca 20
<210> 760
   <211> 20
   <212> DNA
   <213> komplementäre Primersequenz, spezifisch für den Bisulfit-konvertierten demethylierten sense Strang des HERV-K Elements
<400> 760
   aaaatcccta caacctttca 20
<210> 761
   <211> 20
   <212> DNA
   <213> komplementäre Primersequenz, spezifisch für den Bisulfit-konvertierten demethylierten sense Strang des HERV-K Elements
<400> 761
   atttccccct tttcttttca 20
<210> 762
   <211> 20
   <212> DNA
   <213> komplementäre Primersequenz, spezifisch für den Bisulfit-konvertierten demethylierten sense Strang des HERV-K Elements
<400> 762
   ttttcttttc aacaaaacca 20
<210> 763
   <211> 20
   <212> DNA
   <213> komplementäre Primersequenz, spezifisch für den Bisulfit-konvertierten demethylierten sense Strang des HERV-K Elements
<400> 763
   tttcaacaaa accaccatca 20
<210> 764
   <211> 20
   <212> DNA
   <213> komplementäre Primersequenz, spezifisch für den Bisulfit-konvertierten demethylierten sense Strang des HERV-K Elements
<400> 764
   gccatcatca tcataaccca 20
<210> 765
   <211> 20
   <212> DNA
   <213> komplementäre Primersequenz, spezifisch für den Bisulfit-konvertierten demethylierten sense Strang des HERV-K Elements
<400> 765
   gtcatcataa cccattctca 20
<210> 766
   <211> 20
   <212> DNA
   <213> komplementäre Primersequenz, spezifisch für den Bisulfit-konvertierten demethylierten sense Strang des HERV-K Elements
<400> 766
   tcaatatcac tatctcttca 20
<210> 767
   <211> 20
   <212> DNA
   <213> komplementäre Primersequenz, spezifisch für den Bisulfit-konvertierten demethylierten sense Strang des HERV-K Elements
<400> 767
   aacaaaacaa acacacaaca 20
<210> 768
   <211> 20
   <212> DNA
   <213> komplementäre Primersequenz, spezifisch für den Bisulfit-konvertierten demethylierten sense Strang des HERV-K Elements
<400> 768
   taacaaaatt aaaatttaca 20
<210> 769
   <211> 20
   <212> DNA
   <213> komplementäre Primersequenz, spezifisch für den Bisulfit-konvertierten demethylierten sense Strang des HERV-K Elements
<400> 769
   ttttaaatct atttaaaaca 20
<210> 770
   <211> 20
   <212> DNA
   <213> komplementäre Primersequenz, spezifisch für den Bisulfit-konvertierten demethylierten sense Strang des HERV-K Elements
<400> 770
   caaaatataa ataaataaca 20
<210> 771
   <211> 20
   <212> DNA
   <213> komplementäre Primersequenz, spezifisch für den Bisulfit-konvertierten demethylierten sense Strang des HERV-K Elements
<400> 771
   aaataacaaa acctcccaca 20
<210> 772
   <211> 20
   <212> DNA
   <213> komplementäre Primersequenz, spezifisch für den Bisulfit-konvertierten demethylierten sense Strang des HERV-K Elements
<400> 772
   aacaaaacct cccacaatca 20
<210> 773
   <211> 20
   <212> DNA
   <213> komplementäre Primersequenz, spezifisch für den Bisulfit-konvertierten demethylierten sense Strang des HERV-K Elements
<400> 773
   aacctcccac aatcaatcca 20
<210> 774
   <211> 20
   <212> DNA
   <213> komplementäre Primersequenz, spezifisch für den Bisulfit-konvertierten demethylierten sense Strang des HERV-K Elements
<400> 774
   gcaactttat aaaaaaacca 20
<210> 775
   <211> 20
   <212> DNA
   <213> komplementäre Primersequenz, spezifisch für den Bisulfit-konvertierten demethylierten sense Strang des HERV-K Elements
<400> 775
   ttaaaataaa atttaaatca 20
<210> 776
   <211> 20
   <212> DNA
   <213> komplementäre Primersequenz, spezifisch für den Bisulfit-konvertierten demethylierten sense Strang des HERV-K Elements
<400> 776
   ataatataaa ataacttaca 20
<210> 777
   <211> 20
   <212> DNA
   <213> komplementäre Primersequenz, spezifisch für den Bisulfit-konvertierten demethylierten sense Strang des HERV-K Elements
<400> 777
   ctaaactttc tattaaatca 20
<210> 778
   <211> 20
   <212> DNA
   <213> komplementäre Primersequenz, spezifisch für den Bisulfit-konvertierten demethylierten sense Strang des HERV-K Elements
<400> 778
   tttctattaa atcactatca 20
<210> 779
   <211> 20
   <212> DNA
   <213> komplementäre Primersequenz, spezifisch für den Bisulfit-konvertierten demethylierten sense Strang des HERV-K Elements
<400> 779
   aacaatcata ataatttcca 20
<210> 780
   <211> 20
   <212> DNA
   <213> komplementäre Primersequenz, spezifisch für den Bisulfit-konvertierten demethylierten sense Strang des HERV-K Elements
<400> 780
   cttctaaaac tatacctaca 20
<210> 781
   <211> 20
   <212> DNA
   <213> komplementäre Primersequenz, spezifisch für den Bisulfit-konvertierten demethylierten sense Strang des HERV-K Elements
<400> 781
   cttctaaaac tatacctaca 20
<210> 782
   <211> 20
   <212> DNA
   <213> komplementäre Primersequenz, spezifisch für den Bisulfit-konvertierten demethylierten sense Strang des HERV-K Elements
<400> 782
   ctaaaactat acctacacca 20
<210> 783
   <211> 20
   <212> DNA
   <213> komplementäre Primersequenz, spezifisch für den Bisulfit-konvertierten demethylierten sense Strang des HERV-K Elements
<400> 783
   acattatctc ctaataaaca 20
<210> 784
   <211> 20
   <212> DNA
   <213> komplementäre Primersequenz, spezifisch für den Bisulfit-konvertierten demethylierten sense Strang des HERV-K Elements
<400> 784
   taactttcta aaaataacca 20
<210> 785
   <211> 20
   <212> DNA
   <213> komplementäre Primersequenz, spezifisch für den Bisulfit-konvertierten demethylierten sense Strang des HERV-K Elements
<400> 785
   ataaccaata ctaaaattca 20
<210> 786
   <211> 20
   <212> DNA
   <213> komplementäre Primersequenz, spezifisch für den Bisulfit-konvertierten demethylierten sense Strang des HERV-K Elements
<400> 786
   ccaatactaa aattcaacca 20
<210> 787
   <211> 20
   <212> DNA
   <213> komplementäre Primersequenz, spezifisch für den Bisulfit-konvertierten demethylierten sense Strang des HERV-K Elements
<400> 787
   cttattttct ctaacctaca 20
<210> 788
   <211> 20
   <212> DNA
   <213> komplementäre Primersequenz, spezifisch für den Bisulfit-konvertierten demethylierten sense Strang des HERV-K Elements
<400> 788
   ttcacaatat aaaatttcca 20
<210> 789
   <211> 20
   <212> DNA
   <213> komplementäre Primersequenz, spezifisch für den Bisulfit-konvertierten demethylierten sense Strang des HERV-K Elements
<400> 789
   tatcacccta acttcttcca 20
<210> 790
   <211> 20
   <212> DNA
   <213> komplementäre Primersequenz, spezifisch für den Bisulfit-konvertierten demethylierten sense Strang des HERV-K Elements
<400> 790
   ccaaatacac aaacttacca 20
<210> 791
   <211> 20
   <212> DNA
   <213> komplementäre Primersequenz, spezifisch für den Bisulfit-konvertierten demethylierten sense Strang des HERV-K Elements
<400> 791
   actaactata aatatactca 20
<210> 792
   <211> 20
   <212> DNA
   <213> komplementäre Primersequenz, spezifisch für den Bisulfit-konvertierten demethylierten sense Strang des HERV-K Elements
<400> 792
   acttatcctc aataaccaca 20
<210> 793
   <211> 20
   <212> DNA
   <213> komplementäre Primersequenz, spezifisch für den Bisulfit-konvertierten demethylierten sense Strang des HERV-K Elements
<400> 793
   atcctcaata accacactca 20
<210> 794
   <211> 20
   <212> DNA
   <213> komplementäre Primersequenz, spezifisch für den Bisulfit-konvertierten demethylierten sense Strang des HERV-K Elements
<400> 794
   acacctataa atatttctca 20
<210> 795
   <211> 20
   <212> DNA
   <213> komplementäre Primersequenz, spezifisch für den Bisulfit-konvertierten demethylierten sense Strang des HERV-K Elements
<400> 795
   aaaaacccaa aaaaccaaca 20
<210> 796
   <211> 20
   <212> DNA
   <213> komplementäre Primersequenz, spezifisch für den Bisulfit-konvertierten demethylierten sense Strang des HERV-K Elements
<400> 796
   aaaataaaca aacaaacaca 20
<210> 797
   <211> 20
   <212> DNA
   <213> Sequenzidentische Primersequenz, spezifisch für den Bisulfit-konvertierten methylierten antisense Strang des HERV-K Elements
<400> 797
   agaaagaaat aagggggttc 20
<210> 798
   <211> 20
   <212> DNA
   <213> Sequenzidentische Primersequenz, spezifisch für den Bisulfit-konvertierten methylierten antisense Strang des HERV-K Elements
<400> 798
   agggggttcg gggaattagc 20
<210> 799
   <211> 20
   <212> DNA
   <213> Sequenzidentische Primersequenz, spezifisch für den Bisulfit-konvertierten methylierten antisense Strang des HERV-K Elements
<400> 799
   tttagtatat ggaggatttc 20
<210> 800
   <211> 20
   <212> DNA
   <213> Sequenzidentische Primersequenz, spezifisch für den Bisulfit-konvertierten methylierten antisense Strang des HERV-K Elements
<400> 800
   ttagtattta ttgattattc 20
<210> 801
   <211> 20
   <212> DNA
   <213> Sequenzidentische Primersequenz, spezifisch für den Bisulfit-konvertierten methylierten antisense Strang des HERV-K Elements
<400> 801
   ttattcgtgg gtgttttttc 20
<210> 802
   <211> 20
   <212> DNA
   <213> Sequenzidentische Primersequenz, spezifisch für den Bisulfit-konvertierten methylierten antisense Strang des HERV-K Elements
<400> 802
   gagggttagt agataaatac 20
<210> 803
   <211> 20
   <212> DNA
   <213> Sequenzidentische Primersequenz, spezifisch für den Bisulfit-konvertierten methylierten antisense Strang des HERV-K Elements
<400> 803
   taaatatttt aatgttttac 20
<210> 804
   <211> 20
   <212> DNA
   <213> Sequenzidentische Primersequenz, spezifisch für den Bisulfit-konvertierten methylierten antisense Strang des HERV-K Elements
<400> 804
   agtagatgga atgtttaatc 20
<210> 805
   <211> 20
   <212> DNA
   <213> Sequenzidentische Primersequenz, spezifisch für den Bisulfit-konvertierten methylierten antisense Strang des HERV-K Elements
<400> 805
   ttttagtata gattttttac 20
<210> 806
   <211> 20
   <212> DNA
   <213> Sequenzidentische Primersequenz, spezifisch für den Bisulfit-konvertierten methylierten antisense Strang des HERV-K Elements
<400> 806
   atagattttt tacgggtgtc 20
<210> 807
   <211> 20
   <212> DNA
   <213> Sequenzidentische Primersequenz, spezifisch für den Bisulfit-konvertierten methylierten antisense Strang des HERV-K Elements
<400> 807
   ttaggttttt ttttttttac 20
<210> 808
   <211> 20
   <212> DNA
   <213> Sequenzidentische Primersequenz, spezifisch für den Bisulfit-konvertierten methylierten antisense Strang des HERV-K Elements
<400> 808
   ttttaggtag aggtttttgc 20
<210> 809
   <211> 20
   <212> DNA
   <213> Sequenzidentische Primersequenz, spezifisch für den Bisulfit-konvertierten methylierten antisense Strang des HERV-K Elements
<400> 809
   agaggttttt gcggtttttc 20
<210> 810
   <211> 20
   <212> DNA
   <213> Sequenzidentische Primersequenz, spezifisch für den Bisulfit-konvertierten methylierten antisense Strang des HERV-K Elements
<400> 810
   gtatatgttt tagagagtac 20
<210> 811
   <211> 20
   <212> DNA
   <213> Sequenzidentische Primersequenz, spezifisch für den Bisulfit-konvertierten methylierten antisense Strang des HERV-K Elements
<400> 811
   tatttttttt tttttttttc 20
<210> 812
   <211> 20
   <212> DNA
   <213> Sequenzidentische Primersequenz, spezifisch für den Bisulfit-konvertierten methylierten antisense Strang des HERV-K Elements
<400> 812
   tttttttttt cgataaaatc 20
<210> 813
   <211> 20
   <212> DNA
   <213> Sequenzidentische Primersequenz, spezifisch für den Bisulfit-konvertierten methylierten antisense Strang des HERV-K Elements
<400> 813
   ttttcgataa aatcgttatc 20

<210> 814
   <211> 20
   <212> DNA
   <213> Sequenzidentische Primersequenz, spezifisch für den Bisulfit-konvertierten methylierten antisense Strang des HERV-K Elements
<400> 814
   cgttatcgtt attatggttc 20
<210> 815
   <211> 20
   <212> DNA
   <213> Sequenzidentische Primersequenz, spezifisch für den Bisulfit-konvertierten methylierten antisense Strang des HERV-K Elements
<400> 815
   cgttattatg gttcgttttc 20
<210> 816
   <211> 20
   <212> DNA
   <213> Sequenzidentische Primersequenz, spezifisch für den Bisulfit-konvertierten methylierten antisense Strang des HERV-K Elements
<400> 816
   ttcgatgtta ttgttttttc 20
<210> 817
   <211> 20
   <212> DNA
   <213> Sequenzidentische Primersequenz, spezifisch für den Bisulfit-konvertierten methylierten antisense Strang des HERV-K Elements
<400> 817
   agataaaata ggtatataac 20
<210> 818
   <211> 20
   <212> DNA
   <213> Sequenzidentische Primersequenz, spezifisch für den Bisulfit-konvertierten methylierten antisense Strang des HERV-K Elements
<400> 818
   gtgatagggt taagatttgc 20
<210> 819
   <211> 20
   <212> DNA
   <213> Sequenzidentische Primersequenz, spezifisch für den Bisulfit-konvertierten methylierten antisense Strang des HERV-K Elements
<400> 819
   taatttttgt tatagtagtc 20
<210> 820
   <211> 20
   <212> DNA
   <213> Sequenzidentische Primersequenz, spezifisch für den Bisulfit-konvertierten methylierten antisense Strang des HERV-K Elements
<400> 820
   tttttggatt tatttaaaac 20
<210> 821
   <211> 20
   <212> DNA
   <213> Sequenzidentische Primersequenz, spezifisch für den Bisulfit-konvertierten methylierten antisense Strang des HERV-K Elements
<400> 821
   ttaaaatatg gatggatggc **2**0
<210> 822
   <211> 20
   <212> DNA
   <213> Sequenzidentische Primersequenz, spezifisch für den Bisulfit-konvertierten methylierten antisense Strang des HERV-K Elements
<400> 822
   tggatggcga ggttttttac **2**0
<210> 823
   <211> 20
   <212> DNA
   <213> Sequenzidentische Primersequenz, spezifisch für den Bisulfit-konvertierten methylierten antisense Strang des HERV-K Elements
<400> 823
   tggcgaggtt ttttacggtc 20
<210> 824
   <211> 20
   <212> DNA
   <213> Sequenzidentische Primersequenz, spezifisch für den Bisulfit-konvertierten methylierten antisense Strang des HERV-K Elements
<400> 824
   aggtttttta cggtcggttc 20
<210> 825
   <211> 20
   <212> DNA
   <213> Sequenzidentische Primersequenz, spezifisch für den Bisulfit-konvertierten methylierten antisense Strang des HERV-K Elements
<400> 825
   aggtttttta cggtcggttc 20
<210> 826
   <211> 20
   <212> DNA
   <213> Sequenzidentische Primersequenz, spezifisch für den Bisulfit-konvertierten methylierten antisense Strang des HERV-K Elements
<400> 826
   cgtaattttg taaaggaatc 20
<210> 827
   <211> 20
   <212> DNA
   <213> Sequenzidentische Primersequenz, spezifisch für den Bisulfit-konvertierten methylierten antisense Strang des HERV-K Elements
<400> 827
   gttagaatgg aatttaggtc 20
<210> 828
   <211> 20
   <212> DNA
   <213> Sequenzidentische Primersequenz, spezifisch für den Bisulfit-konvertierten methylierten antisense Strang des HERV-K Elements
<400> 828
   gatagtataa aatggtttac 20
<210> 829
   <211> 20
   <212> DNA
   <213> Sequenzidentische Primersequenz, spezifisch für den Bisulfit-konvertierten methylierten antisense Strang des HERV-K Elements
<400> 829
   ttatttgtgt atttggatac 20
<210> 830
   <211> 20
   <212> DNA
   <213> Sequenzidentische Primersequenz, spezifisch für den Bisulfit-konvertierten methylierten antisense Strang des HERV-K Elements
<400> 830
   attgtggtag aattgatttc 20
<210> 831
   <211> 20
   <212> DNA
   <213> Sequenzidentische Primersequenz, spezifisch für den Bisulfit-konvertierten methylierten antisense Strang des HERV-K Elements
<400> 831
   gtttaattta taatagtttc 20
<210> 832
   <211> 20
   <212> DNA
   <213> Sequenzidentische Primersequenz, spezifisch für den Bisulfit-konvertierten methylierten antisense Strang des HERV-K Elements
<400> 832
   gttttgtaaa taatttattc 20
<210> 833
   <211> 20
   <212> DNA
   <213> Sequenzidentische Primersequenz, spezifisch für den Bisulfit-konvertierten methylierten antisense Strang des HERV-K Elements
<400> 833
   cgtggtttga gtgatatttc 20
<210> 834
   <211> 20
   <212> DNA
   <213> Sequenzidentische Primersequenz, spezifisch für den Bisulfit-konvertierten methylierten antisense Strang des HERV-K Elements
<400> 834
   tttaggtttg gtagggtagc 20
<210> 835
   <211> 20
   <212> DNA
   <213> Sequenzidentische Primersequenz, spezifisch für den Bisulfit-konvertierten methylierten antisense Strang des HERV-K Elements
<400> 835
   tgattggtgt tattattttc 20
<210> 836
   <211> 20
   <212> DNA
   <213> Sequenzidentische Primersequenz, spezifisch für den Bisulfit-konvertierten methylierten antisense Strang des HERV-K Elements
<400> 836
   gttattattt tcgtggaggc 20
<210> 837
   <211> 20
   <212> DNA
   <213> Sequenzidentische Primersequenz, spezifisch für den Bisulfit-konvertierten methylierten antisense Strang des HERV-K Elements
<400> 837
   gtattatata tgtagaattc 20
<210> 838
   <211> 20
   <212> DNA
   <213> Sequenzidentische Primersequenz, spezifisch für den Bisulfit-konvertierten methylierten antisense Strang des HERV-K Elements
<400> 838
   agtatttttt aaaggtttac 20
<210> 839
   <211> 20
   <212> DNA
   <213> Sequenzidentische Primersequenz, spezifisch für den Bisulfit-konvertierten methylierten antisense Strang des HERV-K Elements
<400> 839
   aggaatgttt agagttggtc 20
<210> 840
   <211> 20
   <212> DNA
   <213> Sequenzidentische Primersequenz, spezifisch für den Bisulfit-konvertierten methylierten antisense Strang des HERV-K Elements
<400> 840
   atggggttat ataatgtagc 20
<210> 841
   <211> 20
   <212> DNA
   <213> Sequenzidentische Primersequenz, spezifisch für den Bisulfit-konvertierten methylierten antisense Strang des HERV-K Elements
<400> 841
   ttattgttgt aataaatttc 20
<210> 842
   <211> 20
   <212> DNA
   <213> Sequenzidentische Primersequenz, spezifisch für den Bisulfit-konvertierten methylierten antisense Strang des HERV-K Elements
<400> 842
   atttttgagg ttgtgtttac 20
<210> 843
   <211> 20
   <212> DNA
   <213> Sequenzidentische Primersequenz, spezifisch für den Bisulfit-konvertierten methylierten antisense Strang des HERV-K Elements
<400> 843
   tttgaggttg tgtttacgtc 20
<210> 844
   <211> 20
   <212> DNA
   <213> Sequenzidentische Primersequenz, spezifisch für den Bisulfit-konvertierten methylierten antisense Strang des HERV-K Elements
<400> 844
   ttataagtat agttttatgc 20
<210> 845
   <211> 20
   <212> DNA
   <213> Sequenzidentische Primersequenz, spezifisch für den Bisulfit-konvertierten methylierten antisense Strang des HERV-K Elements
<400> 845
   tttttttttt aggtggtatc 20
<210> 846
   <211> 20
   <212> DNA
   <213> Sequenzidentische Primersequenz, spezifisch für den Bisulfit-konvertierten methylierten antisense Strang des HERV-K Elements
<400> 846
   taggtggtat cggttttaac 20
<210> 847
   <211> 20
   <212> DNA
   <213> Sequenzidentische Primersequenz, spezifisch für den Bisulfit-konvertierten methylierten antisense Strang des HERV-K Elements
<400> 847
   ttgatttttt gggggtggtc 20
<210> 848
   <211> 20
   <212> DNA
   <213> Sequenzidentische Primersequenz, spezifisch für den Bisulfit-konvertierten methylierten antisense Strang des HERV-K Elements
<400> 848
   ggtggtcgat attgaagttc 20
<210> 849
   <211> 20
   <212> DNA
   <213> Sequenzidentische Primersequenz, spezifisch für den Bisulfit-konvertierten methylierten antisense Strang des HERV-K Elements
<400> 849
   gtcgatattg aagttcggtc 20
<210> 850
   <211> 20
   <212> DNA
   <213> Sequenzidentische Primersequenz, spezifisch für den Bisulfit-konvertierten methylierten antisense Strang des HERV-K Elements
<400> 850
   ttttattttt tttaatttgc 20
<210> 851
   <211> 20
   <212> DNA
   <213> Sequenzidentische Primersequenz, spezifisch für den Bisulfit-konvertierten methylierten antisense Strang des HERV-K Elements
<400> 851
   gtttgaggtt gtaatgttac 20
<210> 852
   <211> 20
   <212> DNA
   <213> Sequenzidentische Primersequenz, spezifisch für den Bisulfit-konvertierten methylierten antisense Strang des HERV-K Elements
<400> 852
   gcgttgattg agttattaac 20
<210> 853
   <211> 20
   <212> DNA
   <213> Sequenzidentische Primersequenz, spezifisch für den Bisulfit-konvertierten methylierten antisense Strang des HERV-K Elements
<400> 853
   ttgttatttt agtttttttc 20
<210> 854
   <211> 20
   <212> DNA
   <213> Sequenzidentische Primersequenz, spezifisch für den Bisulfit-konvertierten methylierten antisense Strang des HERV-K Elements
<400> 854
   ttcgagtgta taagtttatc 20
<210> 855
   <211> 20
   <212> DNA
   <213> Sequenzidentische Primersequenz, spezifisch für den Bisulfit-konvertierten methylierten antisense Strang des HERV-K Elements
<400> 855
   gatttgtttt taatgattac 20
<210> 856
   <211> 20
   <212> DNA
   <213> Sequenzidentische Primersequenz, spezifisch für den Bisulfit-konvertierten methylierten antisense Strang des HERV-K Elements
<400> 856
   tgtttttaat gattacgttc 20
<210> 857
   <211> 20
   <212> DNA
   <213> Sequenzidentische Primersequenz, spezifisch für den Bisulfit-konvertierten methylierten antisense Strang des HERV-K Elements
<400> 857
   tgtttttaat gattacgttc 20
<210> 858
   <211> 20
   <212> DNA
   <213> Sequenzidentische Primersequenz, spezifisch für den Bisulfit-konvertierten methylierten antisense Strang des HERV-K Elements
<400> 858
   agaaagaaat aagggggttc 20
<210> 859
   <211> 20
   <212> DNA
   <213> Sequenzidentische Primersequenz, spezifisch für den Bisulfit-konvertierten methylierten antisense Strang des HERV-K Elements
<400> 859
   agggggttcg gggaattaac 20
<210> 860
   <211> 20
   <212> DNA
   <213> Sequenzidentische Primersequenz, spezifisch für den Bisulfit-konvertierten demethylierten antisense Strang des HERV-K Elements
<400> 860
   agaaagaaat aagggggttt 20
<210> 861
   <211> 20
   <212> DNA
   <213> Sequenzidentische Primersequenz, spezifisch für den Bisulfit-konvertierten demethylierten antisense Strang des HERV-K Elements
<400> 861
   agggggttct gggaattagt 20
<210> 862
   <211> 20
   <212> DNA
   <213> Sequenzidentische Primersequenz, spezifisch für den Bisulfit-konvertierten demethylierten antisense Strang des HERV-K Elements
<400> 862
   tttagtatat ggaggatttt 20
<210> 863
   <211> 20
   <212> DNA
   <213> Sequenzidentische Primersequenz, spezifisch für den Bisulfit-konvertierten demethylierten antisense Strang des HERV-K Elements
<400> 863
   ttagtattta ttgattattt 20
<210> 864
   <211> 20
   <212> DNA
   <213> Sequenzidentische Primersequenz, spezifisch für den Bisulfit-konvertierten demethylierten antisense Strang des HERV-K Elements
<400> 864
   ttattcttgg gtgttttttt 20
<210> 865
   <211> 20
   <212> DNA
   <213> Sequenzidentische Primersequenz, spezifisch für den Bisulfit-konvertierten demethylierten antisense Strang des HERV-K Elements
<400> 865
   gagggttagt agataaatat 20
<210> 866
   <211> 20
   <212> DNA
   <213> Sequenzidentische Primersequenz, spezifisch für den Bisulfit-konvertierten demethylierten antisense Strang des HERV-K Elements
<400> 866
   taaatatttt aatgttttat 20
<210> 867
   <211> 20
   <212> DNA
   <213> Sequenzidentische Primersequenz, spezifisch für den Bisulfit-konvertierten demethylierten antisense Strang des HERV-K Elements
<400> 867
   agtagatgga atgtttaatt 20
<210> 868
   <211> 20
   <212> DNA
   <213> Sequenzidentische Primersequenz, spezifisch für den Bisulfit-konvertierten demethylierten antisense Strang des HERV-K Elements
<400> 868
   ttttagtata gattttttat 20
<210> 869
   <211> 20
   <212> DNA
   <213> Sequenzidentische Primersequenz, spezifisch für den Bisulfit-konvertierten demethylierten antisense Strang des HERV-K Elements
<400> 869
   atagattttt tactggtgtt 20
<210> 870
   <211> 20
   <212> DNA
   <213> Sequenzidentische Primersequenz, spezifisch für den Bisulfit-konvertierten demethylierten antisense Strang des HERV-K Elements
<400> 870
   ttaggttttt ttttttttat 20
<210> 871
   <211> 20
   <212> DNA
   <213> Sequenzidentische Primersequenz, spezifisch für den Bisulfit-konvertierten demethylierten antisense Strang des HERV-K Elements
<400> 871
   ttttaggtag aggtttttgt 20
<210> 872
   <211> 20
   <212> DNA
   <213> Sequenzidentische Primersequenz, spezifisch für den Bisulfit-konvertierten demethylierten antisense Strang des HERV-K Elements
<400> 872
   agaggttttt gctgtttttt 20
<210> 873
   <211> 20
   <212> DNA
   <213> Sequenzidentische Primersequenz, spezifisch für den Bisulfit-konvertierten demethylierten antisense Strang des HERV-K Elements
<400> 873
   gtatatgttt tagagagtat 20
<210> 874
   <211> 20
   <212> DNA
   <213> Sequenzidentische Primersequenz, spezifisch für den Bisulfit-konvertierten demethylierten antisense Strang des HERV-K Elements
<400> 874
   tatttttttt tttttttttt 20
<210> 875
   <211> 20
   <212> DNA
   <213> Sequenzidentische Primersequenz, spezifisch für den Bisulfit-konvertierten demethylierten antisense Strang des HERV-K Elements
<400> 875
   tttttttttt ctataaaatt 20
<210> 876
   <211> 20
   <212> DNA
   <213> Sequenzidentische Primersequenz, spezifisch für den Bisulfit-konvertierten demethylierten antisense Strang des HERV-K Elements
<400> 876
   ttttctataa aatctttatt 20
<210> 877
   <211> 20
   <212> DNA
   <213> Sequenzidentische Primersequenz, spezifisch für den Bisulfit-konvertierten demethylierten antisense Strang des HERV-K Elements
<400> 877
   ctttatcttt attatggttt 20
<210> 878
   <211> 20
   <212> DNA
   <213> Sequenzidentische Primersequenz, spezifisch für den Bisulfit-konvertierten demethylierten antisense Strang des HERV-K Elements
<400> 878
   ctttattatg gttctttttt 20
<210> 879
   <211> 20
   <212> DNA
   <213> Sequenzidentische Primersequenz, spezifisch für den Bisulfit-konvertierten demethylierten antisense Strang des HERV-K Elements
<400> 879
   ttctatgtta ttgttttttt 20
<210> 880
   <211> 20
   <212> DNA
   <213> Sequenzidentische Primersequenz, spezifisch für den Bisulfit-konvertierten demethylierten antisense Strang des HERV-K Elements
<400> 880
   agataaaata ggtatataat 20
<210> 881
   <211> 20
   <212> DNA
   <213> Sequenzidentische Primersequenz, spezifisch für den Bisulfit-konvertierten demethylierten antisense Strang des HERV-K Elements
<400> 881
   gtgatagggt taagatttgt 20
<210> 882
   <211> 20
   <212> DNA
   <213> Sequenzidentische Primersequenz, spezifisch für den Bisulfit-konvertierten demethylierten antisense Strang des HERV-K Elements
<400> 882
   taatttttgt tatagtagtt 20

<210> 883
   <211> 20
   <212> DNA
   <213> Sequenzidentische Primersequenz, spezifisch für den Bisulfit-konvertierten demethylierten antisense Strang des HERV-K Elements
<400> 883
   tttttggatt tatttaaaat 20
<210> 884
   <211> 20
   <212> DNA
   <213> Sequenzidentische Primersequenz, spezifisch für den Bisulfit-konvertierten demethylierten antisense Strang des HERV-K Elements
<400> 884
   ttaaaatatg gatggatggt 20
<210> 885
   <211> 20
   <212> DNA
   <213> Sequenzidentische Primersequenz, spezifisch für den Bisulfit-konvertierten demethylierten antisense Strang des HERV-K Elements
<400> 885
   tggatggcta ggttttttat 20
<210> 886
   <211> 20
   <212> DNA
   <213> Sequenzidentische Primersequenz, spezifisch für den Bisulfit-konvertierten demethylierten antisense Strang des HERV-K Elements
<400> 886
   tggctaggtt ttttactgtt 20
<210> 887
   <211> 20
   <212> DNA
   <213> Sequenzidentische Primersequenz, spezifisch für den Bisulfit-konvertierten demethylierten antisense Strang des HERV-K Elements
<400> 887
   aggtttttta ctgtctgttt 20
<210> 888
   <211> 20
   <212> DNA
   <213> Sequenzidentische Primersequenz, spezifisch für den Bisulfit-konvertierten demethylierten antisense Strang des HERV-K Elements
<400> 888
   tgtttttatt agtagaatat 20
<210> 889
   <211> 20
   <212> DNA
   <213> Sequenzidentische Primersequenz, spezifisch für den Bisulfit-konvertierten demethylierten antisense Strang des HERV-K Elements
<400> 889
   cttaattttg taaaggaatt 20
<210> 890
   <211> 20
   <212> DNA
   <213> Sequenzidentische Primersequenz, spezifisch für den Bisulfit-konvertierten demethylierten antisense Strang des HERV-K Elements
<400> 890
   gttagaatgg aatttaggtt 20
<210> 891
   <211> 20
   <212> DNA
   <213> Sequenzidentische Primersequenz, spezifisch für den Bisulfit-konvertierten demethylierten antisense Strang des HERV-K Elements
<400> 891
   gatagtataa aatggtttat 20
<210> 892
   <211> 20
   <212> DNA
   <213> Sequenzidentische Primersequenz, spezifisch für den Bisulfit-konvertierten demethylierten antisense Strang des HERV-K Elements
<400> 892
   ttatttgtgt atttggatat 20
<210> 893
   <211> 20
   <212> DNA
   <213> Sequenzidentische Primersequenz, spezifisch für den Bisulfit-konvertierten demethylierten antisense Strang des HERV-K Elements
<400> 893
   attgtggtag aattgatttt 20
<210> 894
   <211> 20
   <212> DNA
   <213> Sequenzidentische Primersequenz, spezifisch für den Bisulfit-konvertierten demethylierten antisense Strang des HERV-K Elements
<400> 894
   gtttaattta taatagtttt 20
<210> 895
   <211> 20
   <212> DNA
   <213> Sequenzidentische Primersequenz, spezifisch für den Bisulfit-konvertierten demethylierten antisense Strang des HERV-K Elements
<400> 895
   gttttgtaaa taatttattt 20
<210> 896
   <211> 20
   <212> DNA
   <213> Sequenzidentische Primersequenz, spezifisch für den Bisulfit-konvertierten demethylierten antisense Strang des HERV-K Elements
<400> 896
   cttggtttga gtgatatttt 20
<210> 897
   <211> 20
   <212> DNA
   <213> Sequenzidentische Primersequenz, spezifisch für den Bisulfit-konvertierten demethylierten antisense Strang des HERV-K Elements
<400> 897
   tttaggtttg gtagggtagt 20
<210> 898
   <211> 20
   <212> DNA
   <213> Sequenzidentische Primersequenz, spezifisch für den Bisulfit-konvertierten demethylierten antisense Strang des HERV-K Elements
<400> 898
   tgattggtgt tattattttt 20
<210> 899
   <211> 20
   <212> DNA
   <213> Sequenzidentische Primersequenz, spezifisch für den Bisulfit-konvertierten demethylierten antisense Strang des HERV-K Elements
<400> 899
   gttattattt tcttggaggt 20
<210> 900
   <211> 20
   <212> DNA
   <213> Sequenzidentische Primersequenz, spezifisch für den Bisulfit-konvertierten demethylierten antisense Strang des HERV-K Elements
<400> 900
   gtattatata tgtagaattt 20
<210> 901
   <211> 20
   <212> DNA
   <213> Sequenzidentische Primersequenz, spezifisch für den Bisulfit-konvertierten demethylierten antisense Strang des HERV-K Elements
<400> 901
   agtatttttt aaaggtttat 20
<210> 902
   <211> 20
   <212> DNA
   <213> Sequenzidentische Primersequenz, spezifisch für den Bisulfit-konvertierten demethylierten antisense Strang des HERV-K Elements
<400> 902
   aggaatgttt agagttggtt 20
<210> 903
   <211> 20
   <212> DNA
   <213> Sequenzidentische Primersequenz, spezifisch für den Bisulfit-konvertierten demethylierten antisense Strang des HERV-K Elements
<400> 903
   atggggttat ataatgtagt 20
<210> 904
   <211> 20
   <212> DNA
   <213> Sequenzidentische Primersequenz, spezifisch für den Bisulfit-konvertierten demethylierten antisense Strang des HERV-K Elements
<400> 904
   ttattgttgt aataaatttt 20
<210> 905
   <211> 20
   <212> DNA
   <213> Sequenzidentische Primersequenz, spezifisch für den Bisulfit-konvertierten demethylierten antisense Strang des HERV-K Elements
<400> 905
   atttttgagg ttgtgtttat 20
<210> 906
   <211> 20
   <212> DNA
   <213> Sequenzidentische Primersequenz, spezifisch für den Bisulfit-konvertierten demethylierten antisense Strang des HERV-K Elements
<400> 906
   tttgaggttg tgtttacttt 20
<210> 907
   <211> 20
   <212> DNA
   <213> Sequenzidentische Primersequenz, spezifisch für den Bisulfit-konvertierten demethylierten antisense Strang des HERV-K Elements
<400> 907
   ttataagtat agttttatgt 20
<210> 908
   <211> 20
   <212> DNA
   <213> Sequenzidentische Primersequenz, spezifisch für den Bisulfit-konvertierten demethylierten antisense Strang des HERV-K Elements
<400> 908
   tttttttttt aggtggtatt 20
<210> 909
   <211> 20
   <212> DNA
   <213> Sequenzidentische Primersequenz, spezifisch für den Bisulfit-konvertierten demethylierten antisense Strang des HERV-K Elements
<400> 909
   taggtggtat ctgttttaat 20
<210> 910
   <211> 20
   <212> DNA
   <213> Sequenzidentische Primersequenz, spezifisch für den Bisulfit-konvertierten demethylierten antisense Strang des HERV-K Elements
<400> 910
   ttgatttttt gggggtggtt 20
<210> 911
   <211> 20
   <212> DNA
   <213> Sequenzidentische Primersequenz, spezifisch für den Bisulfit-konvertierten demethylierten antisense Strang des HERV-K Elements
<400> 911
   ggtggtctat attgaagttt 20
<210> 912
   <211> 20
   <212> DNA
   <213> Sequenzidentische Primersequenz, spezifisch für den Bisulfit-konvertierten demethylierten antisense Strang des HERV-K Elements
<400> 912
   gtctatattg aagttctgtt 20
<210> 913
   <211> 20
   <212> DNA
   <213> Sequenzidentische Primersequenz, spezifisch für den Bisulfit-konvertierten demethylierten antisense Strang des HERV-K Elements
<400> 913
   ttttattttt tttaatttgt 20
<210> 914
   <211> 20
   <212> DNA
   <213> Sequenzidentische Primersequenz, spezifisch für den Bisulfit-konvertierten demethylierten antisense Strang des HERV-K Elements
<400> 914
   gtttgaggtt gtaatgttat 20
<210> 915
   <211> 20
   <212> DNA
   <213> Sequenzidentische Primersequenz, spezifisch für den Bisulfit-konvertierten demethylierten antisense Strang des HERV-K Elements
<400> 915
   gctttgattg agttattaat 20
<210> 916
<211> 20
   <212> DNA
   <213> Sequenzidentische Primersequenz, spezifisch für den Bisulfit-konvertierten demethylierten antisense Strang des HERV-K Elements
<400> 916
   ttgttatttt agtttttttt 20
<210> 917
   <211> 20
   <212> DNA
   <213> Sequenzidentische Primersequenz, spezifisch für den Bisulfit-konvertierten demethylierten antisense Strang des HERV-K Elements
<400> 917
   ttctagtgta taagtttatt 20
<210> 918
   <211> 20
   <212> DNA
   <213> Sequenzidentische Primersequenz, spezifisch für den Bisulfit-konvertierten demethylierten antisense Strang des HERV-K Elements
<400> 918
   gatttgtttt taatgattat 20
<210> 919
   <211> 20
   <212> DNA
   <213> Sequenzidentische Primersequenz, spezifisch für den Bisulfit-konvertierten demethylierten antisense Strang des HERV-K Elements
<400> 919
   tgtttttaat gattactttt 20
<210> 920
   <211> 20
   <212> DNA
   <213> Sequenzidentische Primersequenz, spezifisch für den Bisulfit-konvertierten demethylierten antisense Strang des HERV-K Elements
<400> 920
   tatatttgtg ggtgtttttt 20
<210> 921
   <211> 20
   <212> DNA
   <213> Sequenzidentische Primersequenz, spezifisch für den Bisulfit-konvertierten demethylierten antisense Strang des HERV-K Elements
<400> 921
   agaaagaaat aagggggttt 20
<210> 922
   <211> 20
   <212> DNA
   <213> Sequenzidentische Primersequenz, spezifisch für den Bisulfit-konvertierten demethylierten antisense Strang des HERV-K Elements
<400> 922
   agggggttct gggaattaat 20
<210> 923
   <211> 20
   <212> DNA
   <213> Komplementäre Primersequenz, spezifisch für den Bisulfit-konvertierten methylierten antisense Strang des HERV-K Elements
<400> 923
   taaccttacc cccaaccccg 20
<210> 924
   <211> 20
   <212> DNA
   <213> Komplementäre Primersequenz, spezifisch für den Bisulfit-konvertierten methylierten antisense Strang des HERV-K Elements
<400> 924
   aattaaataa attaaaaacg 20
<210> 925
   <211> 20
   <212> DNA
   <213> Komplementäre Primersequenz, spezifisch für den Bisulfit-konvertierten methylierten antisense Strang des HERV-K Elements
<400> 925
   ccaaaaacac aaaaactacg 20
<210> 926
   <211> 20
   <212> DNA
   <213> Komplementäre Primersequenz, spezifisch für den Bisulfit-konvertierten methylierten antisense Strang des HERV-K Elements
<400> 926
   aaatctaaaa tataacctcg 20
<210> 927
   <211> 20
   <212> DNA
   <213> Komplementäre Primersequenz, spezifisch für den Bisulfit-konvertierten methylierten antisense Strang des HERV-K Elements
<400> 927
   aaaaaaaaaa aacctaaccg 20
<210> 928
   <211> 20
   <212> DNA
   <213> Komplementäre Primersequenz, spezifisch für den Bisulfit-konvertierten methylierten antisense Strang des HERV-K Elements
<400> 928
   cctaaccgtc ccccaacccg 20
<210> 929
   <211> 20
   <212> DNA
   <213> Komplementäre Primersequenz, spezifisch für den Bisulfit-konvertierten methylierten antisense Strang des HERV-K Elements
<400> 929
   ctaaacaata aaatatctcg 20
<210> 930
   <211> 20
   <212> DNA
   <213> Komplementäre Primersequenz, spezifisch für den Bisulfit-konvertierten methylierten antisense Strang des HERV-K Elements
<400> 930
   atatctcgat ataaaacccg 20
<210> 931
   <211> 20
   <212> DNA
   <213> Komplementäre Primersequenz, spezifisch für den Bisulfit-konvertierten methylierten antisense Strang des HERV-K Elements
<400> 931
   atataaaacc cgattatacg 20
<210> 932
   <211> 20
   <212> DNA
   <213> Komplementäre Primersequenz, spezifisch für den Bisulfit-konvertierten methylierten antisense Strang des HERV-K Elements
<400> 932
   tacaaaaacc tttattcacg 20
<210> 933
   <211> 20
   <212> DNA
   <213> Komplementäre Primersequenz, spezifisch für den Bisulfit-konvertierten methylierten antisense Strang des HERV-K Elements
<400> 933
   ctctcaaaaa aacacccacg 20
<210> 934
   <211> 20
   <212> DNA
   <213> Komplementäre Primersequenz, spezifisch für den Bisulfit-konvertierten methylierten antisense Strang des HERV-K Elements
<400> 934
   aatcctccat atactaaacg 20
<210> 935
   <211> 20
   <212> DNA
   <213> Komplementäre Primersequenz, spezifisch für den Bisulfit-konvertierten methylierten antisense Strang des HERV-K Elements
<400> 935
   tactaaacgt taattccccg 20
<210> 936
   <211> 20
   <212> DNA
   <213> Komplementäre Primersequenz, spezifisch für den Bisulfit-konvertierten methylierten antisense Strang des HERV-K Elements
<400> 936
   atctctcatt acaccttacg 20
<210> 937
   <211> 20
   <212> DNA
   <213> Komplementäre Primersequenz, spezifisch für den Bisulfit-konvertierten methylierten antisense Strang des HERV-K Elements
<400> 937
   ccttcatcta atacccaacg 20
<210> 938
   <211> 20
   <212> DNA
   <213> Komplementäre Primersequenz, spezifisch für den Bisulfit-konvertierten methylierten antisense Strang des HERV-K Elements
<400> 938
   ctaaaataaa aatacactcg 20
<210> 939
   <211> 20
   <212> DNA
   <213> Komplementäre Primersequenz, spezifisch für den Bisulfit-konvertierten methylierten antisense Strang des HERV-K Elements
<400> 939
   aataaaaata cactcgaacg 20
<210> 940
   <211> 20
   <212> DNA
   <213> Komplementäre Primersequenz, spezifisch für den Bisulfit-konvertierten methylierten antisense Strang des HERV-K Elements
<400> 940
   taatcattaa aaacaaatcg 20
<210> 941
   <211> 20
   <212> DNA
   <213> Komplementäre Primersequenz, spezifisch für den Bisulfit-konvertierten methylierten antisense Strang des HERV-K Elements
<400> 941
   atttcaaaca aaaaataacg 20
<210> 942
   <211> 20
   <212> DNA
   <213> Komplementäre Primersequenz, spezifisch für den Bisulfit-konvertierten methylierten antisense Strang des HERV-K Elements
<400> 942
   aaaaatccca aaaaaaaacg 20
<210> 943
   <211> 20
   <212> DNA
   <213> Komplementäre Primersequenz, spezifisch für den Bisulfit-konvertierten methylierten antisense Strang des HERV-K Elements
<400> 943
   aacgaaaact ttacattacg 20
<210> 944
   <211> 20
   <212> DNA
   <213> Komplementäre Primersequenz, spezifisch für den Bisulfit-konvertierten methylierten antisense Strang des HERV-K Elements
<400> 944
   acgaatatat aacaaaaccg 20
<210> 945
   <211> 20
   <212> DNA
   <213> Komplementäre Primersequenz, spezifisch für den Bisulfit-konvertierten methylierten antisense Strang des HERV-K Elements
<400> 945
   cgttaataac tcaatcaacg 20
<210> 946
   <211> 20
   <212> DNA
   <213> Komplementäre Primersequenz, spezifisch für den Bisulfit-konvertierten methylierten antisense Strang des HERV-K Elements
<400> 946
   cgttaataac tcaatcaacg 20
<210> 947
   <211> 20
   <212> DNA
   <213> Komplementäre Primersequenz, spezifisch für den Bisulfit-konvertierten methylierten antisense Strang des HERV-K Elements
<400> 947
   ttcaacaaat caaataaccg 20
<210> 948
   <211> 20
   <212> DNA
   <213> Komplementäre Primersequenz, spezifisch für den Bisulfit-konvertierten methylierten antisense Strang des HERV-K Elements
<400> 948
   taacattaca acctcaaacg 20
<210> 949
   <211> 20
   <212> DNA
   <213> Komplementäre Primersequenz, spezifisch für den Bisulfit-konvertierten methylierten antisense Strang des HERV-K Elements
<400> 949
   cttatcaata ctaaccaccg 20
<210> 950
   <211> 20
   <212> DNA
   <213> Komplementäre Primersequenz, spezifisch für den Bisulfit-konvertierten methylierten antisense Strang des HERV-K Elements
<400> 950
   tcaatactaa ccaccgaccg 20
<210> 951
   <211> 20
   <212> DNA
   <213> Komplementäre Primersequenz, spezifisch für den Bisulfit-konvertierten methylierten antisense Strang des HERV-K Elements
<400> 951
   ccgaccgaac ttcaatatcg 20

<210> 952
   <211> 20
   <212> DNA
   <213> Komplementäre Primersequenz, spezifisch für den Bisulfit-konvertierten methylierten antisense Strang des HERV-K Elements
<400> 952
   attaccaata aaaaaacccg 20
<210> 953
   <211> 20
   <212> DNA
   <213> Komplementäre Primersequenz, spezifisch für den Bisulfit-konvertierten methylierten antisense Strang des HERV-K Elements
<400> 953
   aataaaatta ataacatacg 20
<210> 954
   <211> 20
   <212> DNA
   <213> Komplementäre Primersequenz, spezifisch für den Bisulfit-konvertierten methylierten antisense Strang des HERV-K Elements
<400> 954
   attaataaca tacgaaaacg 20
<210> 955
   <211> 20
   <212> DNA
   <213> Komplementäre Primersequenz, spezifisch für den Bisulfit-konvertierten methylierten antisense Strang des HERV-K Elements
<400> 955
   tcaaaatata taaaaacccg 20
<210> 956
   <211> 20
   <212> DNA
   <213> Komplementäre Primersequenz, spezifisch für den Bisulfit-konvertierten methylierten antisense Strang des HERV-K Elements
<400> 956
   ataaaattaa aaaaactacg 20
<210> 957
   <211> 20
   <212> DNA
   <213> Komplementäre Primersequenz, spezifisch für den Bisulfit-konvertierten methylierten antisense Strang des HERV-K Elements
<400> 957
   aaaataaaca accattatcg 20
<210> 958
   <211> 20
   <212> DNA
   <213> Komplementäre Primersequenz, spezifisch für den Bisulfit-konvertierten methylierten antisense Strang des HERV-K Elements
<400> 958
   ctaatcttaa aaaaatcacg 20
<210> 959
   <211> 20
   <212> DNA
   <213> Komplementäre Primersequenz, spezifisch für den Bisulfit-konvertierten methylierten antisense Strang des HERV-K Elements
<400> 959
   aatttaaaaa cactaatccg 20
<210> 960
   <211> 20
   <212> DNA
   <213> Komplementäre Primersequenz, spezifisch für den Bisulfit-konvertierten methylierten antisense Strang des HERV-K Elements
<400> 960
   aactattaca aaacttatcg 20
<210> 961
   <211> 20
   <212> DNA
   <213> Komplementäre Primersequenz, spezifisch für den Bisulfit-konvertierten methylierten antisense Strang des HERV-K Elements
<400> 961
   tattacaaaa cttatcgacg 20
<210> 962
   <211> 20
   <212> DNA
   <213> Komplementäre Primersequenz, spezifisch für den Bisulfit-konvertierten methylierten antisense Strang des HERV-K Elements
<400> 962
   tattacaaca ataaaatacg 20
<210> 963
   <211> 20
   <212> DNA
   <213> Komplementäre Primersequenz, spezifisch für den Bisulfit-konvertierten methylierten antisense Strang des HERV-K Elements
<400> 963
   aaaaatatta attaaattcg 20
<210> 964
   <211> 20
   <212> DNA
   <213> Komplementäre Primersequenz, spezifisch für den Bisulfit-konvertierten methylierten antisense Strang des HERV-K Elements
<400> 964
   attaatccga caaaattacg 20
<210> 965
   <211> 20
   <212> DNA
   <213> Komplementäre Primersequenz, spezifisch für den Bisulfit-konvertierten methylierten antisense Strang des HERV-K Elements
<400> 965
   tcaaaactcc atatcaatcg 20
<210> 966
   <211> 20
   <212> DNA
   <213> Komplementäre Primersequenz, spezifisch für den Bisulfit-konvertierten methylierten antisense Strang des HERV-K Elements
<400> 966
   caaaaaaaaa cgcctccacg 20
<210> 967
   <211> 20
   <212> DNA
   <213> Komplementäre Primersequenz, spezifisch für den Bisulfit-konvertierten methylierten antisense Strang des HERV-K Elements
<400> 967
   gaaatatcac tcaaaccacg 20
<210> 968
   <211> 20
   <212> DNA
   <213> Komplementäre Primersequenz, spezifisch für den Bisulfit-konvertierten methylierten antisense Strang des HERV-K Elements
<400> 968
   attataaatt aaacacctcg 20
<210> 969
   <211> 20
   <212> DNA
   <213> Komplementäre Primersequenz, spezifisch für den Bisulfit-konvertierten methylierten antisense Strang des HERV-K Elements
<400> 969
   actcaaaaca aactcaatcg 20
<210> 970
   <211> 20
   <212> DNA
   <213> Komplementäre Primersequenz, spezifisch für den Bisulfit-konvertierten methylierten antisense Strang des HERV-K Elements
<400> 970
   acaaataaat ccaactatcg 20
<210> 971
   <211> 20
   <212> DNA
   <213> Komplementäre Primersequenz, spezifisch für den Bisulfit-konvertierten methylierten antisense Strang des HERV-K Elements
<400> 971
   aaatccaact atcgataacg 20
<210> 972
   <211> 20
   <212> DNA
   <213> Komplementäre Primersequenz, spezifisch für den Bisulfit-konvertierten methylierten antisense Strang des HERV-K Elements
<400> 972
   actttaaaaa caaaatatcg 20
<210> 973
   <211> 20
   <212> DNA
   <213> Komplementäre Primersequenz, spezifisch für den Bisulfit-konvertierten methylierten antisense Strang des HERV-K Elements
<400> 973
   taaaccattt tatactatcg 20
<210> 974
   <211> 20
   <212> DNA
   <213> Komplementäre Primersequenz, spezifisch für den Bisulfit-konvertierten methylierten antisense Strang des HERV-K Elements
<400> 974
   acctaaattc cattctaacg 20
<210> 975
   <211> 20
   <212> DNA
   <213> Komplementäre Primersequenz, spezifisch für den Bisulfit-konvertierten methylierten antisense Strang des HERV-K Elements
<400> 975
   tataaatccc tatatccacg 20
<210> 976
   <211> 20
   <212> DNA
   <213> Komplementäre Primersequenz, spezifisch für den Bisulfit-konvertierten methylierten antisense Strang des HERV-K Elements
<400> 976
   atccctatat ccacgaaccg 20
<210> 977
   <211> 20
   <212> DNA
   <213> Komplementäre Primersequenz, spezifisch für den Bisulfit-konvertierten methylierten antisense Strang des HERV-K Elements
<400> 977
   ctatatccac gaaccgaccg 20
<210> 978
   <211> 20
   <212> DNA
   <213> Komplementäre Primersequenz, spezifisch für den Bisulfit-konvertierten methylierten antisense Strang des HERV-K Elements
<400> 978
   accgaccgta aaaaacctcg 20
<210> 979
   <211> 20
   <212> DNA
   <213> Komplementäre Primersequenz, spezifisch für den Bisulfit-konvertierten methylierten antisense Strang des HERV-K Elements
<400> 979
   cactaaaaca taattaaacg 20
<210> 980
   <211> 20
   <212> DNA
   <213> Komplementäre Primersequenz, spezifisch für den Bisulfit-konvertierten methylierten antisense strang des HERV-K Elements
<400> 980
   aaaaattact aataacctcg 20
<210> 981
   <211> 20
   <212> DNA
   <213> Komplementäre Primersequenz, spezifisch für den Bisulfit-konvertierten methylierten antisense Strang des HERV-K Elements
<400> 981
   ccgaaaaaac aataacatcg 20
<210> 982
   <211> 20
   <212> DNA
   <213> Komplementäre Primersequenz, spezifisch für den Bisulfit-konvertierten methylierten antisense Strang des HERV-K Elements
<400> 982
   aaacaataac atcgaaaacg 20
<210> 983
   <211> 20
   <212> DNA
   <213> Komplementäre Primersequenz, spezifisch für den Bisulfit-konvertierten methylierten antisense Strang des HERV-K Elements
<400> 983
   gaaaacgaac cataataacg 20
<210> 984
   <211> 20
   <212> DNA
   <213> Komplementäre Primersequenz, spezifisch für den Bisulfit-konvertierten methylierten antisense Strang des HERV-K Elements
<400> 984
   gaaccataat aacgataacg 20
<210> 985
   <211> 20
   <212> DNA
   <213> Komplementäre Primersequenz, spezifisch für den Bisulfit-konvertierten methylierten antisense Strang des HERV-K Elements
<400> 985
   taacgataac gattttatcg 20
<210> 986
   <211> 20
   <212> DNA
   <213> Komplementäre Primersequenz, spezifisch für den Bisulfit-konvertierten demethylierten antisense Strang des HERV-K Elements
<400> 986
   taaccttacc cccaacccca 20
<210> 987
   <211> 20
   <212> DNA
   <213> Komplementäre Primersequenz, spezifisch für den Bisulfit-konvertierten demethylierten antisense Strang des HERV-K Elements
<400> 987
   aattaaataa attaaaaaca 20
<210> 988
   <211> 20
   <212> DNA
   <213> Komplementäre Primersequenz, spezifisch für den Bisulfit-konvertierten demethylierten antisense Strang des HERV-K Elements
<400> 988
   ccaaaaacac aaaaactaca 20
<210> 989
   <211> 20
   <212> DNA
   <213> Komplementäre Primersequenz, spezifisch für den Bisulfit-konvertierten demethylierten antisense Strang des HERV-K Elements
<400> 989
   aaatctaaaa tataacctca 20
<210> 990
   <211> 20
   <212> DNA
   <213> Komplementäre Primersequenz, spezifisch für den Bisulfit-konvertierten demethylierten antisense Strang des HERV-K Elements
<400> 990
   aaaaaaaaaa aacctaacca 20
<210> 991
   <211> 20
   <212> DNA
   <213> Komplementäre Primersequenz, spezifisch für den Bisulfit-konvertierten demethylierten antisense Strang des HERV-K Elements
<400> 991
   cctaaccatc ccccaaccca 20
<210> 992
   <211> 20
   <212> DNA
   <213> Komplementäre Primersequenz, spezifisch für den Bisulfit-konvertierten demethylierten antisense Strang des HERV-K Elements
<400> 992
   ctaaacaata aaatatctca 20
<210> 993
   <211> 20
   <212> DNA
   <213> Komplementäre Primersequenz, spezifisch für den Bisulfit-konvertierten demethylierten antisense Strang des HERV-K Elements
<400> 993
   atatctcaat ataaaaccca 20
<210> 994
   <211> 20
   <212> DNA
   <213> Komplementäre Primersequenz, spezifisch für den Bisulfit-konvertierten demethylierten antisense Strang des HERV-K Elements
<400> 994
   atataaaacc caattataca 20
<210> 995
   <211> 20
   <212> DNA
   <213> Komplementäre Primersequenz, spezifisch für den Bisulfit-konvertierten demethylierten antisense Strang des HERV-K Elements
<400> 995
   tacaaaaacc tttattcaca 20
<210> 996
   <211> 20
   <212> DNA
   <213> Komplementäre Primersequenz, spezifisch für den Bisulfit-konvertierten demethylierten antisense Strang des HERV-K Elements
<400> 996
   ctctcaaaaa aacacccaca 20
<210> 997
   <211> 20
   <212> DNA
   <213> Komplementäre Primersequenz, spezifisch für den Bisulfit-konvertierten demethylierten antisense Strang des HERV-K Elements
<400> 997
   aatcctccat atactaaaca 20
<210> 998
   <211> 20
   <212> DNA
   <213> Komplementäre Primersequenz, spezifisch für den Bisulfit-konvertierten demethylierten antisense Strang des HERV-K Elements
<400> 998
   tactaaacat taattcccca 20
<210> 999
   <211> 20
   <212> DNA
   <213> Komplementäre Primersequenz, spezifisch für den Bisulfit-konvertierten demethylierten antisense Strang des HERV-K Elements
<400> 999
   atctctcatt acaccttaca 20
<210> 1000
   <211> 20
   <212> DNA
   <213> Komplementäre Primersequenz, spezifisch für den Bisulfit-konvertierten demethylierten antisense Strang des HERV-K Elements
<400> 1000
   ccttcatcta atacccaaca 20
<210> 1001
   <211> 20
   <212> DNA
   <213> Komplementäre Primersequenz, spezifisch für den Bisulfit-konvertierten demethylierten antisense Strang des HERV-K Elements
<400> 1001
   ctaaaataaa aatacactca 20
<210> 1002
   <211> 20
   <212> DNA
   <213> Komplementäre Primersequenz, spezifisch für den Bisulfit-konvertierten demethylierten antisense Strang des HERV-K Elements
<400> 1002
   aataaaaata cactcaaaca 20
<210> 1003
   <211> 20
   <212> DNA
   <213> Komplementäre Primersequenz, spezifisch für den Bisulfit-konvertierten demethylierten antisense Strang des HERV-K Elements
<400> 1003
   taatcattaa aaacaaatca 20
<210> 1004
   <211> 20
   <212> DNA
   <213> Komplementäre Primersequenz, spezifisch für den Bisulfit-konvertierten demethylierten antisense Strang des HERV-K Elements
<400> 1004
   atttcaaaca aaaaataaca 20
<210> 1005
   <211> 20
   <212> DNA
   <213> Komplementäre Primersequenz, spezifisch für den Bisulfit-konvertierten demethylierten antisense Strang des HERV-K Elements
<400> 1005
   aaaaatccca aaaaaaaaca 20
<210> 1006
   <211> 20
   <212> DNA
   <213> Komplementäre Primersequenz, spezifisch für den Bisulfit-konvertierten demethylierten antisense Strang des HERV-K Elements
<400> 1006
   aacaaaaact ttacattaca 20
<210> 1007
   <211> 20
   <212> DNA
   <213> Komplementäre Primersequenz, spezifisch für den Bisulfit-konvertierten demethylierten antisense Strang des HERV-K Elements
<400> 1007
   acaaatatat aacaaaacca 20
<210> 1008
   <211> 20
   <212> DNA
   <213> Komplementäre Primersequenz, spezifisch für den Bisulfit-konvertierten demethylierten antisense Strang des HERV-K Elements
<400> 1008
   cattaataac tcaatcaaca 20
<210> 1009
   <211> 20
   <212> DNA
   <213> Komplementäre Primersequenz, spezifisch für den Bisulfit-konvertierten demethylierten antisense Strang des HERV-K Elements
<400> 1009
   ccattaaaat ctaaaccaca 20
<210> 1010
   <211> 20
   <212> DNA
   <213> Komplementäre Primersequenz, spezifisch für den Bisulfit-konvertierten demethylierten antisense Strang des HERV-K Elements
<400> 1010
   ttcaacaaat caaataacca 20
<210> 1011
   <211> 20
   <212> DNA
   <213> Komplementäre Primersequenz, spezifisch für den Bisulfit-konvertierten demethylierten antisense Strang des HERV-K Elements
<400> 1011
   taacattaca acctcaaaca 20
<210> 1012
   <211> 20
   <212> DNA
   <213> Komplementäre Primersequenz, spezifisch für den Bisulfit-konvertierten demethylierten antisense Strang des HERV-K Elements
<400> 1012
   cttatcaata ctaaccacca 20
<210> 1013
   <211> 20
   <212> DNA
   <213> Komplementäre Primersequenz, spezifisch für den Bisulfit-konvertierten demethylierten antisense Strang des HERV-K Elements
<400> 1013
   tcaatactaa ccaccaacca 20
<210> 1014
   <211> 20
   <212> DNA
   <213> Komplementäre Primersequenz, spezifisch für den Bisulfit-konvertierten demethylierten antisense Strang des HERV-K Elements
<400> 1014
   ccaaccaaac ttcaatatca 20
<210> 1015
   <211> 20
   <212> DNA
   <213> Komplementäre Primersequenz, spezifisch für den Bisulfit-konvertierten demethylierten antisense Strang des HERV-K Elements
<400> 1015
   attaccaata aaaaaaccca 20
<210> 1016
   <211> 20
   <212> DNA
   <213> Komplementäre Primersequenz, spezifisch für den Bisulfit-konvertierten demethylierten antisense Strang des HERV-K Elements
<400> 1016
   aataaaatta ataacataca 20
<210> 1017
   <211> 20
   <212> DNA
   <213> Komplementäre Primersequenz, spezifisch für den Bisulfit-konvertierten demethylierten antisense Strang des HERV-K Elements
<400> 1017
   attaataaca tacaaaaaca 20
<210> 1018
   <211> 20
   <212> DNA
   <213> Komplementäre Primersequenz, spezifisch für den Bisulfit-konvertierten demethylierten antisense Strang des HERV-K Elements
<400> 1018
   tcaaaatata taaaaaccca 20
<210> 1019
   <211> 20
   <212> DNA
   <213> Komplementäre Primersequenz, spezifisch für den Bisulfit-konvertierten demethylierten antisense Strang des HERV-K Elements
<400> 1019
   ataaaattaa aaaaactaca 20
<210> 1020
   <211> 20
   <212> DNA
   <213> Komplementäre Primersequenz, spezifisch für den Bisulfit-konvertierten demethylierten antisense Strang des HERV-K Elements
<400> 1020
   aaaataaaca accattatca 20

<210> 1021
   <211> 20
   <212> DNA
   <213> Komplementäre Primersequenz, spezifisch für den Bisulfit-konvertierten demethylierten antisense Strang des HERV-K Elements
<400> 1021
   ctaatcttaa aaaaatcaca 20
<210> 1022
   <211> 20
   <212> DNA
   <213> Komplementäre Primersequenz, spezifisch für den Bisulfit-konvertierten demethylierten antisense Strang des HERV-K Elements
<400> 1022
   aatttaaaaa cactaatcca 20
<210> 1023
   <211> 20
   <212> DNA
   <213> Komplementäre Primersequenz, spezifisch für den Bisulfit-konvertierten demethylierten antisense Strang des HERV-K Elements
<400> 1023
   aactattaca aaacttatca 20
<210> 1024
   <211> 20
   <212> DNA
   <213> Komplementäre Primersequenz, spezifisch für den Bisulfit-konvertierten demethylierten antisense Strang des HERV-K Elements
<400> 1024
   tattacaaaa cttatcaaca 20
<210> 1025
   <211> 20
   <212> DNA
   <213> Komplementäre Primersequenz, spezifisch für den Bisulfit-konvertierten demethylierten antisense Strang des HERV-K Elements
<400> 1025
   tattacaaca ataaaataca 20
<210> 1026
   <211> 20
   <212> DNA
   <213> Komplementäre Primersequenz, spezifisch für den Bisulfit-konvertierten demethylierten antisense Strang des HERV-K Elements
<400> 1026
   aaaaatatta attaaattca 20
<210> 1027
   <211> 20
   <212> DNA
   <213> Komplementäre Primersequenz, spezifisch für den Bisulfit-konvertierten demethylierten antisense Strang des HERV-K Elements
<400> 1027
   attaatccaa caaaattaca 20
<210> 1028
   <211> 20
   <212> DNA
   <213> Komplementäre Primersequenz, spezifisch für den Bisulfit-konvertierten demethylierten antisense Strang des HERV-K Elements
<400> 1028
   tcaaaactcc atatcaatca 20
<210> 1029
   <211> 20
   <212> DNA
   <213> Komplementäre Primersequenz, spezifisch für den Bisulfit-konvertierten demethylierten antisense Strang des HERV-K Elements
<400> 1029
   caaaaaaaaa cacctccaca 20
<210> 1030
   <211> 20
   <212> DNA
   <213> Komplementäre Primersequenz, spezifisch für den Bisulfit-konvertierten demethylierten antisense Strang des HERV-K Elements
<400> 1030
   gaaatatcac tcaaaccaca 20
<210> 1031
   <211> 20
   <212> DNA
   <213> Komplementäre Primersequenz, spezifisch für den Bisulfit-konvertierten demethylierten antisense Strang des HERV-K Elements
<400> 1031
   attataaatt aaacacctca 20
<210> 1032
   <211> 20
   <212> DNA
   <213> Komplementäre Primersequenz, spezifisch für den Bisulfit-konvertierten demethylierten antisense Strang des HERV-K Elements
<400> 1032
   actcaaaaca aactcaatca 20
<210> 1033
   <211> 20
   <212> DNA
   <213> Komplementäre Primersequenz, spezifisch für den Bisulfit-konvertierten demethylierten antisense Strang des HERV-K Elements
<400> 1033
   acaaataaat ccaactatca 20
<210> 1034
   <211> 20
   <212> DNA
   <213> Komplementäre Primersequenz, spezifisch für den Bisulfit-konvertierten demethylierten antisense Strang des HERV-K Elements
<400> 1034
   aaatccaact atcaataaca 20
<210> 1035
   <211> 20
   <212> DNA
   <213> Komplementäre Primersequenz, spezifisch für den Bisulfit-konvertierten demethylierten antisense Strang des HERV-K Elements
<400> 1035
   actttaaaaa caaaatatca 20
<210> 1036
   <211> 20
   <212> DNA
   <213> Komplementäre Primersequenz, spezifisch für den Bisulfit-konvertierten demethylierten antisense Strang des HERV-K Elements
<400> 1036
   taaaccattt tatactatca 20
<210> 1037
   <211> 20
   <212> DNA
   <213> Komplementäre Primersequenz, spezifisch für den Bisulfit-konvertierten demethylierten antisense Strang des HERV-K Elements
<400> 1037
   acctaaattc cattctaaca 20
<210> 1038
   <211> 20
   <212> DNA
   <213> Komplementäre Primersequenz, spezifisch für den Bisulfit-konvertierten demethylierten antisense Strang des HERV-K Elements
<400> 1038
   tataaatccc tatatccaca 20
<210> 1039
   <211> 20
   <212> DNA
   <213> Komplementäre Primersequenz, spezifisch für den Bisulfit-konvertierten demethylierten antisense Strang des HERV-K Elements
<400> 1039
   atccctatat ccacaaacca 20
<210> 1040
   <211> 20
   <212> DNA
   <213> Komplementäre Primersequenz, spezifisch für den Bisulfit-konvertierten demethylierten antisense Strang des HERV-K Elements
<400> 1040
   ctatatccac aaaccaacca 20
<210> 1041
   <211> 20
   <212> DNA
   <213> Komplementäre Primersequenz, spezifisch für den Bisulfit-konvertierten demethylierten antisense Strang des HERV-K Elements
<400> 1041 20
   accaaccata aaaaacctca 20
<210> 1042
   <211> 20
   <212> DNA
   <213> Komplementäre Primersequenz, spezifisch für den Bisulfit-konvertierten demethylierten antisense Strang des HERV-K Elements
<400> 1042
   cactaaaaca taattaaaca 20
<210> 1043
   <211> 20
   <212> DNA
   <213> Komplementäre Primersequenz, spezifisch für den Bisulfit-konvertierten demethylierten antisense Strang des HERV-K Elements
<400> 1043
   aaaaattact aataacctca 20
<210> 1044
   <211> 20
   <212> DNA
   <213> Komplementäre Primersequenz, spezifisch für den Bisulfit-konvertierten demethylierten antisense Strang des HERV-K Elements
<400> 1044
   ccaaaaaaac aataacatca 20
<210> 1045
   <211> 20
   <212> DNA
   <213> Komplementäre Primersequenz, spezifisch für den Bisulfit-konvertierten demethylierten antisense Strang des HERV-K Elements
<400> 1045
   aaacaataac atcaaaaaca 20
<210> 1046
   <211> 20
   <212> DNA
   <213> Komplementäre Primersequenz, spezifisch für den Bisulfit-konvertierten demethylierten antisense Strang des HERV-K Elements
<400> 1046
   gaaaacaaac cataataaca 20
<210> 1047
   <211> 20
   <212> DNA
   <213> Komplementäre Primersequenz, spezifisch für den Bisulfit-konvertierten demethylierten antisense Strang des HERV-K Elements
<400> 1047
   gaaccataat aacaataaca 20
<210> 1048
   <211> 20
   <212> DNA
   <213> Komplementäre Primersequenz, spezifisch für den Bisulfit-konvertierten demethylierten antisense Strang des HERV-K Elements
<400> 1048
   taacaataac aattttatca 20
<210> 1049
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1049
   gtgatttttg tatttttatt 20
<210> 1050
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1050
   ggtttatttt attagggagt 20
<210> 1051
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1051
   agggagtgtt agatagtggg 20
<210> 1052
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1052
   aggtattgtt ttatttggga 20
<210> 1053
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1053
   taaggggtta gggagttttt 20
<210> 1054
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1054
   tttttgagtt aaagaaaggg 20
<210> 1055
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1055
   agattatatt ttatatttgg 20
<210> 1056
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1056
   ttgattgtta gtatagtagt 20
<210> 1057
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1057
   tttgagatta aattgtaagg 20
<210> 1058
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 1058
   ttattgttta ggtttgttta 20
<210> 1059
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1059
   gtttaggtaa ataaagtagt 20
<210> 1060
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1060
   aattgggtgg agtttattat 20
<210> 1061
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1061
   tagtttaagg aggtttgttt 20
<210> 1062
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1062
   gtttttgtag gttttatttt 20
<210> 1063
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1063
   tgggggtagg gtatagataa 20
<210> 1064
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1064
   ataaaaagat agtagtaatt 20
<210> 1065
   <211> 20
   <212> DNA
   <213> SEQUENZIDENTISCHER Primer, spezifisch für den Bisulfit-konvertierten METHYLIERTEN SENSE Strang des Promotorbereichs des LINE-1 Elements
<400> 1065
   tttgtagatt taagtgtttt 20
<210> 1066
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1066
   tgtttgatag ttttgaagag 20
<210> 1067
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1067
   gagtagtggt ttttttagta 20
<210> 1068
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1068
   ggtagataga ttgttttttt 20
<210> 1069
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1069
   aagtgggttt ttgatttttg 20
<210> 1070
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1070
   attttcgagt agtttaattg 20
<210> 1071
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1071
   ggaggtattt tttagtaggg 20
<210> 1072
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1072
   gtagggtatt ttaatagatt 20
<210> 1073
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1073
   gtagggtatt ttaatagatt 20
<210> 1074
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1074
   tgtagttgag ggttttgttt 20
<210> 1075
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1075
   ttagaaggaa aattaataat 20
<210> 1076
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1076
   attagaaagg atatttatat 20
<210> 1077
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1077
   aaaatttatt tgtatattat 20
<210> 1078
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1078
   tattattaaa gattaaaagt 20
<210> 1079
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1079
   agataaaatt ataaagatgg 20
<210> 1080
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1080
   ggaaaaaata gaatagaaaa 20
<210> 1081
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1081
   aaaaattgga aattttaaaa 20
<210> 1082
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1082
   tttttttttt tttaaaggaa 20
<210> 1083
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1083
   tagtttttta ttagtaatag 20
<210> 1084
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1084
   aataaagttg gatggagaat 20
<210> 1085
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1085
   agttgagaga agaaggtttt 20
<210> 1086
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1086
   agacgattaa attattttga 20
<210> 1087
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1087
   ggaggatatt taaattaaag 20
<210> 1088
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1088
   gtaaagaagt tgaaaatttt 20
<210> 1089
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1089
   tgaaaaaaat ttagaagaat 20

<210> 1090
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1090
   gtataattag aataattaat 20
<210> 1091
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1091
   atagagaagt gtttaaagga 20
<210> 1092
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1092
   gttgatggag ttgaaaatta 20
<210> 1093
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1093
   tgaagaatgt agaagtttta 20
<210> 1094
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1094
   attaattgga agaaagggta 20
<210> 1095
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1095
   ttagtaatgg aagatgaaat 20
<210> 1096
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1096
   agaagggaag tttagagaaa 20
<210> 1097
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1097
   ctctatattt cctaaatcta 20
<210> 1098
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1098
   ttaacctacc ttactaaatt 20
<210> 1099
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1099
   taaataatat cctacaaaat 20
<210> 1100
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1100
   cacatcactt tcaaatacac 20
<210> 1101
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1101
   atttaatctt ttcacataat 20
<210> 1102
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1102
   cttaaaaact ttactcattt 20
<210> 1103
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1103
   ttattctttt ttctctaaac 20
<210> 1104
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1104
   ttcatttcat tcatttcatc 20
<210> 1105
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1105
   ataccctttc ttccaattaa 20
<210> 1106
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1106
   cctaaaactt ctacattctt 20
<210> 1107
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1107
   attttcaact ccatcaactc 20
<210> 1108
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1108
   ttattctaat tatacattct 20
<210> 1109
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1109
   aaaattttca acttctttac 20
<210> 1110
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1110
   gtaactcaaa ataatttaat 20
<210> 1111
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1111
   aaaaccttct tctctcaact 20
<210> 1112
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1112
   gtcaaaatca ttctccatcc 20
<210> 1113
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1113
   attctattac taataaaaaa 20
<210> 1114
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1114
   gttcctttaa aaaaaaaaaa 20
<210> 1115
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1115
   tttaaaattt ccaatttttc 20
<210> 1116
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1116
   cccatcttta taattttatc 20
<210> 1117
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1117
   taatctttaa taataataat 20
<210> 1118
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1118
   taaatatcct ttctaattat 20
<210> 1119
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1119
   caaacaaaac cctcaactac 20
<210> 1120
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1120
   gtataaaata tcaatatacc 20
<210> 1121
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1121
   aaatacctcc caattaaact 20
<210> 1122
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1122
   aaaatcaaaa acccacttaa 20
<210> 1123
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1123
   aaaaaaacaa tctatctacc 20
<210> 1124
   <211> 19
   <212> DNA
   <213> Sequenzidentische und komplementäre Primersequenz, spezifisch für den Bisulfit-konvertierten sense Strang des Promotorbereichs des LINE-1 Elements
<400> 1124
   gttctcaaat ctccaacta 19
<210> 1125
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1125
   actaaaaaaa ccactactct 20
<210> 1126
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1126
   aaacaaaaac acttaaatct 20
<210> 1127
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1127
   attactacta tctttttatt 20
<210> 1128
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1128
   ccccaaaaat aaaacctaca 20
<210> 1129
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1129
   caaacctcct taaactataa 20
<210> 1130
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1130
   tttacctaaa caaacctaaa 20
<210> 1131
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1131
   caatttaatc tcaaactact 20
<210> 1132
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1132
   ccaaatataa aatataatct 20
<210> 1133
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1133
   cacccctttc tttaactcaa 20
<210> 1134
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1134
   aaaaactccc taacccctta 20
<210> 1135
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1135
   tcccaaataa aacaatacct 20
<210> 1136
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1136
   tctaacactc cctaataaaa 20
<210> 1137
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1137
   acctcaaata aaaatacaaa 20
<210> 1138
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1138
   accatcttaa ctcctccccc 20
<210> 1139
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1139
   cactaaaaca taattaaaca 20
<210> 1140
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1140
   aaaaattact aataacctca 20
<210> 1141
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1141
   ccaaaaaaac aataacatca 20
<210> 1142
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1142
   aaacaataac atcaaaaaca 20
<210> 1143
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1143
   gaaaacaaac cataataaca 20
<210> 1144
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1144
   gaaccataat aacaataaca 20
<210> 1145
   <211> 20
   <212> DNA
   <213> Sequenzidentische und komplementäre Primersequenz, spezifisch für den Bisulfit-konvertierten sense Strang des Promotorbereichs des LINE-1 Elements
<400> 1145
   taacaataac aattttatca 20
<210> 1146
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1146
   aattttgttg atttttttaa 20
<210> 1147
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1147
   gtgtttttat tttttttagt 20
<210> 1148
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1148
   aatgtgtttg tttttgtttt 20
<210> 1149
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1149
   taattttgga ttttttttgt 20
<210> 1150
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1150
   tttttttata tattgttttg 20
<210> 1151
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1151
   gtatgtggtg tttttgtttt 20
<210> 1152
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1152
   atatttttat ttttgttttt 20
<210> 1153
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1153
   aggttgttta gtttttatgt 20
<210> 1154
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1154
   tgtattgtgg tttgagagat 20
<210> 1155
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1155
   attatgtggt taattttgga 20
<210> 1156
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1156
   gatttggggt ggagagtttt 20
<210> 1157
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1157
   tttttgggta tttttgttga 20
<210> 1158
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1158
   tgttaaagtt ttttattatt 20

<210> 1159
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1159
   gttttatgaa tttgggtgtt 20
<210> 1160
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1160
   tgttgaattg atttttttat 20
<210> 1161
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1161
   tgttgaattg atttttttat 20
<210> 1162
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1162
   gtatattgat gggttttgat 20
<210> 1163
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1163
   gtatattgat gggttttgat 20
<210> 1164
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1164
   ttttttaatt gtagaattta 20
<210> 1165
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1165
   tttgtttatt agttgatgta 20
<210> 1166
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1166
   ttatattttg gtatgatttt 20
<210> 1167
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1167
   gtgttttttt taggagtttt 20
<210> 1168
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1168
   aaagtatttt attttttttt 20
<210> 1169
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1169
   aaattttggg ttgaaaattt 20
<210> 1170
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1170
   ggttgttttt aatatttttt 20
<210> 1171
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1171
   ataattatgt gttttggagt 20
<210> 1172
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1172
   gttttatttt ttatattatt 20
<210> 1173
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1173
   atatagtttt atattttttg 20
<210> 1174
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1174
   ttgatatttt tttttttagt 20
<210> 1175
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1175
   tttgaggttt ttgtattttt 20
<210> 1176
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1176
   attagttttt ttaagtattt 20
<210> 1177
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1177
   ttttaatttt tttgtttttg 20
<210> 1178
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1178
   gaagtttttt ttttttagtt 20
<210> 1179
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1179
   tgttttgttg ttggtgagga 20
<210> 1180
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1180
   tgatggtgat gtatagatgg 20
<210> 1181
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1181
   ttttagttgt aggtttgttg 20
<210> 1182
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1182
   agtgtgtttt tgttgggggg 20
<210> 1183
   <211> 20
   <212> DNA
   <213> Sequenzidenfische und komplementäre Primersequenz, spezifisch für den Bisulfit-konvertierten antisense Strang des Promotorbereichs des LINE-1 Elements
<400> 1183
   tttatttgag gaggtagttt 20
<210> 1184
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1184
   gttgttagat agggatattt 20
<210> 1185
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1185
   ttgtgttttg tttttagagg 20
<210> 1186
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1186
   agttgtggtg ggttttattt 20
<210> 1187
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1187
   ttaagtaagt ttgggtaatg 20
<210> 1188
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1188
   ttgattttag attgttgtgt 20
<210> 1189
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1189
   ttaggtgtgg gatatagttt 20
<210> 1190
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1190
   tttttttgat ttagaaaggg 20
<210> 1191
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1191
   ttggtatttt ttagtgagat 20
<210> 1192
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1192
   tagatggaaa tgtagaaatt 20
<210> 1193
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1193
   tctacatttc catctaaaat 20
<210> 1194
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1194
   taaaaaatac caaacaataa 20
<210> 1195
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1195
   aaattccctt tctaaatcaa 20
<210> 1196
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1196
   actatatccc acacctaact 20
<210> 1197
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1197
   acaacaatct aaaatcaaac 20
<210> 1198
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1198
   aacttactta aataaacaaa 20
<210> 1199
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1199
   caccacaact caaaaaaacc 20
<210> 1200
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1200
   ctaaaaacaa aacacaaaca 20
<210> 1201
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1201
   aacttaaata tccctatcta 20
<210> 1202
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1202
   aaaaaaacaa taattctccc 20
<210> 1203
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1203
   caaataaatc cctaactcct 20
<210> 1204
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1204
   ccaacaaaaa cacactaaca 20
<210> 1205
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1205
   taaaaatcct atctattaaa 20
<210> 1206
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1206
   aaaaaccaaa aataaataaa 20
<210> 1207
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1207
   aacaaaacaa aactaaataa 20
<210> 1208
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1208
   taaaaaaaaa aaacttcaaa 20
<210> 1209
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1209
   aaccaaaaac aaaaaaatta 20
<210> 1210
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1210
   tataactaaa ataaccaata 20
<210> 1211
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1211
   aataaaacta aaaaccaaaa 20
<210> 1212
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1212
   aatacaaaaa cctcaaaaac 2 0
<210> 1213
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1213
   caacaataaa aaataaaata 20
<210> 1214
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1214
   aaaaaaaaaa aaataaaaaa 20
<210> 1215
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1215
   aaaatataaa actatataaa 20
<210> 1216
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1216
   taaaaataat ataaaaaata 20
<210> 1217
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1217
   tacaaaatat tatccaaaaa 20
<210> 1218
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1218
   aattcaaaaa atacaaaaaa 20
<210> 1219
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1219
   aattcaccaa aattaaaata 20
<210> 1220
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1220
   aaaacccatc aaactaacaa 20
<210> 1221
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1221
   aaaaaaaata aaaaccaata 20
<210> 1222
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1222
   aaccaaacta aacttcataa 20
<210> 1223
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1223
   aaatattaaa aaattttatc 20
<210> 1224
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1224
   acactaaaca taaaaaaaaa 20
<210> 1225
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1225
   atcataccaa aatataaaaa 20
<210> 1226
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1226
   atcaactaat aaacaaaatc 20
<210> 1227
   <211> 20
   <212> DNA
   <213> Sequenzidentische und komplementäre Primersequenz, spezifisch für den Bisulfit-konvertierten antisense Strang des Promotorbereichs des LINE-1 Elements
<400> 1227
   caaaatcaaa ttcacacata 20

<210> 1228
   <211> 20
   <212> DNA
   <213> Sequenzidentische und komplementäre Primersequenz, spezifisch für den Bisulfit-konvertierten sense Strang des Alu Elements
<400> 1228
   gtttgtaatt ttagtatttt 20
<210> 1229
   <211> 20
   <212> DNA
   <213> Sequenzidentische und komplementäre Primersequenz, spezifisch für den Bisulfit-konvertierten sense Strang des Alu Elements
<400> 1229
   attttagtat tttgggaggt 20
<210> 1230
   <211> 20
   <212> DNA
   <213> Sequenzidentische und komplementäre Primersequenz, spezifisch für den Bisulfit-konvertierten sense Strang des Alu Elements
<400> 1230
   ggattatttg aggttaggag 20
<210> 1231
   <211> 20
   <212> DNA
   <213> Sequenzidentische und komplementäre Primersequenz, spezifisch für den Bisulfit-konvertierten sense Strang des Alu Elements
<400> 1231
   gagattattt tggttaatat 20
<210> 1232
   <211> 20
   <212> DNA
   <213> Sequenzidentische und komplementäre Primersequenz, spezifisch für den Bisulfit-konvertierten sense Strang des Alu Elements
<400> 1232
   tggttaatat ggtgaaattt 20
<210> 1233
   <211> 20
   <212> DNA
   <213> Sequenzidentische und komplementäre Primersequenz, spezifisch für den Bisulfit-konvertierten sense Strang des Alu Elements
<400> 1233
   gtttttatta aaaatataaa 20
<210> 1234
   <211> 20
   <212> DNA
   <213> Sequenzidentische und komplementäre Primersequenz, spezifisch für den Bisulfit-konvertierten sense Strang des Alu Elements
<400> 1234
   ttaaaaatat aaaaattagt 20
<210> 1235
   <211> 19
   <212> DNA
   <213> Sequenzidentische und komplementäre Primersequenz, spezifisch für den Bisulfit-konvertierten sense Strang des Alu Elements
<400> 1235
   gtttgtaatt ttagttatt 19
<210> 1236
   <211> 20
   <212> DNA
   <213> Sequenzidentische und komplementäre Primersequenz, spezifisch für den Bisulfit-konvertierten sense Strang des Alu Elements
<400> 1236
   gggaggttga ggtaggagaa 20
<210> 1237
   <211> 20
   <212> DNA
   <213> Sequenzidentische und komplementäre Primersequenz, spezifisch für den Bisulfit-konvertierten sense Strang des Alu Elements
<400> 1237
   gttattgtat tttagtttgg 20
<210> 1238
   <211> 20
   <212> DNA
   <213> Sequenzidentische und komplementäre Primersequenz, spezifisch für den Bisulfit-konvertierten sense Strang des Alu Elements
<400> 1238
   cccaaactaa aatacaataa 20
<210> 1239
   <211> 20
   <212> DNA
   <213> Sequenzidentische und komplementäre Primersequenz, spezifisch für den Bisulfit-konvertierten sense Strang des Alu Elements
<400> 1239
   attctcctac ctcaacctcc 20
<210> 1240
   <211> 20
   <212> DNA
   <213> Sequenzidentische und komplementäre Primersequenz, spezifisch für den Bisulfit-konvertierten sense Strang des Alu Elements
<400> 1240
   ttttatattt ttaataaaaa 20
<210> 1241
   <211> 20
   <212> DNA
   <213> Sequenzidentische und komplementäre Primersequenz, spezifisch für den Bisulfit-konvertierten sense Strang des Alu Elements
<400> 1241
   catattaacc aaaataatct 20
<210> 1242
   <211> 20
   <212> DNA
   <213> Sequenzidentische und komplementäre Primersequenz, spezifisch für den Bisulfit-konvertierten sense Strang des Alu Elements
<400> 1242
   tctcctaacc tcaaataatc 20
<210> 1243
   <211> 20
   <212> DNA
   <213> Sequenzidentische und komplementäre Primersequenz, spezifisch für den Bisulfit-konvertierten sense Strang des Alu Elements
<400> 1243
   caaaatacta aaattacaaa 20
<210> 1244
   <211> 20
   <212> DNA
   <213> Sequenzidentische und komplementäre Primersequenz, spezifisch für den Bisulfit-konvertierten antisense Strang des Alu Elements
<400> 1244
   tttaggttgg agtgtagtgg 20
<210> 1245
   <211> 20
   <212> DNA
   <213> Sequenzidentische und komplementäre Primersequenz, spezifisch für den Bisulfit-konvertierten antisense Strang des Alu Elements
<400> 1245
   atttttttgt tttagttttt 20
<210> 1246
   <211> 19
   <212> DNA
   <213> Sequenzidentische und komplementäre Primersequenz, spezifisch für den Bisulfit-konvertierten antisense Strang des Alu Elements
<400> 1246
   gagtagttgg gattatagg 19
<210> 1247
   <211> 20
   <212> DNA
   <213> Sequenzidentische und komplementäre Primersequenz, spezifisch für den Bisulfit-konvertierten antisense Strang des Alu Elements
<400> 1247
   tttttgtatt tttagtagag 20
<210> 1248
   <211> 20
   <212> DNA
   <213> Sequenzidentische und komplementäre Primersequenz, spezifisch für den Bisulfit-konvertierten antisense Strang des Alu Elements
<400> 1248
   ttttattatg ttggttagga 20
<210> 1249
   <211> 20
   <212> DNA
   <213> Sequenzidentische und komplementäre Primersequenz, spezifisch für den Bisulfit-konvertierten antisense Strang des Alu Elements
<400> 1249
   attttttgat tttaggtgat 20
<210> 1250
   <211> 20
   <212> DNA
   <213> Sequenzidentische und komplementäre Primersequenz, spezifisch für den Bisulfit-konvertierten antisense Strang des Alu Elements
<400> 1250
   tttttaaagt gttgggatta 20
<210> 1251
   <211> 20
   <212> DNA
   <213> Sequenzidentische und komplementäre Primersequenz, spezifisch für den Bisulfit-konvertierten antisense Strang des Alu Elements
<400> 1251
   atcccaacac tttaaaaaac 20
<210> 1252
   <211> 20
   <212> DNA
   <213> Sequenzidentische und komplementäre Primersequenz, spezifisch für den Bisulfit-konvertierten antisense Strang des Alu Elements
<400> 1252
   aatcacctaa aatcaaaaaa 20
<210> 1253
   <211> 20
   <212> DNA
   <213> Sequenzidentische und komplementäre Primersequenz, spezifisch für den Bisulfit-konvertierten antisense Strang des Alu Elements
<400> 1253
   tcctaaccaa cataataaaa 20
<210> 1254
   <211> 20
   <212> DNA
   <213> Sequenzidentische und komplementäre Primersequenz, spezifisch für den Bisulfit-konvertierten antisense Strang des Alu Elements
<400> 1254
   tactaaaaat acaaaaatta 20
<210> 1255
   <211> 19
   <212> DNA
   <213> Sequenzidentische und komplementäre Primersequenz, spezifisch für den Bisulfit-konvertierten antisense Strang des Alu Elements
<400> 1255
   gcctataatc ccaactact 19
<210> 1256
   <211> 20
   <212> DNA
   <213> Sequenzidentische und komplementäre Primersequenz, spezifisch für den Bisulfit-konvertierten antisense Strang des Alu Elements
<400> 1256
   gaaaaactaa aacaaaaaaa 20
<210> 1257
   <211> 20
   <212> DNA
   <213> Sequenzidentische und komplementäre Primersequenz, spezifisch für den Bisulfit-konvertierten antisense Strang des Alu Elements
<400> 1257
   ccactacact ccaacctaaa 20
<210> 1258
   <211> 20
   <212> DNA
   <213> Sequenzidentische und komplementäre Primersequenz, spezifisch für den Bisulfit-konvertierten sense Strang des HERV-K Elements
<400> 1258
   gtagttgaga taagaggaag 20
<210> 1259
   <211> 20
   <212> DNA
   <213> Sequenzidentische und komplementäre Primersequenz, spezifisch für den Bisulfit-konvertierten sense Strang des HERV-K Elements
<400> 1259
   agggagaaat tattttaggg 20
<210> 1260
   <211> 20
   <212> DNA
   <213> Sequenzidentische und komplementäre Primersequenz, spezifisch für den Bisulfit-konvertierten sense Strang des HERV-K Elements
<400> 1260
   taaagtattg agatgtttat 20
<210> 1261
   <211> 20
   <212> DNA
   <213> Sequenzidentische und komplementäre Primersequenz, spezifisch für den Bisulfit-konvertierten sense Strang des HERV-K Elements
<400> 1261
   atatattttt tttttagaga 20
<210> 1262
   <211> 20
   <212> DNA
   <213> Sequenzidentische und komplementäre Primersequenz, spezifisch für den Bisulfit-konvertierten sense Strang des HERV-K Elements
<400> 1262
   gaaatattta taggtgtgga 20
<210> 1263
   <211> 20
   <212> DNA
   <213> Sequenzidentische und komplementäre Primersequenz, spezifisch für den Bisulfit-konvertierten sense Strang des HERV-K Elements
<400> 1263
   ggggtaaatt aaaattaaaa 20
<210> 1264
   <211> 20
   <212> DNA
   <213> Sequenzidentische und komplementäre Primersequenz, spezifisch für den Bisulfit-konvertierten sense Strang des HERV-K Elements
<400> 1264
   atagaataat tttgtttatg 20
<210> 1265
   <211> 20
   <212> DNA
   <213> Sequenzidentische und komplementäre Primersequenz, Spezifisch für den Bisulfit-konvertierten sense Strang des HERV-K Elements
<400> 1265
   agtaggtagg aagggtaata 20
<210> 1266
   <211> 20
   <212> DNA
   <213> Sequenzidentische und komplementäre Primersequenz, spezifisch für den Bisulfit-konvertierten sense Strang des HERV-K Elements
<400> 1266
   gttttagaat tattttaaat 20
<210> 1267
   <211> 20
   <212> DNA
   <213> Sequenzidentische und komplementäre Primersequenz, spezifisch für den Bisulfit-konvertierten sense Strang des HERV-K Elements
<400> 1267
   ggaagttgta taatagattg 20
<210> 1268
   <211> 20
   <212> DNA
   <213> Sequenzidentische und komplementäre Primersequenz, spezifisch für den Bisulfit-konvertierten sense Strang des HERV-K Elements
<400> 1268
   aattagtggg gttattagag 20
<210> 1269
   <211> 20
   <212> DNA
   <213> Sequenzidentische und komplementäre Primersequenz, spezifisch für den Bisulfit-konvertierten sense Strang des HERV-K Elements
<400> 1269
   gatttaattg ttagtagttt 20
<210> 1270
   <211> 20
   <212> DNA
   <213> Sequenzidentische und komplementäre Primersequenz, spezifisch für den Bisulfit-konvertierten sense Strang des HERV-K Elements
<400> 1270
   tatggtatta tttagtaggt 20
<210> 1271
   <211> 20
   <212> DNA
   <213> Sequenzidentische und komplementäre Primersequenz, spezifisch für den Bisulfit-konvertierten sense Strang des HERV-K Elements
<400> 1271
   gaaagaggga gtaaaatagt 20
<210> 1272
   <211> 20
   <212> DNA
   <213> Sequenzidentische und komplementäre Primersequenz, spezifisch für den Bisulfit-konvertierten sense Strang des HERV-K Elements
<400> 1272
   gaattgatgg ggtataagaa 20
<210> 1273
   <211> 20
   <212> DNA
   <213> Sequenzidentische und komplementäre Primersequenz, spezifisch für den Bisulfit-konvertierten sense Strang des HERV-K Elements
<400> 1273
   taagtattaa tgtaaaatga 20
<210> 1274
   <211> 20
   <212> DNA
   <213> Sequenzidentische und komplementäre Primersequenz, spezifisch für den Bisulfit-konvertierten sense Strang des HERV-K Elements
<400> 1274
   agagtttggg aaaaaattta 20
<210> 1275
   <211> 20
   <212> DNA
   <213> Sequenzidentische und komplementäre Primersequenz, spezifisch für den Bisulfit-konvertierten sense Strang des HERV-K Elements
<400> 1275
   atagtaagat aaggtttaaa 20
<210> 1276
   <211> 20
   <212> DNA
   <213> Sequenzidentische und komplementäre Primersequenz, spezifisch für den Bisulfit-konvertierten sense Strang des HERV-K Elements
<400> 1276
   gatgtaattt tagagtatgt 20
<210> 1277
   <211> 20
   <212> DNA
   <213> Sequenzidentische und komplementäre Primersequenz, spezifisch für den Bisulfit-konvertierten sense Strang des HERV-K Elements
<400> 1277
   atattgggtt agttaatgtt 20
<210> 1278
   <211> 20
   <212> DNA
   <213> Sequenzidentische und komplementäre Primersequenz, spezifisch für den Bisulfit-konvertierten sense Strang des HERV-K Elements
<400> 1278
   ataaaaaatt tttataggag 20
<210> 1279
   <211> 20
   <212> DNA
   <213> Sequenzidentische und komplementäre Primersequenz, spezifisch für den Bisulfit-konvertierten sense Strang des HERV-K Elements
<400> 1279
   ttagaagtgt attaaagtat 20
<210> 1280
   <211> 20
   <212> DNA
   <213> Sequenzidentische und komplementäre Primersequenz, spezifisch für den Bisulfit-konvertierten sense Strang des HERV-K Elements
<400> 1280
   tggtttatat agggttaaaa 20
<210> 1281
   <211> 20
   <212> DNA
   <213> Sequenzidentische und komplementäre Primersequenz, spezifisch für den Bisulfit-konvertierten sense Strang des HERV-K Elements
<400> 1281
   ttagttatat ggatggataa 20
<210> 1282
   <211> 20
   <212> DNA
   <213> Sequenzidentische und komplementäre Primersequenz, spezifisch für den Bisulfit-konvertierten sense Strang des HERV-K Elements
<400> 1282
   gatttaattt ttaattggta 20
<210> 1283
   <211> 20
   <212> DNA
   <213> Sequenzidentische und komplementäre Primersequenz, spezifisch für den Bisulfit-konvertierten sense Strang des HERV-K Elements
<400> 1283
   atattttgat tgaaatatta 20
<210> 1284
   <211> 20
   <212> DNA
   <213> Sequenzidentische und komplementäre Primersequenz, spezifisch für den Bisulfit-konvertierten sense Strang des HERV-K Elements
<400> 1284
   gagtattatt gggatatggt 20
<210> 1285
   <211> 20
   <212> DNA
   <213> Sequenzidentische und komplementäre Primersequenz, spezifisch für den Bisulfit-konvertierten sense Strang des HERV-K Elements
<400> 1285
   ttatgattat aaattttata 20
<210> 1286
   <211> 20
   <212> DNA
   <213> Sequenzidentische und komplementäre Primersequenz, spezifisch für den Bisulfit-konvertierten sense Strang des HERV-K Elements
<400> 1286
   agtagatata ggagatttta 20
<210> 1287
   <211> 20
   <212> DNA
   <213> Sequenzidentische und komplementäre Primersequenz, spezifisch für den Bisulfit-konvertierten sense Strang des HERV-K Elements
<400> 1287
   tttgtttagg aaagttaggt 20
<210> 1288
   <211> 20
   <212> DNA
   <213> Sequenzidentische und komplementäre Primersequenz, spezifisch für den Bisulfit-konvertierten sense Strang des HERV-K Elements
<400> 1288
   tttattgaga tagggaaaaa 20
<210> 1289
   <211> 20
   <212> DNA
   <213> Sequenzidentische und komplementäre Primersequenz, spezifisch für den Bisulfit-konvertierten sense Strang des HERV-K Elements
<400> 1289
   ataaatatta agggaattta 20
<210> 1290
   <211> 20
   <212> DNA
   <213> Sequenzidentische und komplementäre Primersequenz, spezifisch für den Bisulfit-konvertierten sense Strang des HERV-K Elements
<400> 1290
   cttaatattt attaatcatt 20
<210> 1291
   <211> 20
   <212> DNA
   <213> Sequenzidentische und komplementäre Primersequenz, spezifisch für den Bisulfit-konvertierten sense Strang des HERV-K Elements
<400> 1291
   tacatacaca taaacatctc 20
<210> 1292
   <211> 20
   <212> DNA
   <213> Sequenzidentische und komplementäre Primersequenz, spezifisch für den Bisulfit-konvertierten sense Strang des HERV-K Elements
<400> 1292
   ttccctatct caataaataa 20
<210> 1293
   <211> 20
   <212> DNA
   <213> Sequenzidentische und komplementäre Primersequenz, spezifisch für den Bisulfit-konvertierten sense Strang des HERV-K Elements
<400> 1293
   aacattccat tacccaaaaa 20
<210> 1294
   <211> 20
   <212> DNA
   <213> Sequenzidentische und komplementäre Primersequenz, spezifisch für den Bisulfit-konvertierten sense Strang des HERV-K Elements
<400> 1294
   ctcacataaa aaaaaacctt 20
<210> 1295
   <211> 20
   <212> DNA
   <213> Sequenzidentische und komplementäre Primersequenz, spezifisch für den Bisulfit-konvertierten sense Strang des HERV-K Elements
<400> 1295
   taaaaaataa taataactct 20
<210> 1296
   <211> 20
   <212> DNA
   <213> Sequenzidentische und komplementäre Primersequenz, spezifisch für den Bisulfit-konvertierten sense Strang des HERV-K Elements
<400> 1296
   tccatttaac ccaaaattta 20

<210> 1297
   <211> 20
   <212> DNA
   <213> Sequenzidentische und komplementäre Primersequenz, spezifisch für den Bisulfit-konvertierten sense Strang des HERV-K Elements
<400> 1297
   aacaaaaaaa tttttcttaa 20
<210> 1298
   <211> 20
   <212> DNA
   <213> Sequenzidentische und komplementäre Primersequenz, spezifisch für den Bisulfit-konvertierten sense Strang des HERV-K Elements
<400> 1298
   actaacaaca aacaaaacaa 20
<210> 1299
   <211> 20
   <212> DNA
   <213> Sequenzidentische und komplementäre Primersequenz, spezifisch für den Bisulfit-konvertierten sense Strang des HERV-K Elements
<400> 1299
   tcctaacacc aaatttaaat 20
<210> 1300
   <211> 20
   <212> DNA
   <213> Sequenzidentische und komplementäre Primersequenz, spezifisch für den Bisulfit-konvertierten sense Strang des HERV-K Elements
<400> 1300
   ctaaaataaa attatcttct 20
<210> 1301
   <211> 20
   <212> DNA
   <213> Sequenzidentische und komplementäre Primersequenz, spezifisch für den Bisulfit-konvertierten sense Strang des HERV-K Elements
<400> 1301
   tattctaaaa tcataaacct 20
<210> 1302
   <211> 20
   <212> DNA
   <213> Sequenzidentische und komplementäre Primersequenz, spezifisch für den Bisulfit-konvertierten sense Strang des HERV-K Elements
<400> 1302
   aacttaccaa tttttaatca 20
<210> 1303
   <211> 20
   <212> DNA
   <213> Sequenzidentische und komplementäre Primersequenz, spezifisch für den Bisulfit-konvertierten sense Strang des HERV-K Elements
<400> 1303
   aatcaaaata taaataaata 20
<210> 1304
   <211> 20
   <212> DNA
   <213> Sequenzidentische und komplementäre Primersequenz, spezifisch für den Bisulfit-konvertierten sense Strang des HERV-K Elements
<400> 1304
   aactaattta ataactatat 20
<210> 1305
   <211> 20
   <212> DNA
   <213> Sequenzidentische und komplementäre Primersequenz, spezifisch für den Bisulfit-konvertierten sense Strang des HERV-K Elements
<400> 1305
   tatacttata tttatctaaa 20
<210> 1306
   <211> 20
   <212> DNA
   <213> Sequenzidentische und komplementäre Primersequenz, spezifisch für den Bisulfit-konvertierten sense Strang des HERV-K Elements
<400> 1306
   cttaaaacaa attttccctt 20
<210> 1307
   <211> 20
   <212> DNA
   <213> Sequenzidentische und komplementäre Primersequenz, spezifisch für den Bisulfit-konvertierten sense Strang des HERV-K Elements
<400> 1307
   catcctaata ctctccctaa 20
<210> 1308
   <211> 20
   <212> DNA
   <213> Sequenzidentische und komplementäre Primersequenz, spezifisch für den Bisulfit-konvertierten sense Strang des HERV-K Elements
<400> 1308
   cattataaaa cttcaaatat 20
<210> 1309
   <211> 20
   <212> DNA
   <213> Sequenzidentische und komplementäre Primersequenz, spezifisch für den Bisulfit-konvertierten sense Strang des HERV-K Elements
<400> 1309
   taaaattttc cactaactta 20
<210> 1310
   <211> 20
   <212> DNA
   <213> Sequenzidentische und komplementäre Primersequenz, spezifisch für den Bisulfit-konvertierten sense Strang des HERV-K Elements
<400> 1310
   cattactaaa accatcaata 20
<210> 1311
   <211> 20
   <212> DNA
   <213> Sequenzidentische und komplementäre Primersequenz, spezifisch für den Bisulfit-konvertierten sense Strang des HERV-K Elements
<400> 1311
   tttactaata aatataaaac 20
<210> 1312
   <211> 20
   <212> DNA
   <213> Sequenzidentische und komplementäre Primersequenz, spezifisch für den Bisulfit-konvertierten sense Strang des HERV-K Elements
<400> 1312
   atataaaatc tcaatacttt 20
<210> 1313
   <211> 20
   <212> DNA
   <213> Sequenzidentische und komplementäre Primersequenz, spezifisch für den Bisulfit-konvertierten sense Strang des HERV-K Elements
<400> 1313
   aaccttaata tataacaaaa 20
<210> 1314
   <211> 20
   <212> DNA
   <213> Sequenzidentische und komplementäre Primersequenz, spezifisch für den Bisulfit-konvertierten sense Strang des HERV-K Elements
<400> 1314
   aactccccta aaaacaaaaa 20
<210> 1315
   <211> 20
   <212> DNA
   <213> Sequenzidentische und komplementäre Primersequenz, spezifisch für den Bisulfit-konvertierten sense Strang des HERV-K Elements
<400> 1315
   ctctacctat tattataata 20
<210> 1316
   <211> 20
   <212> DNA
   <213> Sequenzidentische und komplementäre Primersequenz, spezifisch für den Bisulfit-konvertierten sense Strang des HERV-K Elements
<400> 1316
   tctaaaatta catctaatcc 20
<210> 1317
   <211> 20
   <212> DNA
   <213> Sequenzidentische und komplementäre Primersequenz, spezifisch für den Bisulfit-konvertierten sense Strang des HERV-K Elements
<400> 1317
   accctaccta ctaaataata 20
<210> 1318
   <211> 20
   <212> DNA
   <213> Sequenzidentische und komplementäre Primersequenz, spezifisch für den Bisulfit-konvertierten sense Strang des HERV-K Elements
<400> 1318
   acctcctata attaattata 20
<210> 1319
   <211> 20
   <212> DNA
   <213> Sequenzidentische und komplementäre Primersequenz, spezifisch für den Bisulfit-konvertierten sense Strang des HERV-K Elements
<400> 1319
   attttaataa aactaaaata 20
<210> 1320
   <211> 20
   <212> DNA
   <213> Sequenzidentische und komplementäre Primersequenz, spezifisch für den Bisulfit-konvertierten sense Strang des HERV-K Elements
<400> 1320
   cataaacaaa ataaaaaatt 20
<210> 1321
   <211> 20
   <212> DNA
   <213> Sequenzidentische und komplementäre Primersequenz, spezifisch für den Bisulfit-konvertierten sense Strang des HERV-K Elements
<400> 1321
   ctaatcctcc tcaacacaaa 20
<210> 1322
   <211> 20
   <212> DNA
   <213> Sequenzidentische und komplementäre Primersequenz, spezifisch für den Bisulfit-konvertierten sense Strang des HERV-K Elements
<400> 1322
   ccttcaaaca tctatttaac 20
<210> 1323
   <211> 20
   <212> DNA
   <213> Sequenzidentische und komplementäre Primersequenz, spezifisch für den Bisulfit-konvertierten sense Strang des HERV-K Elements
<400> 1323
   ttaacaacat ctcaaaacaa 20
<210> 1324
   <211> 20
   <212> DNA
   <213> Sequenzidentische und komplementäre Primersequenz, spezifisch für den Bisulfit-konvertierten antisense Strang des HERV-K Elements
<400> 1324
   ttttttagta tttattgatt 20
<210> 1325
   <211> 20
   <212> DNA
   <213> Sequenzidentische und komplementäre Primersequenz, spezifisch für den Bisulfit-konvertierten antisense Strang des HERV-K Elements
<400> 1325
   ggggatgtgt tagggttata 20
<210> 1326
   <211> 20
   <212> DNA
   <213> Sequenzidentische und komplementäre Primersequenz, spezifisch für den Bisulfit-konvertierten antisense Strang des HERV-K Elements
<400> 1326
   tgtattatag ataaggtaaa 20
<210> 1327
   <211> 20
   <212> DNA
   <213> Sequenzidentische und komplementäre Primersequenz, spezifisch für den Bisulfit-konvertierten antisense Strang des HERV-K Elements
<400> 1327
   atatgtatat atataaatat 20
<210> 1328
   <211> 20
   <212> DNA
   <213> Sequenzidentische und komplementäre Primersequenz, spezifisch für den Bisulfit-konvertierten antisense Strang des HERV-K Elements
<400> 1328
   ttttttttta ttttagtaga 20
<210> 1329
   <211> 20
   <212> DNA
   <213> Sequenzidentische und komplementäre Primersequenz, spezifisch für den Bisulfit-konvertierten antisense Strang des HERV-K Elements
<400> 1329
   gatgtttttt ttttttttta 20
<210> 1330
   <211> 20
   <212> DNA
   <213> Sequenzidentische und komplementäre Primersequenz, spezifisch für den Bisulfit-konvertierten antisense Strang des HERV-K Elements
<400> 1330
   ggatggttag gttttttttt 20
<210> 1331
   <211> 20
   <212> DNA
   <213> Sequenzidentische und komplementäre Primersequenz, spezifisch für den Bisulfit-konvertierten antisense Strang des HERV-K Elements
<400> 1331
   agattaggga gtggtgatga 20
<210> 1332
   <211> 20
   <212> DNA
   <213> Sequenzidentische und komplementäre Primersequenz, spezifisch für den Bisulfit-konvertierten antisense Strang des HERV-K Elements
<400> 1332
   ttgatatagt atatgtttta 20
<210> 1333
   <211> 20
   <212> DNA
   <213> Sequenzidentische und komplementäre Primersequenz, spezifisch für den Bisulfit-konvertierten antisense Strang des HERV-K Elements
<400> 1333
   gattaatagt attttaaggt 20
<210> 1334
   <211> 20
   <212> DNA
   <213> Sequenzidentische und komplementäre Primersequenz, spezifisch für den Bisulfit-konvertierten antisense Strang des HERV-K Elements
<400> 1334
   gtaataattt tatttttttt 20
<210> 1335
   <211> 20
   <212> DNA
   <213> Sequenzidentische und komplementäre Primersequenz, spezifisch für den Bisulfit-konvertierten antisense Strang des HERV-K Elements
<400> 1335
   gatttataat tatagtattt 20
<210> 1336
   <211> 20
   <212> DNA
   <213> Sequenzidentische und komplementäre Primersequenz, spezifisch für den Bisulfit-konvertierten antisense Strang des HERV-K Elements
<400> 1336
   aatttttgta attgttttag 20
<210> 1337
   <211> 20
   <212> DNA
   <213> Sequenzidentische und komplementäre Primersequenz, spezifisch für den Bisulfit-konvertierten antisense Strang des HERV-K Elements
<400> 1337
   tggaaatgtt taaagtgaga 20
<210> 1338
   <211> 20
   <212> DNA
   <213> Sequenzidentische und komplementäre Primersequenz, spezifisch für den Bisulfit-konvertierten antisense Strang des HERV-K Elements
<400> 1338
   gtttagattt attataaatt 20
<210> 1339
   <211> 20
   <212> DNA
   <213> Sequenzidentische und komplementäre Primersequenz, spezifisch für den Bisulfit-konvertierten antisense Strang des HERV-K Elements
<400> 1339
   ttgtaattaa agtaaaaatg 20
<210> 1340
   <211> 20
   <212> DNA
   <213> Sequenzidentische und komplementäre Primersequenz, spezifisch für den Bisulfit-konvertierten antisense Strang des HERV-K Elements
<400> 1340
   ggtttaataa ttatattttt 20
<210> 1341
   <211> 20
   <212> DNA
   <213> Sequenzidentische und komplementäre Primersequenz, spezifisch für den Bisulfit-konvertierten antisense Strang des HERV-K Elements
<400> 1341
   ttttggggta gagatttttt 20
<210> 1342
   <211> 20
   <212> DNA
   <213> Sequenzidentische und komplementäre Primersequenz, spezifisch für den Bisulfit-konvertierten antisense Strang des HERV-K Elements
<400> 1342
   ttgagtaatt gtggtagaat 20
<210> 1343
   <211> 20
   <212> DNA
   <213> Sequenzidentische und komplementäre Primersequenz, spezifisch für den Bisulfit-konvertierten antisense Strang des HERV-K Elements
<400> 1343
   ttaaattaaa atttttgtat 20
<210> 1344
   <211> 20
   <212> DNA
   <213> Sequenzidentische und komplementäre Primersequenz, spezifisch für den Bisulfit-konvertierten antisense Strang des HERV-K Elements
<400> 1344
   gataagtgaa tttattgtta 20
<210> 1345
   <211> 20
   <212> DNA
   <213> Sequenzidentische und komplementäre Primersequenz, spezifisch für den Bisulfit-konvertierten antisense Strang des HERV-K Elements
<400> 1345 20
   aggtattaaa tattttggtg 20
<210> 1346
   <211> 20
   <212> DNA
   <213> Sequenzidentische und komplementäre Primersequenz, spezifisch für den Bisulfit-konvertierten antisense Strang des HERV-K Elements
<400> 1346
   tatttttata ggattattta 20
<210> 1347
   <211> 20
   <212> DNA
   <213> Sequenzidentische und komplementäre Primersequenz, spezifisch für den Bisulfit-konvertierten antisense Strang des HERV-K Elements
<400> 1347
   attttaaatg tttagtgggt 20
<210> 1348
   <211> 20
   <212> DNA
   <213> Sequenzidentische und komplementäre Primersequenz, spezifisch für den Bisulfit-konvertierten antisense Strang des HERV-K Elements
<400> 1348
   tattttttat gttttatttt 20
<210> 1349
   <211> 20
   <212> DNA
   <213> Sequenzidentische und komplementäre Primersequenz, spezifisch für den Bisulfit-konvertierten antisense Strang des HERV-K Elements
<400> 1349
   gttagattaa gttgtatttg 20
<210> 1350
   <211> 20
   <212> DNA
   <213> Sequenzidentische und komplementäre Primersequenz, spezifisch für den Bisulfit-konvertierten antisense Strang des HERV-K Elements
<400> 1350
   agttgtatat atgaaatgtg 20
<210> 1351
   <211>. 20
   <212> DNA
   <213> Sequenzidentische und komplementäre Primersequenz, spezifisch für den Bisulfit-konvertierten antisense Strang des HERV-K Elements
<400> 1351
   ttttaatatt ttttgtagtt 20
<210> 1352
   <211> 20
   <212> DNA
   <213> Sequenzidentische und komplementäre Primersequenz, spezifisch für den Bisulfit-konvertierten antisense Strang des HERV-K Elements
<400> 1352
   gttatttttt tattgttgga 20
<210> 1353
   <211> 20
   <212> DNA
   <213> Sequenzidentische und komplementäre Primersequenz, spezifisch für den Bisulfit-konvertierten antisense Strang des HERV-K Elements
<400> 1353
   ttattttgtt tgggttattt 20
<210> 1354
   <211> 20
   <212> DNA
   <213> Sequenzidentische und komplementäre Primersequenz, spezifisch für den Bisulfit-konvertierten antisense Strang des HERV-K Elements
<400> 1354
   tattttttag taatttttga 20
<210> 1355
   <211> 20
   <212> DNA
   <213> Sequenzidentische und komplementäre Primersequenz, spezifisch für den Bisulfit-konvertierten antisense Strang des HERV-K Elements
<400> 1355
   gatttttttt gaattataaa 20
<210> 1356
   <211> 20
   <212> DNA
   <213> Sequenzidentische und komplementäre Primersequenz, spezifisch für den Bisulfit-konvertierten antisense Strang des HERV-K Elements
<400> 1356
   tttattttgg ttataatttt 20
<210> 1357
   <211> 20
   <212> DNA
   <213> Sequenzidentische und komplementäre Primersequenz, spezifisch für den Bisulfit-konvertierten antisense Strang des HERV-K Elements
<400> 1357
   ggttttaagt aatgtaaaat 20
<210> 1358
   <211> 20
   <212> DNA
   <213> Sequenzidentische und komplementäre Primersequenz, spezifisch für den Bisulfit-konvertierten antisense Strang des HERV-K Elements
<400> 1358
   aattttttaa gttgtttttt 20
<210> 1359
   <211> 20
   <212> DNA
   <213> Sequenzidentische und komplementäre Primersequenz, spezifisch für den Bisulfit-konvertierten antisense Strang des HERV-K Elements
<400> 1359
   ttttaatata tggtaggagt 20
<210> 1360
   <211> 20
   <212> DNA
   <213> Sequenzidentische und komplementäre Primersequenz, spezifisch für den Bisulfit-konvertierten antisense Strang des HERV-K Elements
<400> 1360
   aagattagtt ttatagtttt 20
<210> 1361
   <211> 20
   <212> DNA
   <213> Sequenzidentische und komplementäre Primersequenz, spezifisch für den Bisulfit-konvertierten antisense Strang des HERV-K Elements
<400> 1361
   ttaaatttag aatgatattg 20
<210> 1362
   <211> 20
   <212> DNA
   <213> Sequenzidentische und komplementäre Primersequenz, spezifisch für den Bisulfit-konvertierten antisense Strang des HERV-K Elements
<400> 1362
   tgtttgggtt ataagtatag 20
<210> 1363
   <211> 20
   <212> DNA
   <213> Sequenzidentische und komplementäre Primersequenz, spezifisch für den Bisulfit-konvertierten antisense Strang des HERV-K Elements
<400> 1363
   gtttttttga attttgtttt 20
<210> 1364
   <211> 20
   <212> DNA
   <213> Sequenzidentische und komplementäre Primersequenz, spezifisch für den Bisulfit-konvertierten antisense Strang des HERV-K Elements
<400> 1364
   atagttgatt tgtatttatg 20
<210> 1365
   <211> 20
   <212> DNA
   <213> Sequenzidentische und komplementäre Primersequenz, spezifisch für den Bisulfit-konvertierten antisense Strang des HERV-K Elements
<400> 1365
   tattgtttta tttttttttt 20

<210> 1366
   <211> 20
   <212> DNA
   <213> Sequenzidentische und komplementäre Primersequenz, spezifisch für den Bisulfit-konvertierten antisense Strang des HERV-K Elements
<400> 1366
   gattttttaa taattttatg 20
<210> 1367
   <211> 20
   <212> DNA
   <213> Sequenzidentische und komplementäre Primersequenz, spezifisch für den Bisulfit-konvertierten antisense Strang des HERV-K Elements
<400> 1367
   aaatggtttt aaagttgttt 20
<210> 1368
   <211> 20
   <212> DNA
   <213> Sequenzidentische und komplementäre Primersequenz, spezifisch für den Bisulfit-konvertierten antisense Strang des HERV-K Elements
<400> 1368
   tttttatatt tgtgggtgtt 20
<210> 1369
   <211> 20
   <212> DNA
   <213> Sequenzidentische und komplementäre Primersequenz, spezifisch für den Bisulfit-konvertierten antisense Strang des HERV-K Elements
<400> 1369
   taatagtggg gagagggtga 20
<210> 1370
   <211> 20
   <212> DNA
   <213> Sequenzidentische und komplementäre Primersequenz, spezifisch für den Bisulfit-konvertierten antisense Strang des HERV-K Elements
<400> 1370
   ggaaatagat gttttttttt 20
<210> 1371
   <211> 20
   <212> DNA
   <213> Sequenzidentische und komplementäre Primersequenz, spezifisch für den Bisulfit-konvertierten antisense Strang des HERV-K Elements
<400> 1371
   tttgagatta gggagtggtg 20
<210> 1372
   <211> 20
   <212> DNA
   <213> Sequenzidentische und komplementäre Primersequenz, spezifisch für den Bisulfit-konvertierten antisense Strang des HERV-K Elements
<400> 1372
   atctataacc ttacccccaa 20
<210> 1373
   <211> 20
   <212> DNA
   <213> Sequenzidentische und komplementäre Primersequenz, spezifisch für den Bisulfit-konvertierten antisense Strang des HERV-K Elements
<400> 1373
   aacaaatact taaaaacaac 20
<210> 1374
   <211> 20
   <212> DNA
   <213> Sequenzidentische und komplementäre Primersequenz, spezifisch für den Bisulfit-konvertierten antisense Strang des HERV-K Elements
<400> 1374
   aatctcaaat acccaaaaac 20
<210> 1375
   <211> 20
   <212> DNA
   <213> Sequenzidentische und komplementäre Primersequenz, spezifisch für den Bisulfit-konvertierten antisense Strang des HERV-K Elements
<400> 1375
   tccccatata aaaatctaaa 20
<210> 1376
   <211> 20
   <212> DNA
   <213> Sequenzidentische und komplementäre Primersequenz, spezifisch für den Bisulfit-konvertierten antisense Strang des HERV-K Elements
<400> 1376
   aaaaaaaatt aatataaaaa 20
<210> 1377
   <211> 20
   <212> DNA
   <213> Sequenzidentische und komplementäre Primersequenz, spezifisch für den Bisulfit-konvertierten antisense Strang des HERV-K Elements
<400> 1377
   caccttaaaa ctaaaaataa 20
<210> 1378
   <211> 20
   <212> DNA
   <213> Sequenzidentische und komplementäre Primersequenz, spezifisch für den Bisulfit-konvertierten antisense Strang des HERV-K Elements
<400> 1378
   cacatctccc tctcaaaaaa 20
<210> 1379
   <211> 20
   <212> DNA
   <213> Sequenzidentische und komplementäre Primersequenz, spezifisch für den Bisulfit-konvertierten antisense Strang des HERV-K Elements
<400> 1379
   tttttctttt ccaaatctct 20
<210> 1380
   <211> 20
   <212> DNA
   <213> Sequenzidentische und komplementäre Primersequenz, spezifisch für den Bisulfit-konvertierten antisense Strang des HERV-K Elements
<400> 1380
   tttctctaaa ataaaaatac 20
<210> 1381
   <211> 20
   <212> DNA
   <213> Sequenzidentische und komplementäre Primersequenz, spezifisch für den Bisulfit-konvertierten antisense Strang des HERV-K Elements
<400> 1381
   cttaacttca ttaaaattct 20
<210> 1382
   <211> 20
   <212> DNA
   <213> Sequenzidentische und komplementäre Primersequenz, spezifisch für den Bisulfit-konvertierten antisense Strang des HERV-K Elements
<400> 1382
   aacaaataaa aaaaataata 20
<210> 1383
   <211> 20
   <212> DNA
   <213> Sequenzidentische und komplementäre Primersequenz, spezifisch für den Bisulfit-konvertierten antisense Strang des HERV-K Elements
<400> 1383
   aattacaaaa aataatatat 20
<210> 1384
   <211> 20
   <212> DNA
   <213> Sequenzidentische und komplementäre Primersequenz, spezifisch für den Bisulfit-konvertierten antisense Strang des HERV-K Elements
<400> 1384
   ccaactacca ataacttatc 20
<210> 1385
   <211> 20
   <212> DNA
   <213> Sequenzidentische und komplementäre Primersequenz, spezifisch für den Bisulfit-konvertierten antisense Strang des HERV-K Elements
<400> 1385
   aataaacaaa ataataaatt 20
<210> 1386
   <211> 20
   <212> DNA
   <213> Sequenzidentische und komplementäre Primersequenz, spezifisch für den Bisulfit-konvertierten antisense Strang des HERV-K Elements
<400> 1386
   ccaaataaaa atctttttaa 20
<210> 1387
   <211> 20
   <212> DNA
   <213> Sequenzidentische und komplementäre Primersequenz, spezifisch für den Bisulfit-konvertierten antisense Strang des HERV-K Elements
<400> 1387
   tttacaattt aaaacttaat 20
<210> 1388
   <211> 20
   <212> DNA
   <213> Sequenzidentische und komplementäre Primersequenz, spezifisch für den Bisulfit-konvertierten antisense Strang des HERV-K Elements
<400> 1388
   aattaaaact atctacctta 20
<210> 1389
   <211> 20
   <212> DNA
   <213> Sequenzidentische und komplementäre Primersequenz, spezifisch für den Bisulfit-konvertierten antisense Strang des HERV-K Elements
<400> 1389
   ccattaaacc attaaaaaaa 20
<210> 1390
   <211> 20
   <212> DNA
   <213> Sequenzidentische und komplementäre Primersequenz, spezifisch für den Bisulfit-konvertierten antisense Strang des HERV-K Elements
<400> 1390
   atcaaaataa tcatttaaaa 20
<210> 1391
   <211> 20
   <212> DNA
   <213> Sequenzidentische und komplementäre Primersequenz, spezifisch für den Bisulfit-konvertierten antisense Strang des HERV-K Elements
<400> 1391
   taataaaaat aaacaaccat 20
<210> 1392
   <211> 20
   <212> DNA
   <213> Sequenzidentische und komplementäre Primersequenz, spezifisch für den Bisulfit-konvertierten antisense Strang des HERV-K Elements
<400> 1392
   caacccccac tatcccaaat 20
<210> 1393
   <211> 20
   <212> DNA
   <213> Sequenzidentische und komplementäre Primersequenz, spezifisch für den Bisulfit-konvertierten antisense Strang des HERV-K Elements
<400> 1393
   ataccaatcc aaaaaacaaa 20
<210> 1394
   <211> 20
   <212> DNA
   <213> Sequenzidentische und komplementäre Primersequenz, spezifisch für den Bisulfit-konvertierten antisense Strang des HERV-K Elements
<400> 1394
   aaatcaataa ccaaaaaatt 20
<210> 1395
   <211> 20
   <212> DNA
   <213> Sequenzidentische und komplementäre Primersequenz, spezifisch für den Bisulfit-konvertierten antisense Strang des HERV-K Elements
<400> 1395
   taaccaaaat aaaatatata 20
<210> 1396
   <211> 20
   <212> DNA
   <213> Sequenzidentische und komplementäre Primersequenz, spezifisch für den Bisulfit-konvertierten antisense Strang des HERV-K Elements
<400> 1396
   taattcaaaa aaaatccaac 20
<210> 1397
   <211> 20
   <212> DNA
   <213> Sequenzidentische und komplementäre Primersequenz, spezifisch für den Bisulfit-konvertierten antisense Strang des HERV-K Elements
<400> 1397
   aaaattacca atacaaaact 20
<210> 1398
   <211> 20
   <212> DNA
   <213> Sequenzidentische und komplementäre Primersequenz, spezifisch für den Bisulfit-konvertierten antisense Strang des HERV-K Elements
<400> 1398
   atcccttaac cccactccaa 20
<210> 1399
   <211> 20
   <212> DNA
   <213> Sequenzidentische und komplementäre Primersequenz, spezifisch für den Bisulfit-konvertierten antisense Strang des HERV-K Elements
<400> 1399
   ataaaaataa cccaaacaaa 20
<210> 1400
   <211> 20
   <212> DNA
   <213> Sequenzidentische und komplementäre Primersequenz, spezifisch für den Bisulfit-konvertierten antisense Strang des HERV-K Elements
<400> 1400
   aaaaaaataa cttacacaaa 20
<210> 1401
   <211> 20
   <212> DNA
   <213> Sequenzidentische und komplementäre Primersequenz, spezifisch für den Bisulfit-konvertierten antisense Strang des HERV-K Elements
<400> 1401
   aaaaaacttc ccattttata 20
<210> 1402
   <211> 20
   <212> DNA
   <213> Sequenzidentische und komplementäre Primersequenz, spezifisch für den Bisulfit-konvertierten antisense Strang des HERV-K Elements
<400> 1402
   acttattcac atttcatata 20
<210> 1403
   <211> 20
   <212> DNA
   <213> Sequenzidentische und komplementäre Primersequenz, spezifisch für den Bisulfit-konvertierten antisense Strang des HERV-K Elements
<400> 1403
   cacataaaaa aaaactaatt 20
<210> 1404
   <211> 20
   <212> DNA
   <213> Sequenzidentische und komplementäre Primersequenz, spezifisch für den Bisulfit-konvertierten antisense Strang des HERV-K Elements
<400> 1404
   taataataat atataaatac 20
<210> 1405
   <211> 20
   <212> DNA
   <213> Sequenzidentische und komplementäre Primersequenz, spezifisch für den Bisulfit-konvertierten antisense Strang des HERV-K Elements
<400> 1405
   cttatcaaaa atcattaaaa 20
<210> 1406
   <211> 20
   <212> DNA
   <213> Sequenzidentische und komplementäre Primersequenz, spezifisch für den Bisulfit-konvertierten antisense Strang des HERV-K Elements
<400> 1406
   tacacaaata aatccaacta 20
<210> 1407
   <211> 20
   <212> DNA
   <213> Sequenzidentische und komplementäre Primersequenz, spezifisch für den Bisulfit-konvertierten antisense Strang des HERV-K Elements
<400> 1407
   ctataaccta taaaaattat 20
<210> 1408
   <211> 20
   <212> DNA
   <213> Sequenzidentische und komplementäre Primersequenz, spezifisch für den Bisulfit-konvertierten antisense Strang des HERV-K Elements
<400> 1408
   acaaaaaaat tctacaaaat 20
<210> 1409
   <211> 20
   <212> DNA
   <213> Sequenzidentische und komplementäre Primersequenz, spezifisch für den Bisulfit-konvertierten antisense Strang des HERV-K Elements
<400> 1409
   taaatctaaa catcactaaa 20
<210> 1410
   <211> 20
   <212> DNA
   <213> Sequenzidentische und komplementäre Primersequenz, spezifisch für den Bisulfit-konvertierten antisense Strang des HERV-K Elements
<400> 1410
   tattattaat ctacaaatat 20
<210> 1411
   <211> 20
   <212> DNA
   <213> Sequenzidentische und komplementäre Primersequenz, spezifisch für den Bisulfit-konvertierten antisense Strang des HERV-K Elements
<400> 1411
   aaaataatac aaaatatact 20
<210> 1412
   <211> 20
   <212> DNA
   <213> Sequenzidentische und komplementäre Primersequenz, spezifisch für den Bisulfit-konvertierten antisense Strang des HERV-K Elements
<400> 1412
   taatataaaa aaaaaacata 20
<210> 1413
   <211> 20
   <212> DNA
   <213> Sequenzidentische und komplementäre Primersequenz, spezifisch für den Bisulfit-konvertierten antisense Strang des HERV-K Elements
<400> 1413
   actaccttaa aactaaaaat 20
<210> 1414
   <211> 20
   <212> DNA
   <213> Sequenzidentische und komplementäre Primersequenz, spezifisch für den Bisulfit-konvertierten antisense Strang des HERV-K Elements
<400> 1414
   tattatctta taaccctaac 20
<210> 1415
   <211> 20
   <212> DNA
   <213> Sequenzidentische und komplementäre Primersequenz, spezifisch für den Bisulfit-konvertierten antisense Strang des HERV-K Elements
<400> 1415
   tccaccttat aaaaaacacc 20